# EUROPÄISCHE PATENTSCHRIFT

(11) **EP 2 398 788 B1**
(45) Veröffentlichungstag und Bekanntmachung des Hinweises auf die Patenterteilung: **09.09.2015**
(21) Anmeldenummer: 10711322.7
(22) Anmeldetag: 16.02.2010
(51) Int. Cl.: C09K 19/04

(54) **THIOPHEN-VERBINDUNGEN FÜR FLÜSSIGKRISTALLINE MEDIEN**
THIOPHENE COMPOUNDS FOR LIQUID-CRYSTALLINE MEDIA
COMPOSÉS THIOPHÈNE POUR MILIEUX CRISTALLINS LIQUIDES

(30) Priorität: 19.02.2009 DE 102009009630; 07.09.2009 DE 102009040215
(43) Veröffentlichungstag der Anmeldung: 28.12.2011
(73) Patentinhaber: Merck Patent GmbH, 64293 Darmstadt (DE)
(72) Erfinder: LIETZAU, Lars, 64295 Darmstadt (DE); FARRAND, Louise, Diane, Dorset DT11 7BW (GB); CZANTA, Markus, 64285 Darmstadt (DE); HIRSCHMANN, Harald, 64291 Darmstadt (DE); WITTEK, Michael, 64285 Darmstadt (DE); SAITO, Izumi, 64285 Darmstadt (DE); SCHULER, Brigitte, 63762 Grossostheim (DE)
(86) Internationale Anmeldenummer: PCT/EP2010/000968
(87) Internationale Veröffentlichungsnummer: WO 2010/094455

(56) Entgegenhaltungen:
- EP-A1- 0 786 445
- EP-A2- 0 467 260
- DATABASE WPI Week 200767 Thomson Scientific, London, GB; AN 2007-711534 XP002585579 -& JP 2007 084487 A (ASAHI DENKA KOGYO KK) 5. April 2007 (2007-04-05)

## Beschreibung

Die Erfindung betrifft Thiophen-Derivate mit einer Difluormethylenoxy-Gruppe sowie ihre Verwendung als Komponente(n) in flüssigkristallinen Medien (LC-Medien). Darüber hinaus betrifft die vorliegende Erfindung Flüssigkristall- und elektrooptische Anzeigeelemente, welche die erfindungsgemäßen, flüssigkristallinen Medien enthalten.

In den vergangenen Jahren wurden die Anwendungsgebiete für flüssigkristalline Verbindungen auf verschiedene Arten von Anzeigevorrichtungen, elektrooptische Geräte, elektronische Komponenten, Sensoren, etc. erheblich ausgeweitet, Aus diesem Grund wurden eine Reihe verschiedener Strukturen vorgeschlagen, insbesondere auf dem Gebiet der nematischen Flüssigkristalle. Die nematischen Flüssigkristallmischungen haben bisher die breiteste Anwendung in flachen Anzeigevorrichtungen gefunden. Sie wurden besonders in passiven TN- oder STN-Matrixanzeigen oder Systemen mit einer TFT-Aktivmatrix eingesetzt.

Die erfindungsgemäßen Verbindungen können als Komponente(n) flüssigkristalliner Medien (LC-Medien) verwendet werden, insbesondere für Displays, die auf dem Prinzip der verdrillten Zelle, dem Guest-Host-Effekt; dem Effekt der Deformation aufgerichteter Phasen DAP oder ECB (electrically controlled birefringence), dem IPS-Effekt (in-plane switching) oder dem Effekt der dynamischen Streuung beruhen.

Verschiedene Verbindungen mit einer Difluormethylenoxy-Brücke als flüssigkristallines Material und dessen Herstellung wurden beschrieben, wie z. B. in der Druckschrift EP 0786445 A1.

Thiophen-Derivate wurden vereinzelt als flüssigkristalline Substanzen untersucht. Die EP 0467260 A2 offenbart Verbindungen mit einer 2,5-Thiophen-diyleinheit. Die Verbindungen sind jedoch überwiegend smektisch. Für moderne Displayanwendungen werden meistens nematische Flüssigkristallmedien verwendet.

In der JP 2007084487 A werden Thiophenverbindungen beschrieben, die in 2-Position durch 1-(2,3-Difluorphenyloxy)difluormethyl-Gruppen substituert sind und die sich als Komponenten flüssigkristalliner Medien und insbesondere für Displays eignen.

EP 0786445 A1 offenbart Difluoroxymethan-Derivate enthaltende Flüssigkristallmedien, die sich für niedrige Spannungen in Displays nach beispielsweise dem Aktiv-Modus und STN-Modus eignen.

Der vorliegenden Erfindung lag die Aufgabe zugrunde, neue stabile Verbindungen aufzufinden, die als Komponente(n) flüssigkristalliner Medien geeignet sind. Insbesondere sollen die Verbindungen gleichzeitig eine vergleichsweise geringe Viskosität, sowie eine dielektrische Anisotropie im positiven Bereich besitzen. Für viele aktuelle Mischungskonzept im Bereich der Flüssigkristalle ist es vorteilhaft, Verbindungen mit einer hohen dielektrischen Anisotropie Δε zu verwenden.

Im Hinblick auf die verschiedensten Einsatzbereiche derartiger Verbindungen mit hohem Δε war es wünschenswert, weitere Verbindungen, vorzugsweise mit hoher Nematogenität zur Verfügung zu haben, die auf die jeweiligen Anwendungen genau maßgeschneiderte Eigenschaften aufweisen.

Der Erfindung lag somit als eine Aufgabe zugrunde, neue stabile, Verbindungen aufzufinden, die als Komponente(n) flüssigkristalliner Medien, insbesondere für z. B. TN-, STN-, IPS- und TN-TFT-Displays, geeignet sind.

Eine weitere Aufgabe der vorliegenden Erfindung war es, Verbindungen bereitzustellen, die für sich oder in Mischungen eine hohe dielektrische Anisotropie Δε sowie einen hohen Klärpunkt aufweisen. Darüber hinaus sollten die erfindungsgemäßen Verbindungen unter den in den Anwendungsgebieten vorherrschenden Bedingungen thermisch und photochemisch stabil sein. Ferner sollten die erfindungsgemäßen Verbindungen möglichst eine breite nematische Phase aufweisen. Als Mesogene sollten sie eine breite nematische Phase in Mischungen mit flüssigkristallinen Cokomponenten ermöglichen sowie hervorragend mit nematischen Basismischungen, insbesondere bei tiefen Temperaturen, mischbar sein.

Lufteinwirkung, und farblos, Auch zeichnen sie sich durch besonders stark positive dielektrische Anisotropien Δε aus, aufgrund derer in der Anwendung in optischen Schaltelementen niedrigere Schwellenspannungen erforderlich sind. Sie besitzen für sich oder in Mischungen einen breiten nematischen Phasenbereich. Darüber hinaus weisen die erfindungsgemäßen Verbindungen einen besonders niedrigen Schmelzpunkt, einen hohen Klärpunkt, sowie gleichzeitig niedere Werte für die Rotationsviskosität γ1 auf. Im Vergleich mit Stoffen aus dem Stand der Technik, die ein ähnliches Eigenschaftsprofil aufweisen, wird insbesondere bei den Verbindungen mit drei Ringsystemen ein besonders niedriger Schmelzpunkt beobachtet. Die Stoffe neigen damit weit weniger zur Kristallisation als entsprechende herkömmliche Verbindungen. Die erfindungsgemäßen Verbindungen können daher z. B. in höherer Konzentration eingesetzt werden.

Mit der Bereitstellung der erfindungsgemäßen Thiophen-Derivate wird ganz allgemein die Palette der flüssigkristallinen Substanzen, die sich unter verschiedenen, anwendungstechnischen Gesichtspunkten zur Herstellung flüssigkristalliner Mischungen eignen, erheblich verbreitert.

Gegenstand der Erfindung sind somit Verbindungen der Verbindungen der Formel I, 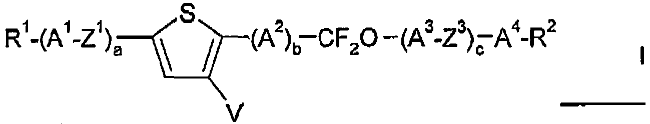 worin
- R¹: H, einen halogenierten oder unsubstituierten Alkylrest mit 1 bis 15 C-Atomen wobei in diesen Resten auch eine oder mehrere CH₂-Gruppen jeweils unabhängig voneinander durch -C≡C-, -CH=CH-, -(CO)O-, -O(CO)-, -(CO)- oder -O- so ersetzt sein können, dass O-Atome nicht direkt miteinander verknüpft sind, bedeutet,
- R²: F, Cl, OCF₃, OCHF₂, OCHFCF₃, OCF₂CHFCF₃, CF₃, CN, SF₅, NCS, NCO, SCN oder OCN bedeutet,
- A¹, A² und A³: jeweils unabhängig voneinander, gleich oder verschieden:
a) trans-1,4-Cyclohexylen oder Cyclohexenylen, worin auch eine oder mehrere nicht benachbarte CH₂-Gruppen durch -O- und/oder -S- ersetzt sein können und worin H durch F substituiert sein kann,
b) 1,4-Phenylen, worin eine oder zwei CH-Gruppen durch N ersetzt sein können und worin auch ein oder mehrere H-Atome gegen Br. Cl, F, CN, Methyl, Methoxy oder eine ein- oder mehrfach fluorierte Methyl- oder Methoxygruppe ersetzt sein können, oder
c) ein Rest aus der Gruppe 1,4-Bicyclo(2,2,2)-octylen, Piperidin-1,4-diyl, Cylcobut-1,3-diyl, Spiro[3.3]heptan-2,6-diyl, 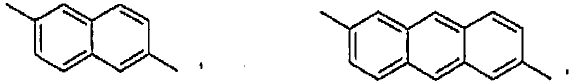 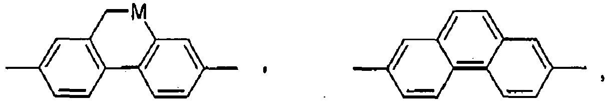 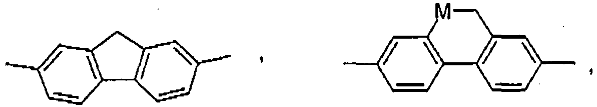 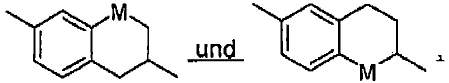 worin ein oder mehrere Wasserstoffatome durch F, CN, SCN, SF₅, CH₂F, CHF₂, CF₃, OCH₂F, OCHF₂ oder OCF₃ ersetzt sein können,
   ein oder mehrere CH-Gruppen durch N ersetzt sein können,
   eine oder mehrere Doppelbindungen durch Einfachbindungen ersetzt sein können,
   M -O-, -S-, -CH₂-, -CHY- oder -CYY¹-,
   und Y und Y¹ Cl, F, CN, OCF₃ oder CF₃ bedeuten,
- A⁴: 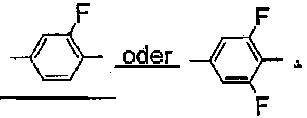
- V: H oder F,
- Z¹ und: Z³ jeweils unabhängig voneinander, gleich oder verschieden,
eine Einfachbindung, -CH₂O-, -(CO)O-, -CF₂O-, -CH₂CH₂CF₂O-, -CF₂CF₂-, -CH₂CF₂-, -CH₂CH₂-, -(CH₂)₄-, -CH=CH-, -CH=CF-, -CF=CF- oder -C≡C-, Wobei asymmetrische Brücken nach beiden Seiten orientiert sein können,
- a: 0, 1 oder 2,
- b: 1, 2 oder 3, und
- c: 0, 1 oder 2
wobei a + b + c ≤ 4 ist,
bedeuten. 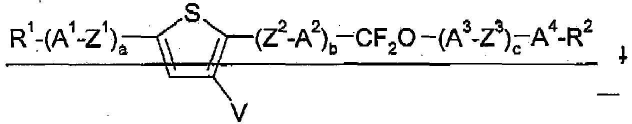 dielektrische und/oder optische Anisotropie eines solchen Dielektrikums zu beeinflussen und/oder um dessen Schwellenspannung und/oder dessen Viskosität zu optimieren.

Die Verbindungen der Formel I sind in reinem Zustand farblos und bilden für sich oder in Mischungen flüssigkristalline Mesophasen in einem für die elektrooptische Verwendung günstig gelegenen Temperaturbereich. Mit den erfindungsgemäßen Verbindungen können sich breite nematischen Phasenbereiche erzielen lassen. In flüssigkristallinen Mischungen erhöhen die erfindungsgemäßen Verbindungen den Klärpunkt und erhöhen die Polarität der Mischung deutlich.

Z¹ und/oder Z³ bedeuten bevorzugt eine Einfachbindung, -CF₂O-, -OCF₂-, -CF₄-, -CH₂O-, -OCH₂- oder -(CO)O-, insbesondere eine Einfachbindung. Z² bedeutet bevorzugt -CH₂CH₂-, -CH=CH-, -C≡C- oder eine Einfachbindung, insbesondere eine Einfachbindung.

A¹, A² und A³ -bedeuten, soweit vorhanden, bevorzugt 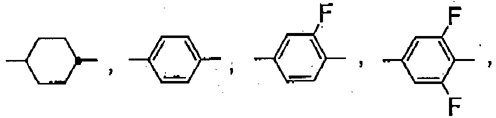 und ferner, 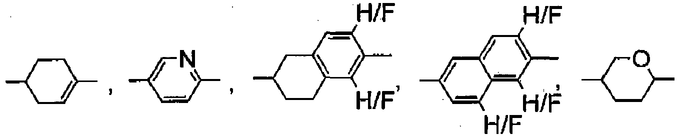 oder

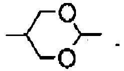

Die Gruppe A¹, soweit vorhanden, bedeutet dabei bevorzugt

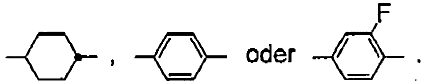

A² bedeutet, soweit vorhanden, bevorzugt

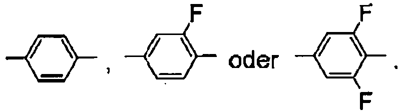

A⁴ bedeutet

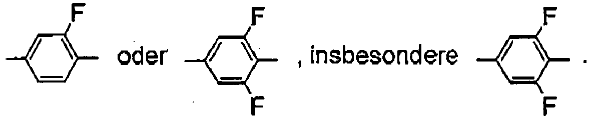

R¹ bedeutet bevorzugt Alkyl, Alkoxy, Alkenyl oder Alkenyloxy mit bis zu 8 Kohlenstoffatomen. R¹ bedeutet besonders bevorzugt geradkettiges Alkyl oder Alkenyl.

Bevorzugt bedeutet R² F, Cl, CN, CF₃ oder OCF₃ und ganz besonders F oder OCF₃ bedeutet.

Vorzugsweise bedeuten R¹ und R² nicht gleichzeitig H.

Besonders bevorzugt sind Verbindungen der Formeln IA 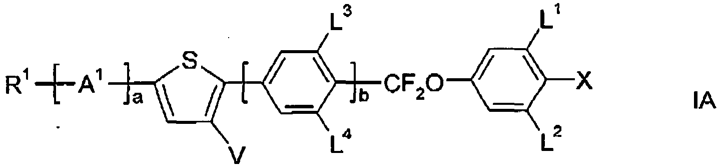 worin
R¹, A¹, X, a, b und V die oben für Formel I angegebenen Bedeutungen haben, sowie
- L¹,: F
- L², L³ und L⁴: H oder F
bedeuten.

Bevorzugt sind Verbindungen der Formel IA worin L¹ ein Fluor bedeutet. b bedeutet bevorzugt 1 oder 2, insbesondere 1. V ist bevorzugt H. L³ ist bevorzugt F. a + b ist bevorzugt 1 oder 2. Ganz besonders bevorzugt ist b 1, und a ist bevorzugt 0. Besonders bevorzugt sind 2, 3 oder vier der Gruppen L¹ bis L⁴ ein Fluor.

In einer weiteren Ausführungsform der Erfindung sind Verbindungen der Formel bevorzugt, worin V ein F bedeutet. Die Verbindungen besitzen eine besonders hohe dielektrische Anisotropie.

Besonders bevorzugte Verbindungen der Formel I sind die Verbindungen der Formeln I1 bis I6, 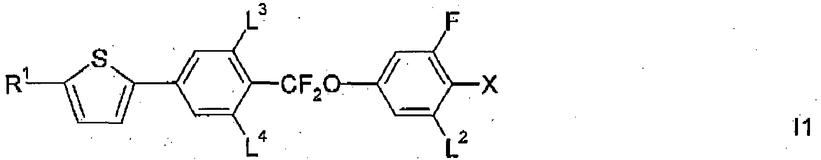 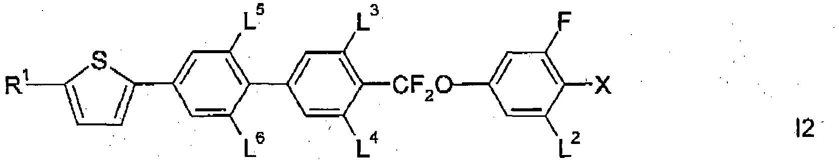 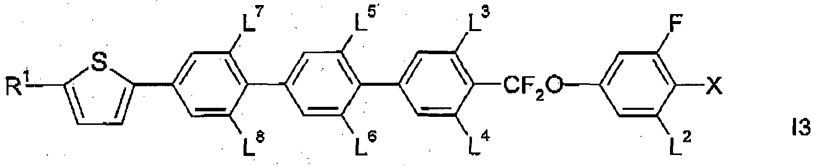 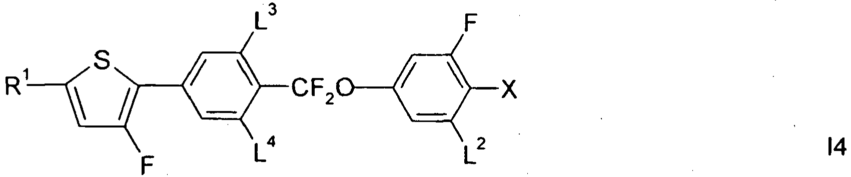 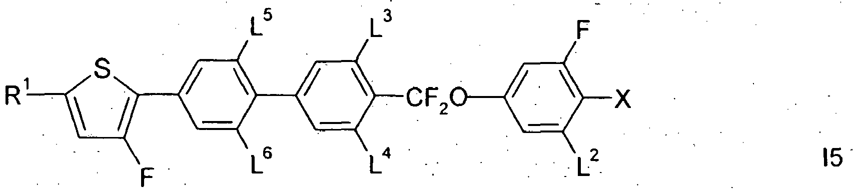 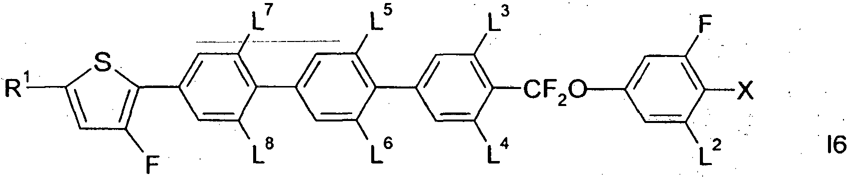 worin R¹ und X die oben angegebenen Bedeutungen haben. L², L³, L⁴, L⁵, L⁶, L⁷ und L⁸ bedeuten unabhängig voneinander H oder F.
Besonders bevorzugt bedeuten unabhängig voneinander L², L³ und L⁴ einen Rest F.
L⁵ und L⁷ bedeuten bevorzugt unabhängig voneinander H.

Beispiele für besonders bevorzugte Verbindungen sind die folgenden:

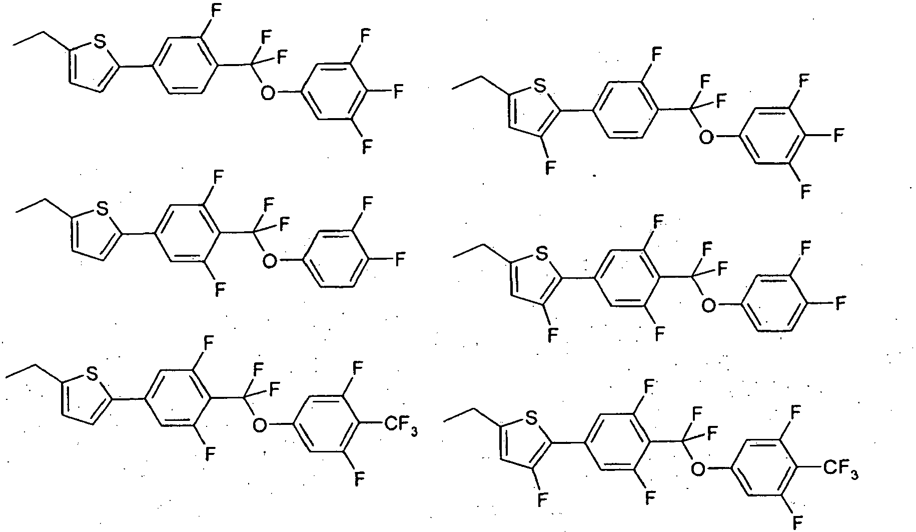

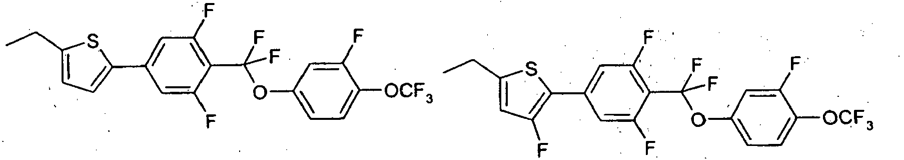

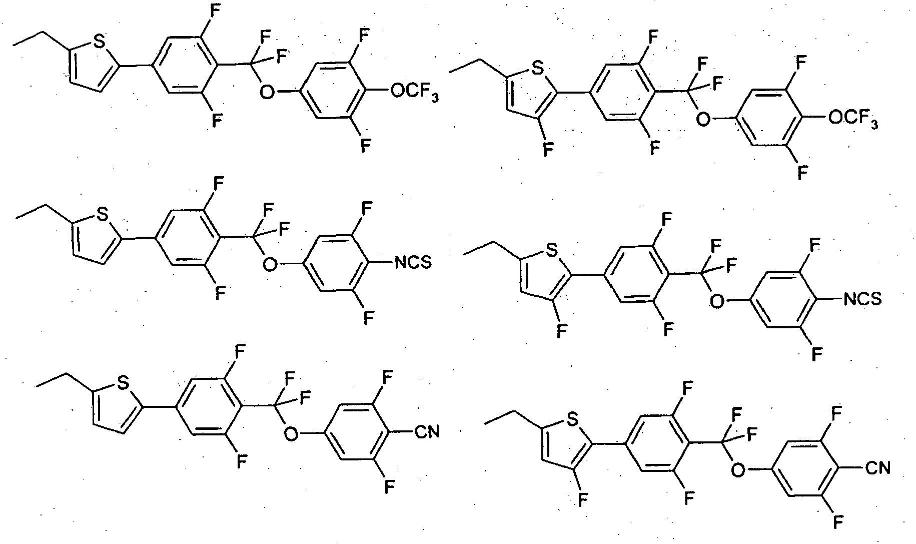

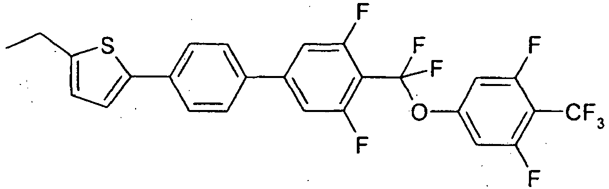

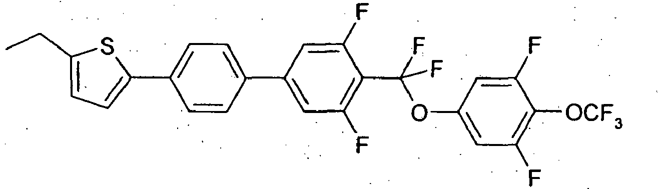

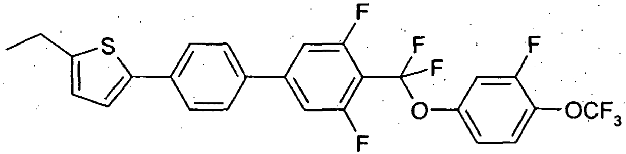

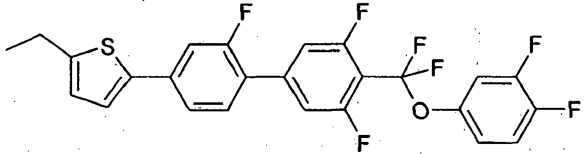

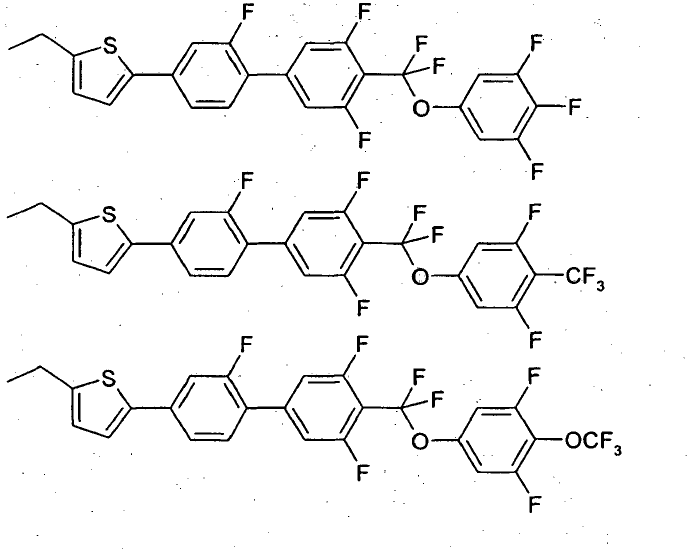

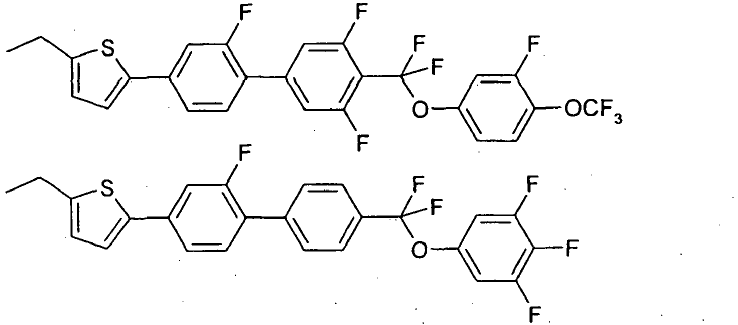

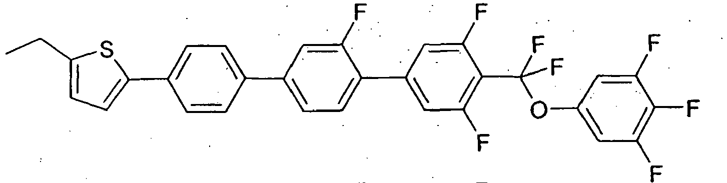

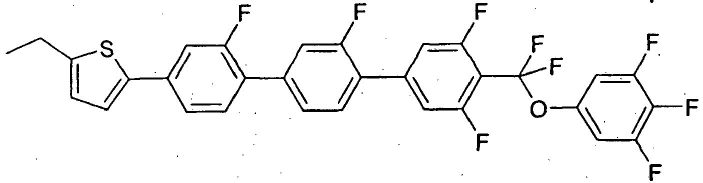

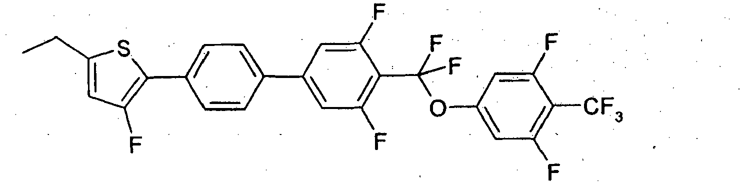

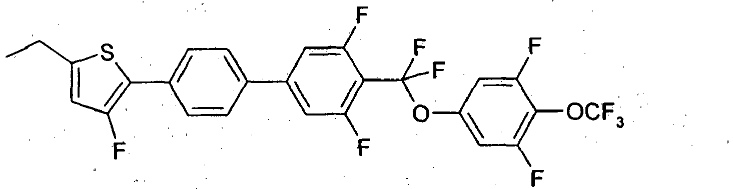

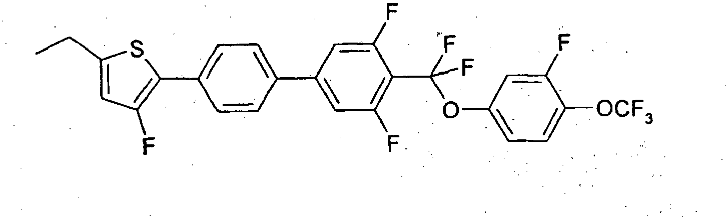

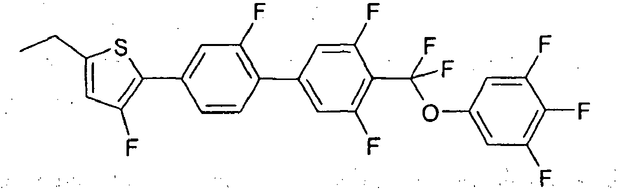

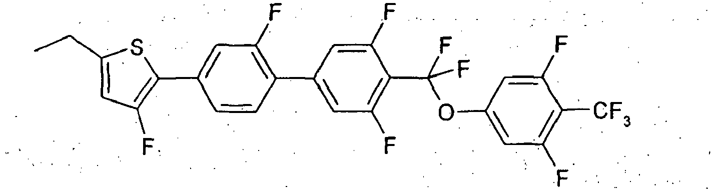

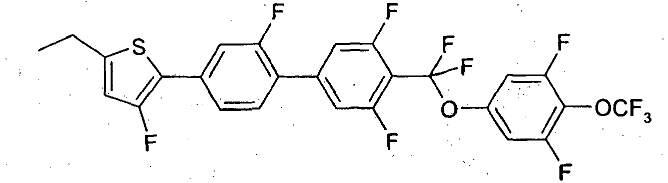

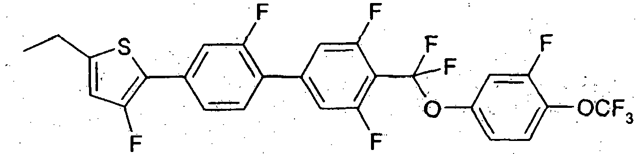

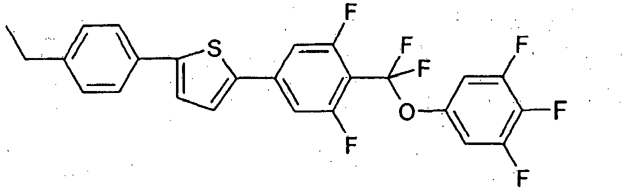

Bei Verbindungen, die in Diastereomeren auftreten können, sind sowohl die Reinsubstanzen als auch jedes Mischungsverhältnis der Isomeren umfasst und jeweils als geeignete Mischungskomponente anzusehen.

Die Verbindungen der Formel I werden nach an sich bekannten Methoden dargestellt, wie sie in der Literatur (z.B. in den Standardwerken wie Houben-Weyl, Methoden der Organischen Chemie, Georg-Thieme-Verlag, Stuttgart) beschrieben sind und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Verbindungen der Formel I können vorteilhaft wie an den folgenden beispielhaften Synthesen ersichtlich hergestellt werden (Schema 1 und 2):

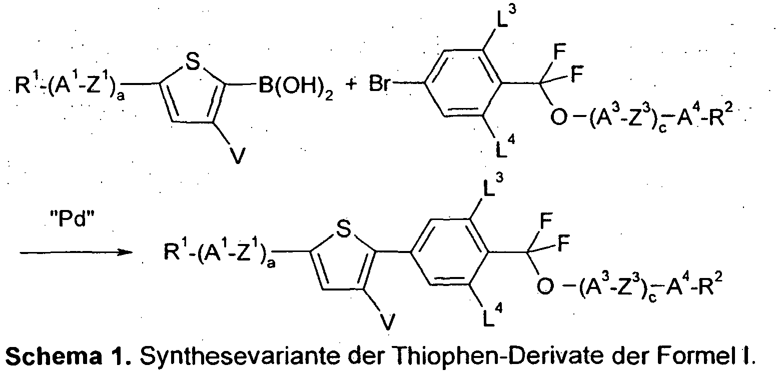

Der Synthese laut Schema 1 liegt eine Suzuki-Kopplung zugrunde.

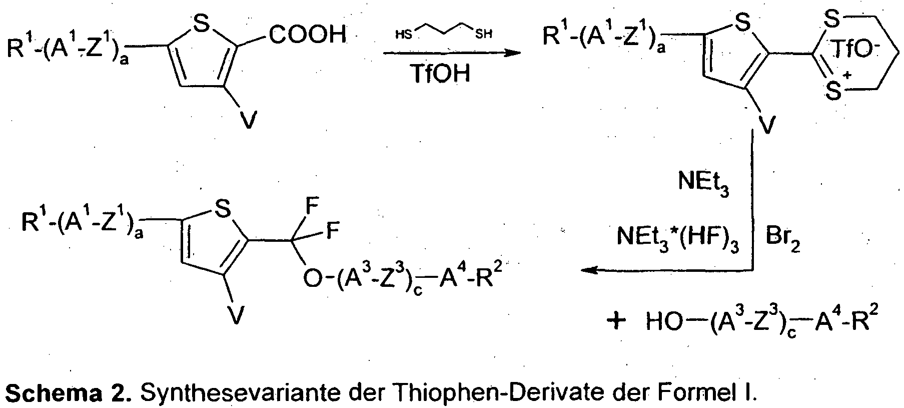

Die Synthese in Schema 2 beruht auf einer bekannten Synthese der Gruppe -CF₂O- wie z. B. in der Druckschrift EP 1341742 A1 angegeben.

Die unbeteiligten Gruppen der Formeln in Schema 1 und 2 lassen sich variieren, so weit es die Definitionen der Verbindungen der Formel I nahe legen. Entsprechende Ausgangsprodukte lassen sich in der Regel vom Fachmann ohne weiteres herstellen. So lassen sich die Verbindungen der Formeln I oder IA herstellen.

Die Erfindung hat daher auch ein Verfahren zur Herstellung von Verbindungen der Formel I zum Gegenstand:
Ein Verfahren zur Herstellung von Verbindungen der Formel I, worin V Wasserstoff oder Fluor bedeutet, ist dadurch gekennzeichnet, dass es einen Verfahrensschritt umfasst, worin ein 2-substituiertes Thiophen der Formeln IIa oder IIb 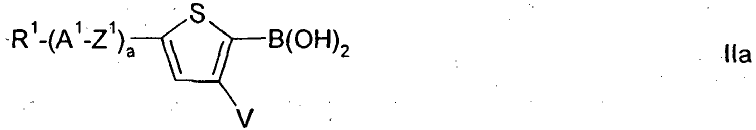 oder ein entsprechendes Boronat mit der Endgruppe -B(OH)₃⁻ oder -B(OH)₃M,
   worin M einen einwertigen ionischen Rest als Gegenion zum Boronat (insbesondere Alkalimetallionen wie Na⁺, K⁺, etc.) bedeutet,
   oder

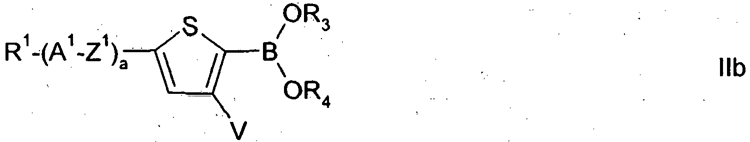

- worin R¹, A¹, Z¹, V und a: wie in Anspruch 1 definiert sind, und
- R³, R⁴: Alkyl mit 1-12 C-Atomen oder R³+R⁴ zusammen auch ein C₁-C₆-Alkylen, insbesondere der Formeln -CH₂-(CH₂)ₚ-CH₂- und -C(CH₃)₂C(CH₃)₂-, oder 1,2-Phenylen bedeuten, wobei R³, R⁴ und R³+R⁴ auch substituiert sein können, insbesondere durch C₁-C₆-Alkyl, F, Cl, C₁-C₆-Alkoxy, und wobei p 0 oder 1 ist,
mit einer Verbindung der Formel III

Hal-(Z²-A²)_{b}-CF₂O-(A³-Z³)_{c}-A⁴-R² III

worin Z², Z³, A², A³, A⁴, b, c und R² wie in Anspruch 1 definiert sind, und
- Hal: OSO₂CF₃, Cl, Br oder I bedeutet,
in Gegenwart eines Übergangsmetallkatalysators, bevorzugt eines Palladiumkomplexes, zur Reaktion gebracht wird. Bei den Komplexen handelt es sich bevorzugt um Palladium(II)-Komplexe, insbesondere um Bis(triphenylphosphin)palladium(II)chlorid. Hal bedeutet bevorzugt Chlor oder Brom, insbesondere Brom. In Formel III bedeutet bevorzugt b 1 oder 2 und Z² eine Einfachbindung. Der Rest Hal ist bevorzugt direkt mit einem Rest A² verbunden A² bedeutet bevorzugt ein aromatisches Ringsystem. Weiterhin sind die für die Verbindungen der Formel I angegebenen Unterformen bevorzugt.

Weitere bevorzugte Verfahrensvarianten lassen sich den Beispielen entnehmen, deren Details - auch verallgemeinert nach allgemeiner Fachkenntnis - repräsentativ für bevorzugte Ausführungsformen des erfindungsgemäßen Verfahren und seiner Produkte sind.

Gegenstand der Erfindung sind auch flüssigkristalline Medien enthaltend eine oder mehrere der erfindungsgemäßen Verbindungen der Formel I. Die flüssigkristallinen Medien enthalten wenigstens zwei Komponenten, vorzugsweise eine oder mehrere Verbindungen der Formel I und wenigstens eine weitere Verbindung, die vorzugsweise mesogen ist Man erhält die erfindungsgemäßen Medien vorzugsweise indem man die Komponenten miteinander vermischt. Ein erfindungsgemäßes Verfahren zur Herstellung eines flüssigkristallinen Mediums ist daher dadurch gekennzeichnet, dass man mindestens eine Verbindung der Formel I mit mindestens einer weiteren mesogenen Verbindung vermischt und gegebenenfalls Additive zugibt.

Die erzielbaren Kombinationen aus Klärpunkt, Viskosität bei tiefer Temperatur, thermischer/UV-Stabilität und hoher dielektrischer und optischer Anisotropie übertreffen bisherige Materialien aus dem Stand der Technik. Es werden gleichzeitig niedrige Schwellenspannungen, gute VHR-Werte (VHR: 'voltage holding ratio') und gute Tieftemperaturbeständigkeit realisiert.

Die erfindungsgemäßen, flüssigkristallinen Medien enthalten vorzugsweise neben einer oder mehreren erfindungsgemäßen Verbindungen als weitere Bestandteile 2 bis 40, besonders bevorzugt 4 bis 30 Komponenten. Insbesondere enthalten diese Medien neben einer oder mehreren erfindungsgemäßen Verbindungen 7 bis 25 Komponenten.

Die Herstellung der erfindungsgemäßen Flüssigkristallmedien erfolgt in an sich üblicher Weise. In der Regel wird die gewünschte Menge der in geringerer Menge verwendeten Komponenten in der den Hauptbestandteil ausmachenden Komponenten gelöst, vorzugsweise bei erhöhter Temperatur. Es ist auch möglich, Lösungen der Komponenten in einem organischen Lösungsmittel, z.B. in Aceton, Chloroform oder Methanol, zu mischen und das Lösungsmittel nach Durchmischung wieder zu entfernen, beispielsweise durch Destillation. Weiterhin ist es möglich, die Mischungen auf andere herkömmliche Arten, z. B. durch Verwendung von Vormischungen, z.B. Homologen-Mischungen oder unter Verwendung von sogenannten "Multi-Bottle"-Systemen herzustellen.

Die Dielektrika können auch weitere, dem Fachmann bekannte und in der Literatur beschriebene Zusätze enthalten. Beispielsweise können 0 bis 15 %, vorzugsweise 0 bis 10 %, pleochroitische Farbstoffe, chirale Dotierstoffe, Stabilisatoren oder Nanopartikel zugesetzt werden. Die einzelnen, zugesetzten Verbindungen werden in Konzentrationen von 0,01 bis 6 %, vorzugsweise von 0,1 bis 3 %, eingesetzt. Dabei werden jedoch die Konzentrationsangaben der übrigen Bestandteile der Flüssigkristallmischungen, also der flüssigkristallinen oder mesogenen Verbindungen, ohne Berücksichtigung der Konzentration dieser Zusatzstoffe angegeben. Die erfindungsgemäßen Flüssigkristallmedien ermöglichen eine bedeutende Erweiterung des zur Verfügung stehenden Parameterraumes.

Gegenstand der Erfindung sind auch elektrooptische Anzeigen (insbesondere TFT-Anzeigen mit zwei planparallelen Trägerplatten, die mit einer Umrandung eine oder mehrere Zellen bilden, integrierten nicht-linearen Elementen zur Schaltung einzelner Bildpunkte auf den Trägerplatten und einer in der Zelle befindlichen nematischen Flüssigkristallmischung mit positiver dielektrischer Anisotropie und hohem spezifischem Widerstand), die derartige Medien enthalten sowie die Verwendung dieser Medien für elektrooptische Zwecke.

Die Gesamtmenge an Verbindungen der Formeln I in den erfindungsgemäßen Gemischen ist nicht kritisch. Die Gemische können daher eine oder mehrere weitere Komponenten enthalten zwecks Optimierung verschiedener Eigenschaften. Der beobachtete Effekt auf die Ansprechzeiten und die Schwellenspannung ist jedoch in der Regel umso größer je höher die Gesamtkonzentration an Verbindungen der Formel I ist.

Besonders bevorzugte erfindungsgemäße LC-Medien werden im Folgenden genannt:

LC-Medium, welches zusätzlich eine oder mehrere Verbindungen der Formel II und/oder III enthält: 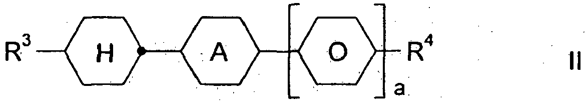 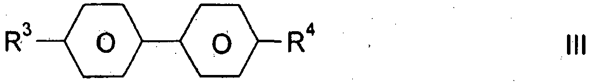 worin
- Ring A: 1,4-Phenylen oder trans-1,4-Cyclohexylen bedeutet,
- a: 0 oder 1 ist,
- R³: jeweils unabhängig voneinander Alkyl mit 1 bis 9 C-Atomen oder Alkenyl mit 2 bis 9 C-Atomen bedeutet, vorzugsweise Alkenyl mit 2 bis 9 C-Atomen, und
- R⁴: jeweils unabhängig voneinander einen unsubstituierten oder halogenierten Alkylrest mit 1 bis 12 C-Atomen, wobei auch eine oder zwei nicht benachbarte CH₂-Gruppen durch -O-, -CH=CH-, -CH=CF-, -(CO)-, -O(CO)- oder -(CO)O- so ersetzt sein können, dass O-Atome nicht direkt miteinander verknüpft sind, und vorzugsweise Alkyl mit 1 bis 12 C-Atomen oder Alkenyl mit 2 bis 9 C-Atomen bedeutet.

Die Verbindungen der Formel II sind vorzugsweise ausgewählt aus der Gruppe bestehend aus folgenden Formeln: 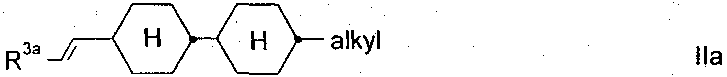 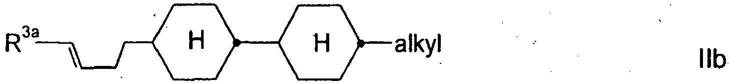 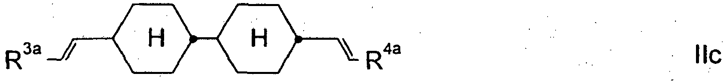 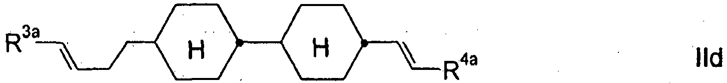 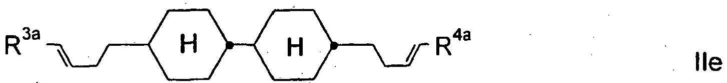 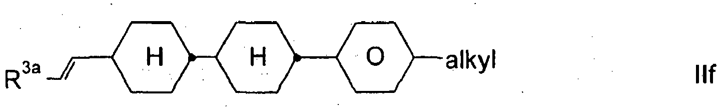 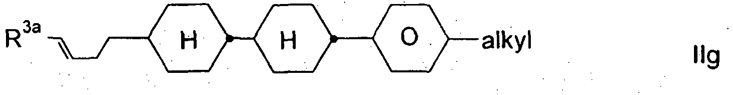 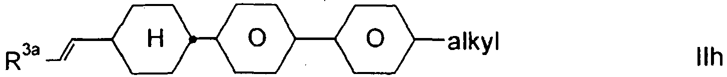 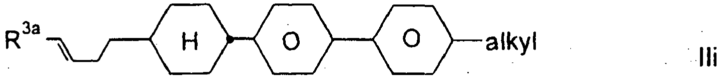 worin R^{3a} und R^{4a} jeweils unabhängig voneinander H, CH₃, C₂H₅ oder C₃H₇, bedeuten, und "alkyl" eine geradkettige Alkylgruppe mit 1 bis 8, vorzugsweise 1, 2, 3, 4 oder 5 C-Atomen bedeutet. Besonders bevorzugt sind Verbindungen der Formel IIa und IIf, insbesondere solche, worin R^{3a} H oder CH₃, vorzugsweise H, bedeutet, und Verbindungen der Formel IIc, insbesondere solche, worin R^{3a} und R^{4a} H, CH₃ oder C₂H₅ bedeuten.

Die Verbindungen der Formel III sind vorzugsweise ausgewählt aus der Gruppe bestehend aus den folgenden Formeln: 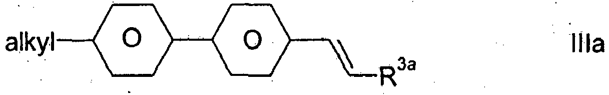 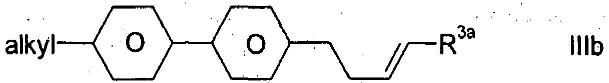 worin "alkyl" und R^{3a} die oben angegebenen Bedeutungen haben und R^{3a} vorzugsweise H oder CH₃ bedeutet. Besonders bevorzugt sind Verbindungen der Formel IIIb;
LC-Medium, welches zusätzlich eine oder mehrere Verbindungen ausgewählt aus der Gruppe bestehend aus den folgenden Formeln enthält: 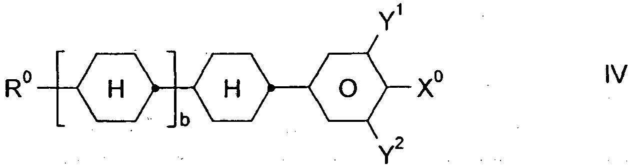 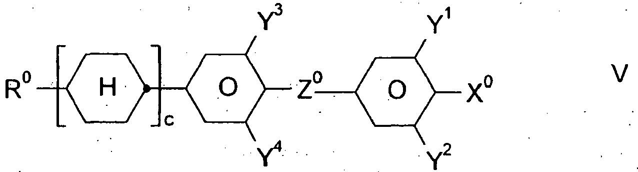 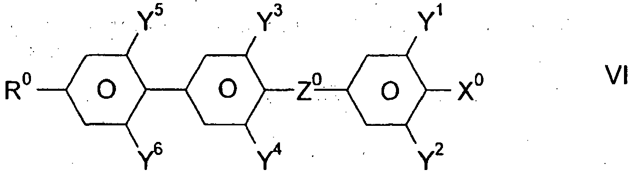 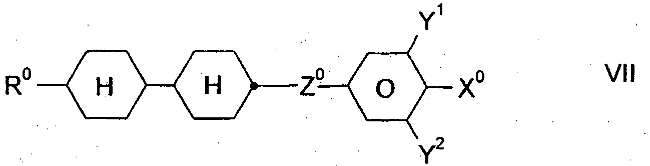 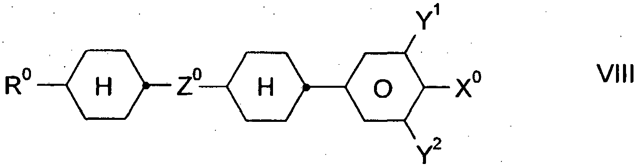 worin
- R⁰: einen Alkyl- oder Alkoxyrest mit 1 bis 15 C-Atomen, wobei in diesen Resten auch eine oder mehrere CH₂-Gruppen jeweils unabhängig voneinander durch -C≡C-, -CF₂O-, -CH=CH-, 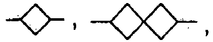 -O-, -(CO)O- oder -O(CO)- so ersetzt sein können, dass O-Atome nicht direkt miteinander verknüpft sind, und worin auch ein oder mehrere H-Atome durch Halogen ersetzt sein können,
- X⁰: F, Cl, CN, SF₅, SCN, NCS, einen halogenierten Alkylrest, halogenierten Alkenylrest, halogenierten Alkoxyrest oder halogenierten Alkenyloxyrest mit jeweils bis zu 6 C-Atomen,
- Y¹⁻⁶: jeweils unabhängig voneinander H oder F, Z⁰ -C₂H₄-, =(CH₂)₄-, -CH=CH-, -CF=CF-, -C₂F₄-, -CH₂CF₂-, -CF₂CH₂-, -CH₂O-, -OCH₂-, -COO-, -CF₂O- oder -OCF₂-, in den Formeln V und VI auch eine Einfachbindung, und
b und c jeweils unabhängig voneinander 0 oder 1
bedeuten.

In den Verbindungen der Formel IV bis VIII bedeutet X⁰ vorzugsweise F oder OCF₃, ferner OCHF₂, CF₃, CF₂H, Cl, OCH=CF₂. R⁰ ist vorzugsweise geradkettiges Alkyl oder Alkenyl mit jeweils bis zu 6 C-Atomen.

Die Verbindungen der Formel IV sind vorzugsweise ausgewählt aus der Gruppe bestehend aus den folgenden Formeln: 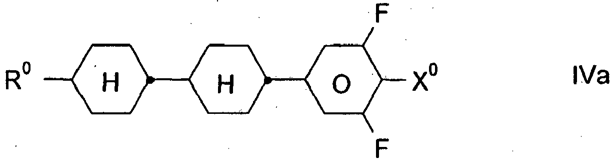 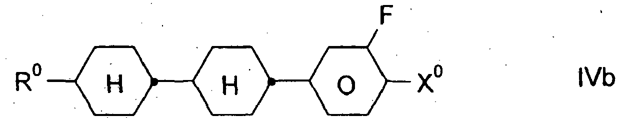 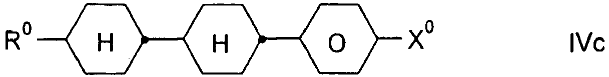 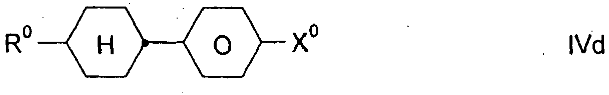 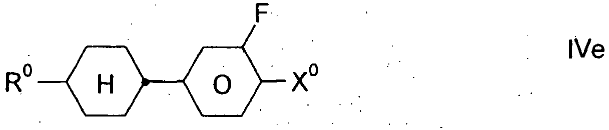 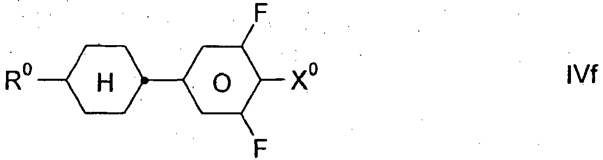 worin R⁰ und X⁰ die oben angegebenen Bedeutungen haben.

Vorzugsweise bedeutet in Formel IV R⁰ Alkyl mit 1 bis 8 C-Atomen und X⁰ F, Cl, OCHF₂ oder OCF₃, ferner OCH=CF₂. In der Verbindung der Formel IVb bedeutet R⁰ vorzugsweise Alkyl oder Alkenyl. In der Verbindung der Formel IVd bedeutet X⁰ vorzugsweise Cl, ferner F.

Die Verbindungen der Formel V sind vorzugsweise ausgewählt aus der Gruppe bestehend aus den folgenden Formeln: 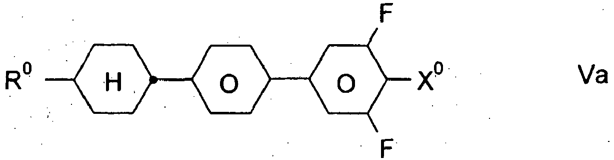 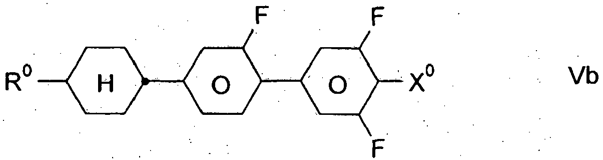 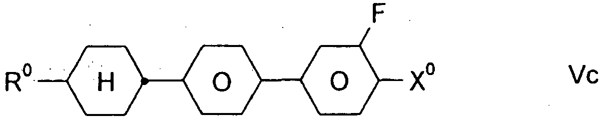 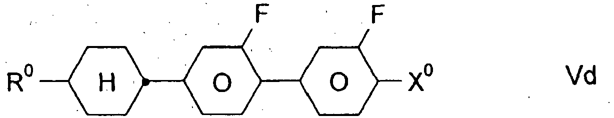 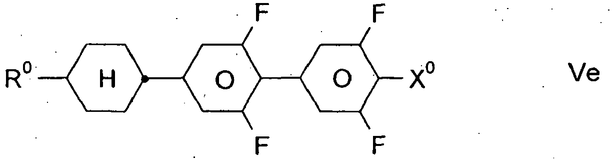 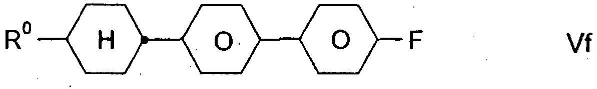 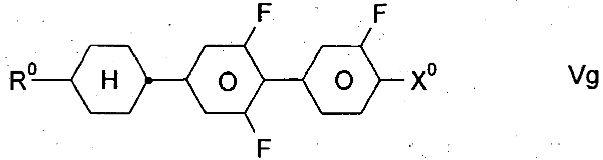 worin R⁰ und X⁰ die oben angegebenen Bedeutungen haben. Vorzugsweise bedeutet R⁰ in Formel V Alkyl mit 1 bis 8 C-Atomen und X⁰ F;

LC-Medium, welches eine oder mehrere Verbindungen der Formel VI-1 enthält: 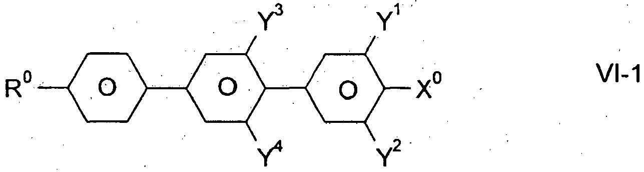 besonders bevorzugt solche ausgewählt aus der Gruppe bestehend aus den folgenden Formeln: 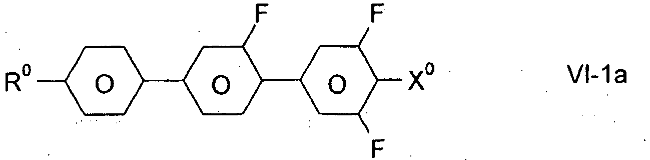 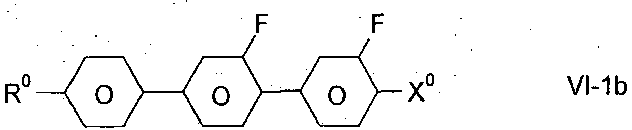 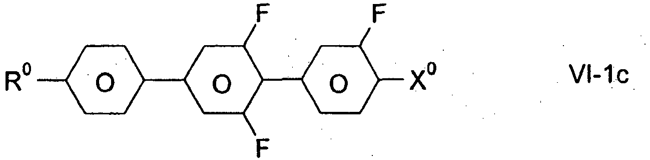 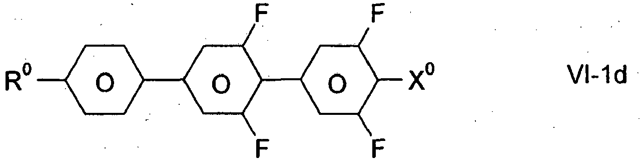 worin R⁰ und X⁰ die oben angegebenen Bedeutungen haben. Vorzugsweise bedeutet R⁰ in Formel VI Alkyl mit 1 bis 8 C-atomen und X⁰ F, ferner OCF₃.

LC-Medium, welches eine oder mehrere Verbindungen der Formel VI-2 enthält: 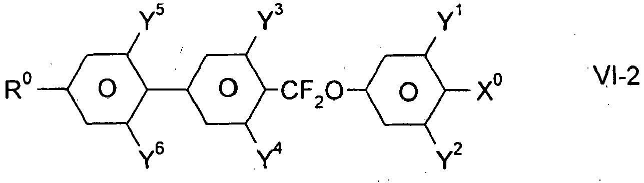 besonders bevorzugt solche ausgewählt aus der Gruppe bestehend aus den folgenden Formeln: 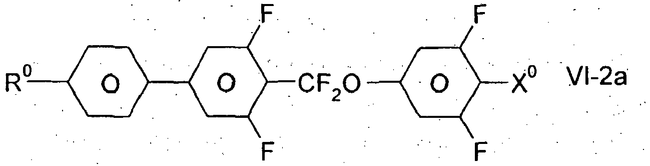 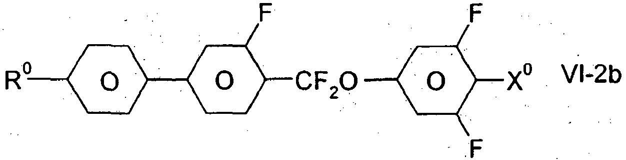 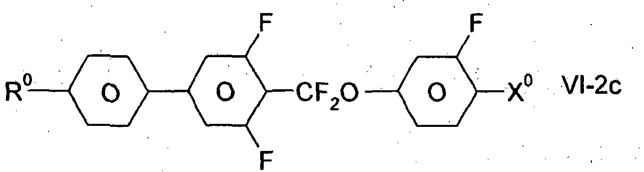 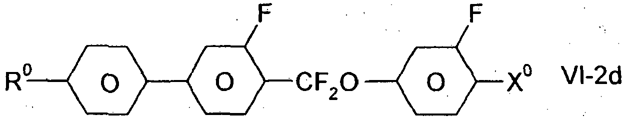 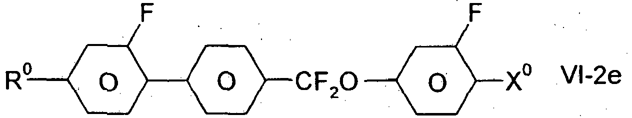 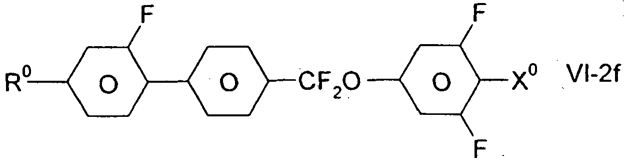 worin R⁰ und X⁰ die oben angegebenen Bedeutungen haben.

Vorzugsweise bedeutet R⁰ in Formel VI Alkyl mit 1 bis 8 C-Atomen und X⁰ F;

LC-Medium, welches vorzugsweise eine oder mehrere Verbindungen der Formel VII, worin Z⁰ -CF₂O-, -CH₂CH₂ oder -(CO)O- bedeutet, enthält, besonders bevorzugt solche ausgewählt aus der Gruppe bestehend aus den folgenden Formeln: 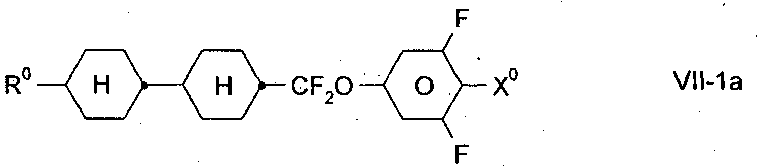 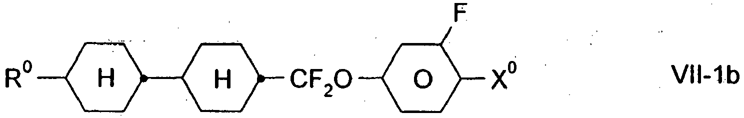 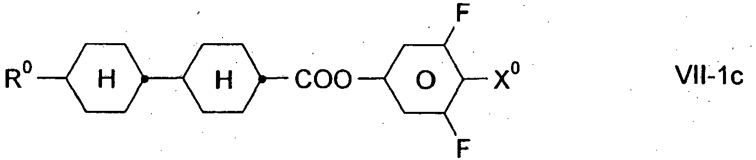 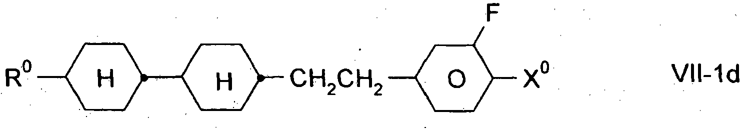 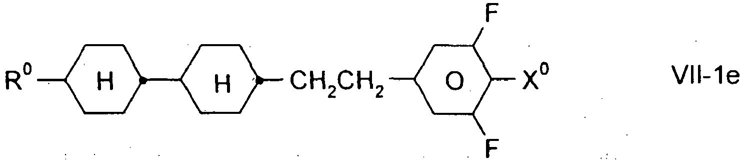 worin R⁰ und X⁰ die oben angegebenen Bedeutungen haben. Vorzugsweise bedeutet R⁰ in Formel VII Alkyl mit 1 bis 8 C-Atomen und X⁰ F, ferner OCF₃.

Die Verbindungen der Formel VIII sind vorzugsweise ausgewählt aus der Gruppe bestehend aus den folgenden Formeln: 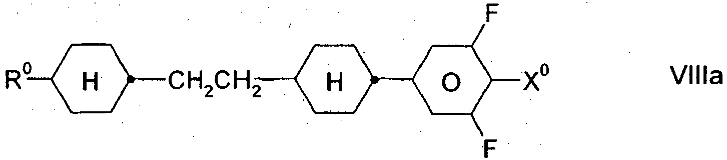 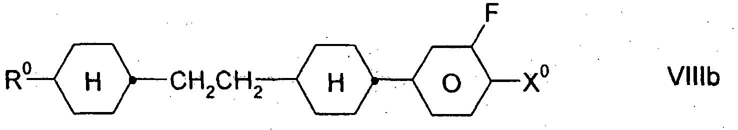 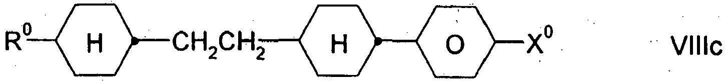 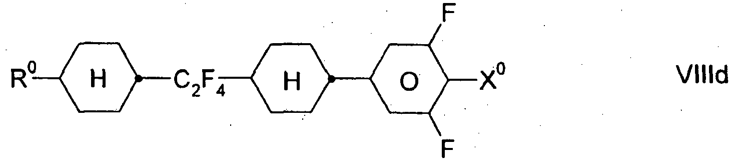 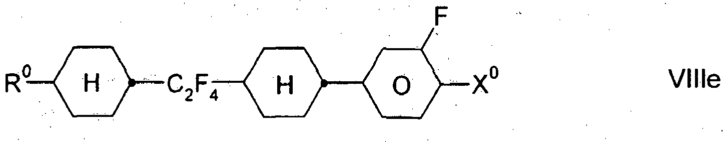 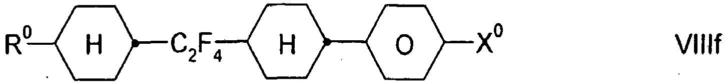 worin R⁰ und X⁰ die oben angegebenen Bedeutungen haben. Vorzugsweise bedeutet R⁰ einen geradkettigen Alkylrest mit 1 bis 8 C-Atomen. X⁰ bedeutet vorzugsweise F.

LC-Medium, welches zusätzlich eine oder mehrere Verbindungen der folgenden Formel enthält: 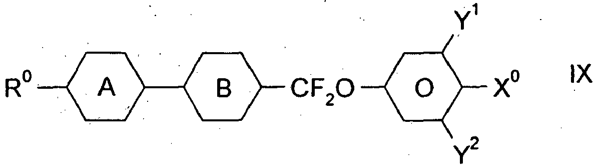 worin R⁰, X⁰, Y¹ und Y² die oben angegebene Bedeutung besitzen, und 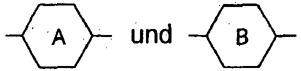 jeweils unabhängig voneinander 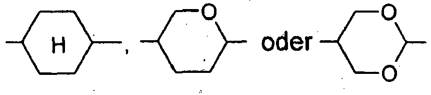 bedeuten, wobei die Ringe A und B nicht beide gleichzeitig Cyclohexylen bedeuten;

Die Verbindungen der Formel IX sind vorzugsweise ausgewählt aus der Gruppe bestehend aus den folgenden Formeln: 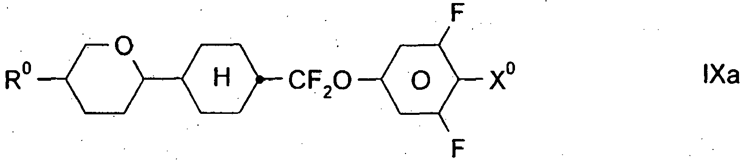 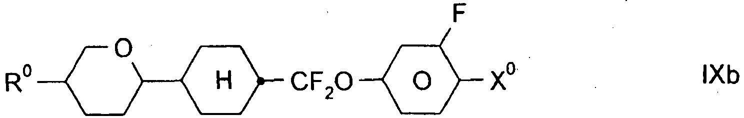 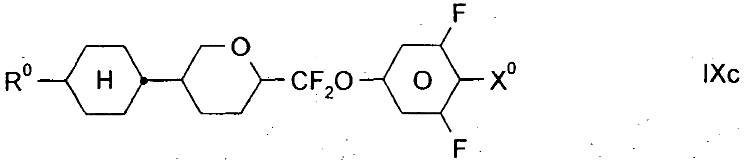 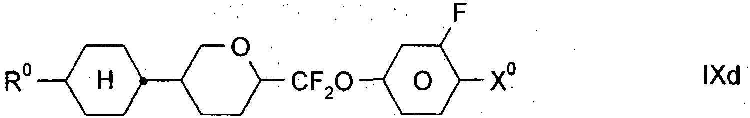 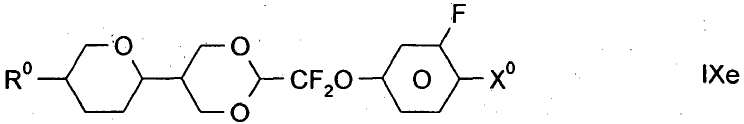 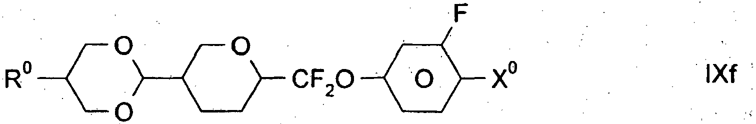 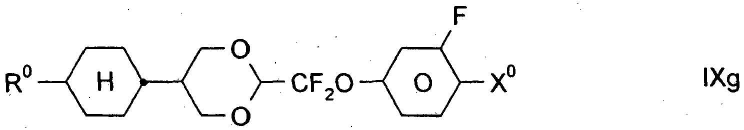 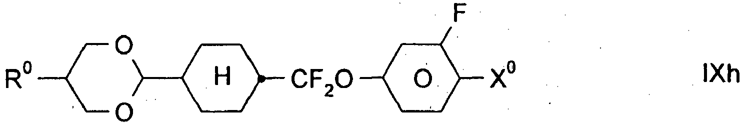 worin R⁰ und X⁰ die oben angegebenen Bedeutungen haben. Vorzugsweise bedeutet R⁰ Alkyl mit 1 bis 8 C-Atomen und X⁰ F. Besonders bevorzugt sind Verbindungen der Formel IXa; LC-Medium, welches zusätzlich eine oder mehrere Verbindungen ausgewählt aus der Gruppe bestehend aus den folgenden Formeln enthält: 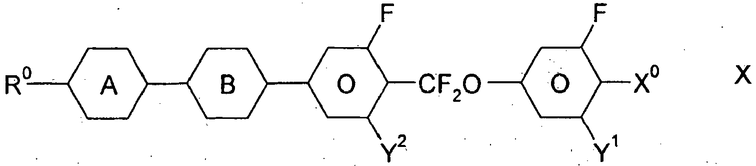 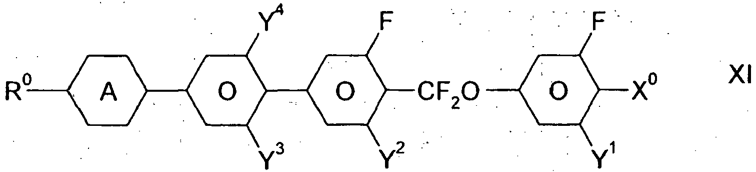 worin R⁰, X⁰ und Y¹⁻⁴ die oben angegebenen Bedeutungen besitzen, und 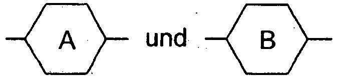 jeweils unabhängig voneinander 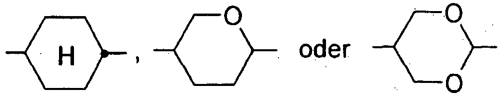 bedeuten;

Die Verbindungen der Formeln X und XI sind vorzugsweise ausgewählt aus der Gruppe bestehend aus den folgenden Formeln: 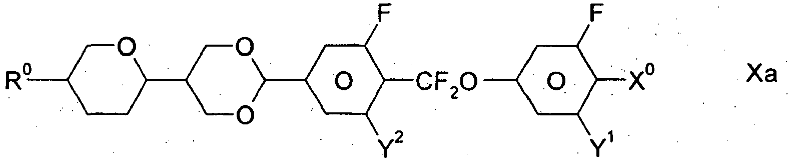 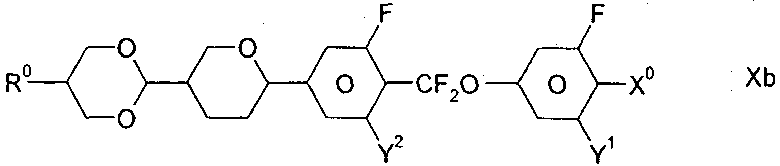 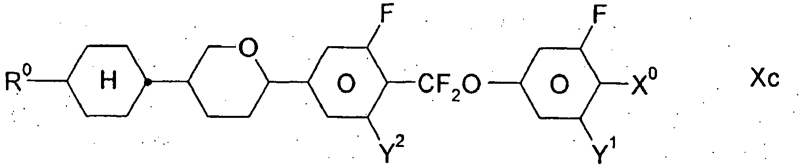 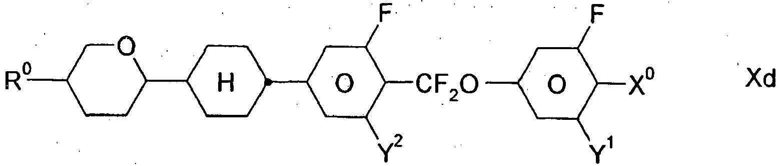 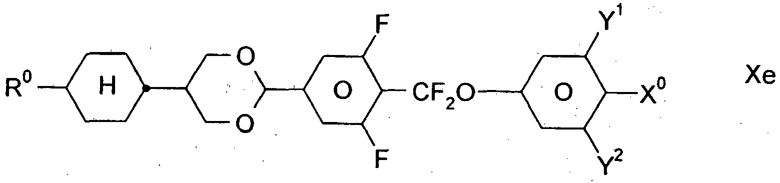 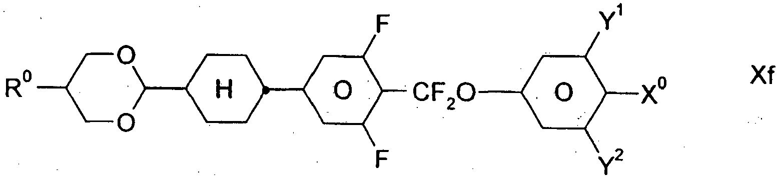 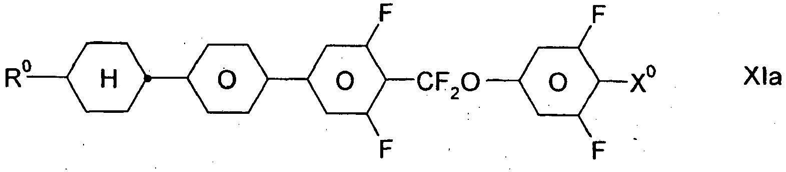 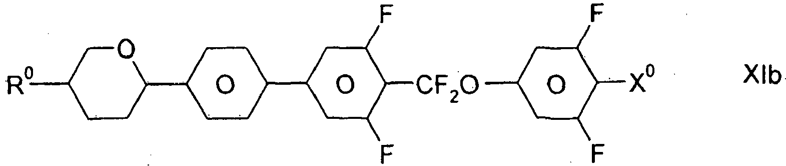 worin R⁰, und X⁰ die oben angegebenen Bedeutungen haben. Vorzugsweise bedeutet R⁰ Alkyl mit 1 bis 8 C-Atomen und/oder X⁰ F. Besonders bevorzugte Verbindungen sind solche, worin Y¹ F und Y² H oder F, vorzugsweise F, bedeuten;

LC-Medium, welches zusätzlich eine oder mehrere Verbindungen der folgenden Formel XII enthält: 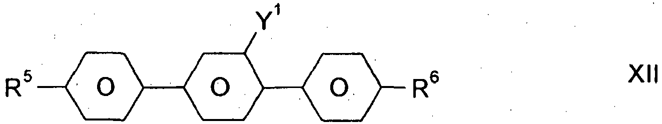 worin R⁵ und R⁶ jeweils unabhängig voneinander n-Alkyl, Alkoxy, Oxaalkyl, Fluoralkyl oder Alkenyl mit jeweils bis zu 9 C-Atomen bedeuten, und vorzugsweise jeweils unabhängig voneinander Alkyl mit 1 bis 7 C-Atomen oder Alkenyl mit 2 bis 7 C-Atomen bedeuten. Y¹ bedeutet H oder F.

Bevorzugte Verbindungen der Formel XII sind solche ausgewählt aus der Gruppe bestehend aus den folgenden Formeln: 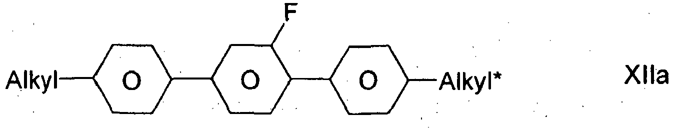 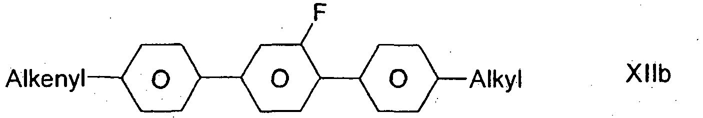 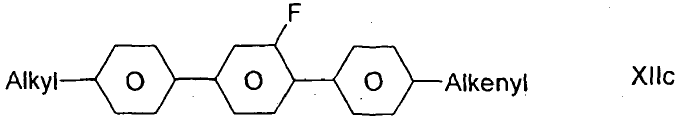 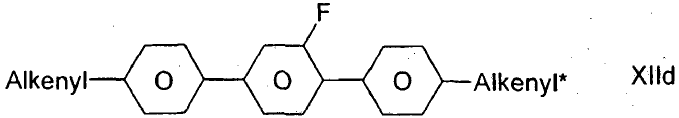 worin
- Alkyl und Alkyl*: jeweils unabhängig voneinander einen geradkettigen Alkylrest mit 1 bis 6 C-Atomen, und
- Alkenyl und Alkenyl*: jeweils unabhängig voneinander einen geradkettigen Alkenylrest mit 2 bis 6 C-Atomen
bedeuten.

Ganz besonders bevorzugt sind Verbindungen der folgenden Formel 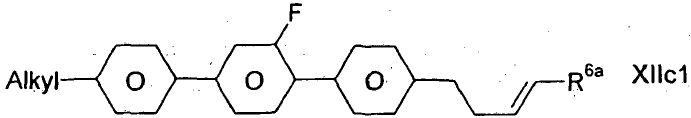 worin Alkyl die oben angegebene Bedeutung hat und R^{6a} H oder CH₃ bedeutet.

LC-Medium, welches zusätzlich eine oder mehrere Verbindungen ausgewählt aus der Gruppe bestehend aus den folgenden Formeln enthält: 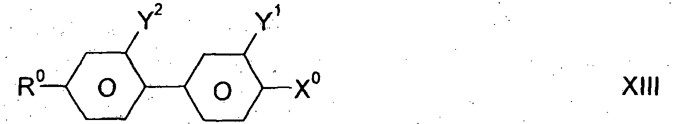 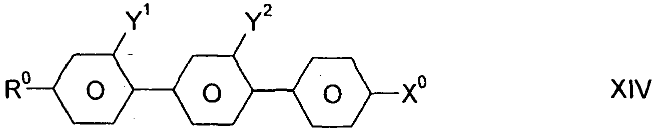 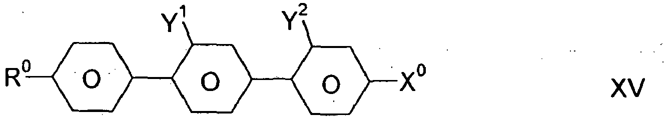 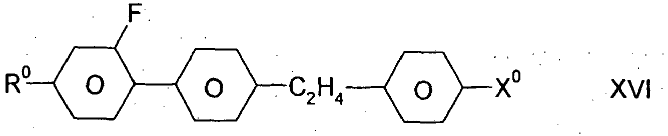 worin R⁰, X⁰, Y¹ und Y² die oben angegebenen Bedeutungen haben. Vorzugsweise bedeutet R⁰ Alkyl mit 1 bis 8 C-Atomen und X⁰ F oder Cl;

Die Verbindungen der Formeln XIII und XIV sind vorzugsweise ausgewählt aus der Gruppe bestehend aus den folgenden Formeln: 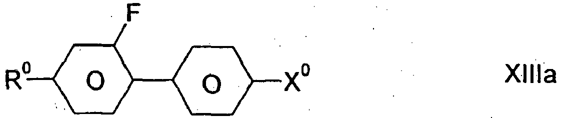 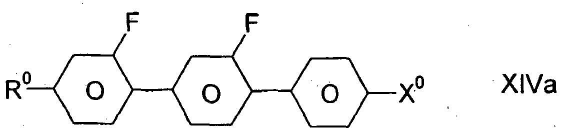 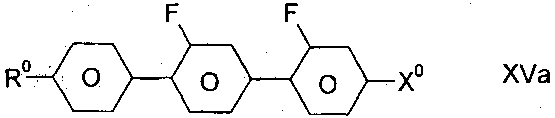 worin R⁰ und X⁰ die oben angegebenen Bedeutungen haben. Vorzugsweise bedeutet R⁰ Alkyl mit 1 bis 8 C-Atomen. In den Verbindungen der Formel XIII bedeutet X⁰ vorzugsweise F oder Cl.

LC-Medium, welches zusätzlich eine oder mehrere Verbindungen der Formel D1 und/oder D2 enthält: 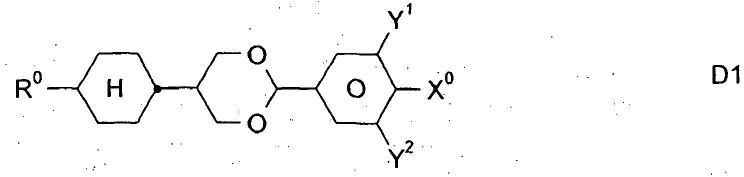 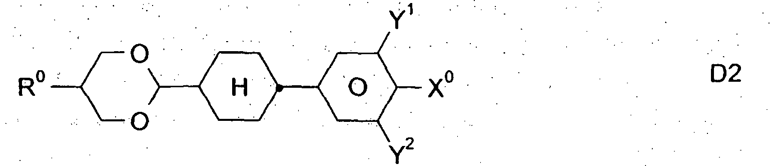 worin Y¹, Y², R⁰ und X⁰ die oben angegebene Bedeutung besitzen. Vorzugsweise bedeutet R⁰ Alkyl mit 1 bis 8 C-Atomen und X⁰ F. Besonders bevorzugt sind Verbindungen der folgenden Formeln: 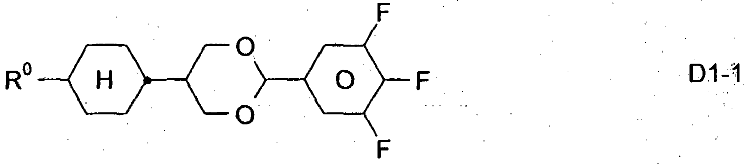 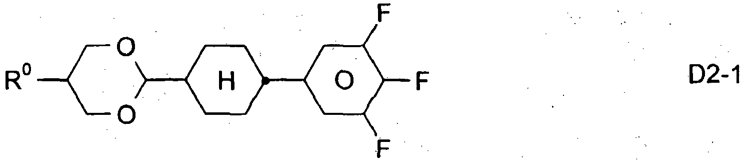 worin R⁰ die oben angegebenen Bedeutungen hat und vorzugsweise geradkettiges Alkyl mit 1 bis 6 C-Atomen, insbesondere C₂H₅, n-C₃H₇ oder n-C₅H₁₁ bedeutet.

LC-Medium, welches zusätzlich eine oder mehrere Verbindungen der folgenden Formeln enthält: 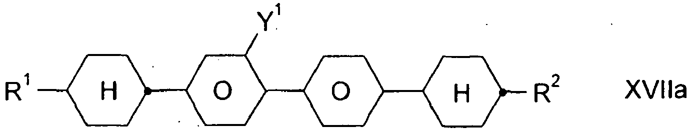 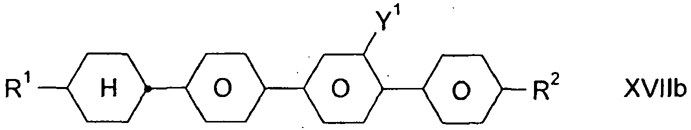 worin Y¹, R¹ und R² die oben angegebene Bedeutung besitzen. R¹ und R² bedeuten vorzugsweise jeweils unabhängig voneinander Alkyl mit 1 bis 8 C-Atomen. Y¹ bedeutet vorzugsweise F. Bevorzugte Medien enthalten 1 - 15 Gew.%, insbesondere 1 - 10 Gew.% dieser Verbindungen.

LC-Medium, welches zusätzlich eine oder mehrere Verbindungen der folgenden Formel enthält: 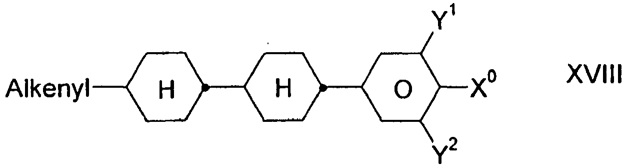 worin X⁰, Y¹ und Y² die oben angegebenen Bedeutungen besitzen und "Alkenyl" C₂₋₇-Alkenyl bedeutet. Besonders bevorzugt sind Verbindungen der folgenden Formel, 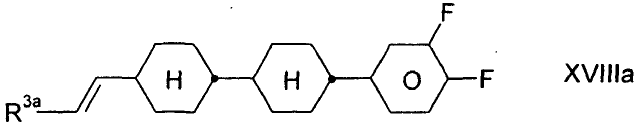 worin R^{3a} die oben angegebene Bedeutung hat und vorzugsweise H bedeutet; LC-Medium, welches zusätzlich eine oder mehrere Vierkern-Verbindungen ausgewählt aus der Gruppe bestehend aus den Formeln XIX bis XXV enthält: 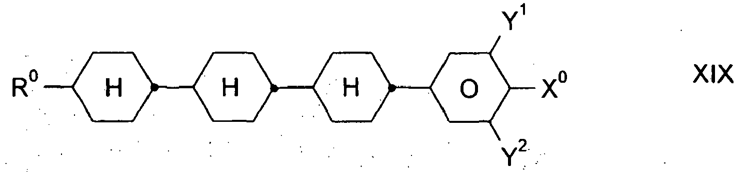 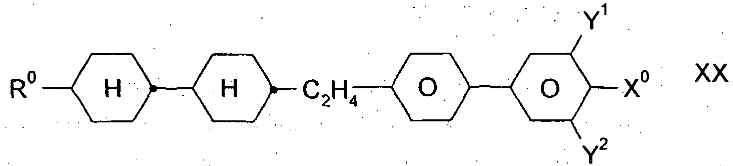 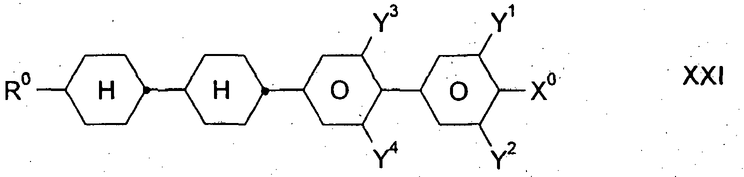 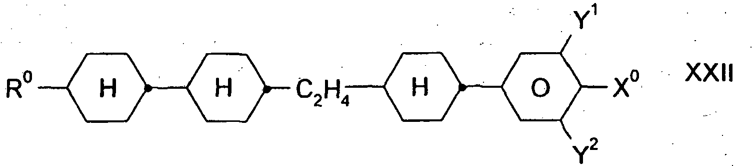 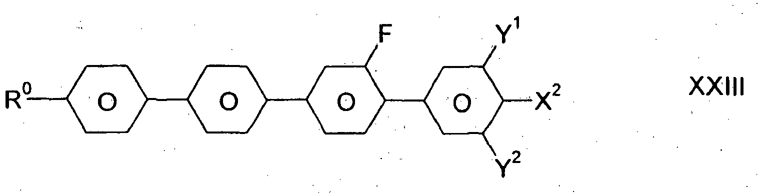 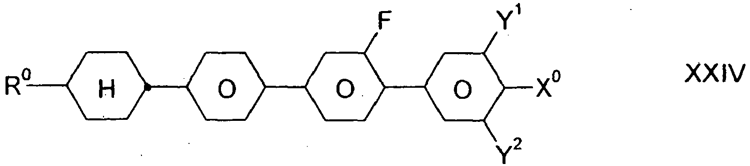 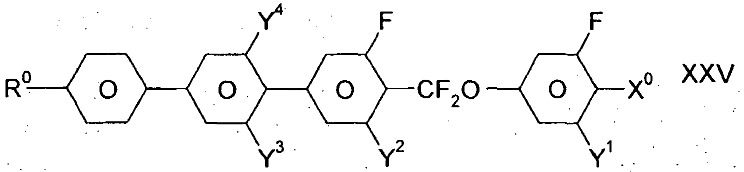 worin Y¹⁻⁴, R⁰ und X⁰ jeweils unabhängig voneinander eine der oben angegebenen Bedeutungen haben. X⁰ ist vorzugsweise F, Cl, CF₃, OCF₃ oder OCHF₂. R⁰ bedeutet vorzugsweise Alkyl, Alkoxy, Oxaalkyl, Fluoralkyl oder Alkenyl mit jeweils bis zu 8 C-Atomen.

LC-Medium, welches zusätzlich eine oder mehrere Verbindungen der folgenden Formel enthält: 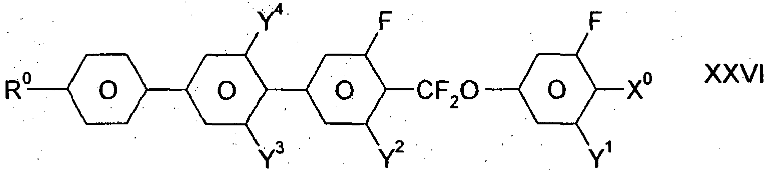 worin R⁰, X⁰ und Y¹⁻⁴ die oben angegebenen Bedeutungen besitzen. Besonders bevorzugt sind Verbindungen der folgenden Formel: 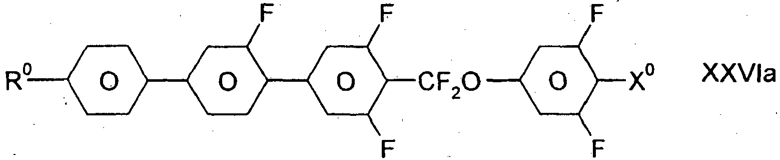
- LC-Medium, welches zusätzlich eine oder mehrere Verbindungen der folgenden Formel enthält: 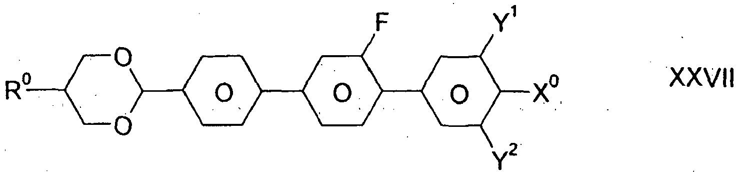 worin R⁰, Y¹, Y² und X⁰ wie oben definiert sind. R⁰ bedeutet besonders bevorzugt einen n-Butylrest. 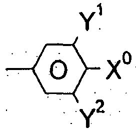 ist vorzugsweise 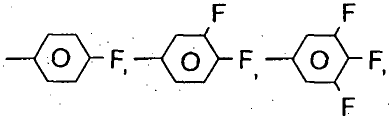 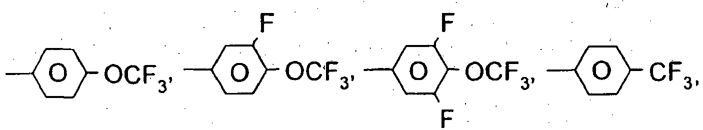 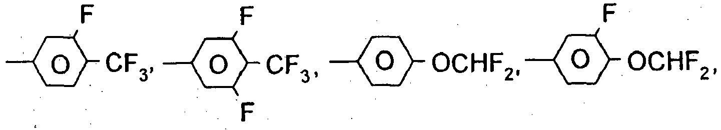 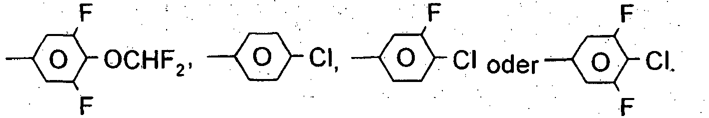
- R⁰ ist im Allgemeinen vorzugsweise geradkettiges Alkyl oder Alkenyl mit 2 bis 7 C-Atomen;
- X⁰ ist vorzugsweise F, ferner OCF₃, Cl oder CF₃;
- Das Medium enthält vorzugsweise eine, zwei oder drei Verbindungen der Formel I;
- Das Medium enthält vorzugsweise jeweils eine oder mehrere Verbindungen ausgewählt aus der Gruppe der Verbindungen der Formeln I und II;
- Das Medium enthält vorzugsweise eine oder mehrere Verbindungen ausgewählt aus der Gruppe der Verbindungen der Formeln VI-2, VII-1a, VII-1b, IX, X, XI und XXV (CF₂O-verbrückte Verbindungen); der Gesamtgehalt an Verbindungen der Formeln VI-2, VII-1a, VII-1b, IX, X, XI und XXV und der erfindungsgemäßen Verbindungen der Formel I beträgt bevorzugt 35 Gew.-%. oder mehr, besonders bevorzugt 40 Gew.-% oder mehr und ganz besonders bevorzugt 45 Gew.-% oder mehr.
- Das Medium enthält vorzugsweise 1-25 Gew.%, bevorzugt 10-20 Gew.% an Verbindungen der Formel I;
- Der Anteil an Verbindungen der Formeln II-XXVII im Gesamtgemisch beträgt vorzugsweise 20 bis 99 Gew.%;
- Das Medium enthält vorzugsweise 25-80 Gew.%, besonders bevorzugt 30-70 Gew.% an Verbindungen der Formel II und/oder III;
- Das Medium enthält vorzugsweise 20-70 Gew.%, besonders bevorzugt 25-60 Gew.% an Verbindungen der Formel IIa;
- Das Medium enthält vorzugsweise 2-25 Gew.%, besonders bevorzugt 3-20 Gew.% ausgewählt aus der Gruppe der Verbindungen der Formeln I und VI-2; in einer besonders bevorzugten Ausführungsform ist ein geringer Anteil oder keine Verbindung der Formel VI-2 enthalten. Die Verbindung der Formel I ersetzt dann diese Komponente ganz oder teilweise;
- Das Medium enthält insgesamt 2-30 Gew.%, besonders bevorzugt 3-20 Gew.% an Verbindungen der Formeln XI und XXVI zusammen;
- Das Medium enthält vorzugsweise 1-20 Gew.%, besonders bevorzugt 2-15 Gew.% an Verbindungen der Formel XXIV;
- Das Medium enthält insgesamt 15-65 Gew.%, besonders bevorzugt 30-55 Gew.% ausgewählt aus den hochpolaren Verbindungen der Formeln VI-2, X, XI und XXVII zusammen.

Es wurde gefunden, dass bereits ein relativ geringer Anteil an Verbindungen der Formel I im Gemisch mit üblichen Flüssigkristallmaterialien, insbesondere jedoch mit einer oder mehreren Verbindungen der Formeln II bis XXIX zu einer beträchtlichen Erhöhung der dielektrischen Anisotropie und zu niedrigen Werten für die Rotationsviskosität führt, wobei gleichzeitig breite nematische Phasen mit tiefen Übergangstemperaturen smektisch-nematisch beobachtet werden, wodurch die Lagerstabilität verbessert wird. Gleichzeitig zeigen die Medien sehr niedrige Schwellenspannungen und sehr gute Werte für die VHR bei UV-Belastung.

Der Ausdruck "Alkyl" umfasst geradkettige und verzweigte Alkylgruppen mit 1-9 Kohlenstoffatomen, insbesondere die geradkettigen Gruppen Methyl, Ethyl, Propyl, Butyl, Pentyl, Hexyl und Heptyl. Gruppen mit 2-5 Kohlenstoffatomen sind im allgemeinen bevorzugt.

Der Ausdruck "Alkenyl" umfasst geradkettige und verzweigte Alkenylgruppen mit bis zu 9 Kohlenstoffatomen, insbesondere die geradkettigen Gruppen. Besonders bevorzugte Alkenylgruppen sind C₂-C₇-1E-Alkenyl, C₄-C₇-3E-Alkenyl, C₅-C₇-4-Alkenyl, C₆-C₇-5-Alkenyl und C₇-6-Alkenyl, insbesondere C₂-C₇-1 E-Alkenyl, C₄-C₇-3E-Alkenyl und C₅-C₇-4-Alkenyl. Beispiele bevorzugter Alkenylgruppen sind Vinyl, 1 E-Propenyl, 1E-Butenyl, 1E-Pentenyl, 1E-Hexenyl, 1E-Heptenyl, 3-Butenyl, 3E-Pentenyl, 3E-Hexenyl, 3E-Heptenyl, 4-Pentenyl, 4Z-Hexenyl, 4E-Hexenyl, 4Z-Heptenyl, 5-Hexenyl, 6-Heptenyl und dergleichen. Gruppen mit bis zu 5 Kohlenstoffatomen sind im allgemeinen bevorzugt.

Der Ausdruck "Fluoralkyl" umfasst in dieser Anmeldung geradkettige Gruppen mit mindestens einem Fluoratom, vorzugsweise einem endständigem Fluor, d.h. Fluormethyl, 2-Fluorethyl, 3-Fluorpropyl, 4-Fluorbutyl, 5-Fluorpentyl, 6-Fluorhexyl und 7-Fluorheptyl. Andere Positionen des Fluors sind jedoch nicht ausgeschlossen.

Der Ausdruck "halogenierter Alkylrest" umfasst vorzugsweise ein- oder mehrfach fluorierte und/oder chlorierte Reste. Perhalogenierte Reste sind eingeschlossen. Besonders bevorzugt sind fluorierte Alkylreste, insbesondere CF₃, CH₂CF₃, CH₂CHF₂, CHF₂, CH₂F, CHFCF₃ und CF₂CHFCF₃.

Der Ausdruck "Alkylen" umfasst geradkettige und verzweigte Alkandiylgruppen mit 1-12 Kohlenstoffatomen, insbesondere die geradkettigen Gruppen Methylen, Ethylen, Propylen, Butylen und Pentylen. Gruppen mit 2-8 Kohlenstoffatomen sind im Allgemeinen bevorzugt.

Der Ausdruck "Oxaalkyl" bzw. "Alkoxy" umfasst in dieser Anmeldung geradkettige Reste der Formel CₙH₂ₙ₊₁-O-(CH₂)ₘ, worin n und m jeweils unabhängig voneinander 1 bis 6 bedeuten. m kann auch 0 bedeuten. Vorzugsweise ist n = 1 und m 1-6 oder m = 0 und n = 1-3.

Falls in den oben- und untenstehenden Formeln R⁰ einen Alkylrest und/oder einen Alkoxyrest bedeutet, so kann dieser geradkettig oder verzweigt sein. Vorzugsweise ist er geradkettig, hat 2, 3, 4, 5, 6 oder 7 C-Atome und bedeutet demnach bevorzugt Ethyl, Propyl, Butyl, Pentyl, Hexyl, Heptyl, Ethoxy, Propoxy, Butoxy, Pentoxy, Hexoxyoder Heptoxy, ferner Methyl, Octyl, Nonyl, Decyl, Undecyl, Dodecyl, Tridecyl, Tetradecyl, Pentadecyl, Methoxy, Octoxy, Nonoxy, Decoxy, Undecoxy, Dodecoxy, Tridecoxy oder Tetradedoxy.

Oxaalkyl bedeutet vorzugsweise geradkettiges 2-Oxapropyl (= Methoxymethyl), 2-(= Ethoxymethyl) oder 3-Oxabutyl (= 2-Methoxyethyl), 2-, 3-oder 4-Oxapentyl, 2-, 3-, 4- oder 5-Oxahexyl, 2-, 3-, 4-, 5- oder 6-Oxaheptyl, 2-, 3-, 4-, 5-, 6-, oder 7-Oxaoctyl, 2-, 3-, 4-, 5-, 6-, 7- oder 8-Oxanonyl, 2-, 3-, 4-, 5-, 6-, 7-, 8- oder 9-Oxadexyl.

Falls R⁰ einen Alkylrest bedeutet, in dem eine CH₂-Gruppe durch -CH=CH-ersetzt ist, so kann dieser geradkettig oder verzweigt sein. Vorzugsweise ist er geradkettig und hat 2 bis 10 C-Atome. Er bedeutet demnach besonders Vinyl, Prop-1-, oder Prop-2-enyl, Blut-1-, 2- oder But-3-enyl, Pent-1-, 2-, 3- oder Pent-4-enyl, Hex-1-, 2-, 3-, 4- oder Hex-5-enyl, Hept-1-, 2-, 3-, 4-, 5- oder Hept-6-enyl, Oct-1-, 2-, 3-, 4-, 5-, 6- oder Oct-7-enyl, Non-1-, 2-, 3-, 4-, 5-, 6-, 7- oder Non-8-enyl, Dec-1-, 2-, 3-, 4-, 5-, 6-, 7-, 8- oder Dec-9-enyl. Diese Reste können auch ein- oder mehrfach halogeniert sein.

Falls R⁰ einen mindestens einfach durch Halogen substituierten Alkyl- oder Alkenylrest bedeutet, so ist dieser Rest vorzugsweise geradkettig und Halogen ist vorzugsweise F oder Cl. Bei Mehrfachsubstitution ist Halogen vorzugsweise F. Die resultierenden Reste schließen auch perfluorierte Reste ein. Bei Einfachsubstitution kann der Fluor- oder Chlorsubstituent in beliebiger Position sein, vorzugsweise jedoch in ω-Position.

In den oben- und untenstehenden Formeln ist X⁰ vorzugsweise F, Cl oder ein ein- oder mehrfach fluorierter Alkyl- oder Alkoxyrest mit 1, 2 oder 3 C-Atomen oder ein ein- oder mehrfach fluorierter Alkenylrest mit 2 oder 3 C-Atomen. X⁰ ist besonders bevorzugt F, Cl, CF₃, CHF₂, OCF₃, OCHF₂, OCFHCF₃, OCFHCHF₂, OCFHCH₂F, OCF₂CH₃, OCF₂CHF₂, OCF₂CH₂F, OCF₂CF₂CHF₂, OCF₂CF₂CH₂F, OCFHCF₂CF₃, OCFHCF₂CHF₂, OCH=CF₂, OCF=CF₂, OCF₂CHFCF₃, OCF₂CF₂CF₃, OCF₂CF₂CClF₂, OCClFCF₂CF₃, CF=CF₂, CF=CHF, oder CH=CF2, ganz besonders bevorzugt F oder OCF₃.

Durch geeignete Wahl der Bedeutungen von R⁰ und X⁰ können die Ansprechzeiten, die Schwellenspannung, die Steilheit der Transmissionskennlinien etc. in gewünschter Weise modifiziert werden. Beispielsweise führen 1E-Alkenylreste, 3E-Alkenylreste, 2E-Alkenyloxyreste und dergleichen in der Regel zu kürzeren Ansprechzeiten, verbesserten nematischen Tendenzen und einem höheren Verhältnis der elastischen Konstanten k₃₃ (bend) und k₁₁ (splay) im Vergleich zu Alkyl- bzw. Alkoxyresten. 4-Alkenylreste, 3-Alkenylreste und dergleichen ergeben im allgemeinen tiefere Schwellenspannungen und kleinere Werte von k₃₃/k₁₁ im Vergleich zu Alkyl- und Alkoxyresten. Die erfindungsgemäßen Medien zeichnen sich insbesondere durch hohe K₁-Werte aus und besitzen somit deutlich schnellere Schaltzeilen als die Medien aus dem Stand der Technik.

Das optimale Mengenverhältnis der Verbindungen der oben genannten Formeln hängt weitgehend von den gewünschten Eigenschaften, von der Wahl der Komponenten der oben genannten Formeln und der Wahl weiterer gegebenenfalls vorhandener Komponenten ab.

Geeignete Mengenverhältnisse innerhalb des oben angegebenen Bereichs können von Fall zu Fall leicht ermittelt werden.

In einer bevorzugten Ausführungsform der erfindungsgemäßen Medien beträgt die dielektrische Anisotropie 13 oder mehr, bevorzugt 17 oder mehr. Die optische Anisotropie beträgt dabei bevorzugt zwischen 0, 10 oder mehr und 0,14 oder weniger, besonders bevorzugt zwischen 0,11 und 0,13. Der Klärpunkt beträgt dabei bevorzugt 70 °C oder mehr und 120 °C oder weniger. Die Mischung ist bevorzugt bis -25 °C stabil. Solche Medien besitzen eine sehr geringe Schwellenspannung. Solche Medien enthalten bevorzugt 55 Gew.-% oder mehr an hochpolaren Verbindungen mit einer dielektrischen Anisotropie von 10 oder mehr. Insbesondere enthalten sie bevorzugt 45 Gew.-% oder mehr an hochpolaren Verbindungen mit einer dielektrischen Anisotropie von 20 oder mehr. Solche Verbindungen sind in der Regel ausgewählt aus Verbindungen der Formeln I, VI-2, X, XI und XXVII. Geeignete Einzelverbindungen sind durch Vergleich der Formeln I, VI-2, X, XI und XXVII mit den Beispielen gegeben.

In einer weiteren bevorzugten Ausführungsform der erfindungsgemäßen Medien beträgt die Rotationsviskosität 90 mPas oder weniger, bevorzugt 80 mPas oder weniger. Die optische Anisotropie beträgt dabei bevorzugt zwischen 0,08 oder mehr und 0,14 oder weniger, besonders bevorzugt zwischen 0,11 und 0,13. Der Klärpunkt beträgt dabei bevorzugt 70 °C oder mehr und 100 °C oder weniger. Solche Medien besitzen in der Regel eine relativ schnelle Schaltzeit. Der Anteil an Verbindungen der Formeln II beträgt vorzugsweise 40 Gew.-% oder mehr, besonders bevorzugt 45 Gew.-% oder mehr. Für den Fall, dass keine Verbindungen der Formel II, worin a = 1 ist, eingesetzt werden, wird der Anteil an Verbindungen der Formel II, worin a = 0 ist entsprechend erhöht. Der Anteil an unpolaren Verbindungen der Formeln IIa und IIb zusammen ist gemäß dieser Ausführungsform bevorzugt über 30 Gew.-%, besonders bevorzugt 35 Gew.-% oder mehr. Demnach lässt sich der Anteil an Verbindungen der Formel II, worin a = 1 ist, bevorzugt zwischen 0 und 25 Gew.-% oder weniger variieren.

Die Gesamtmenge an Verbindungen der oben genannten Formeln in den erfindungsgemäßen Gemischen ist nicht kritisch. Die Gemische können daher eine oder mehrere weitere Komponenten enthalten zwecks Optimierung verschiedener Eigenschaften. Der beobachtete Effekt auf die gewünschte Verbesserung der Eigenschaften der Mischung ist jedoch in der Regel umso größer je höher die Gesamtkonzentration an Verbindungen der oben genannten Formeln ist.

Die einzelnen Verbindungen der oben genannten Formeln und deren Unterformeln, die in den erfindungsgemäßen Medien verwendet werden können, sind entweder bekannt, oder ihre Herstellungsweisen sind für den einschlägigen Fachmann aus dem Stand der Technik ohne weiteres abzuleiten, da sie auf in der Literatur beschriebenen Standardverfahren basieren.

Die erfindungsgemäßen Flüssigkristallmedien ermöglichen eine bedeutende Erweiterung des zur Verfügung stehenden Parameterraumes.

Die erfindungsgemäßen Medien sind insbesondere für mobile Anwendungen und high-Δn-TFT-Anwendungen wie z. B. PDAs, Notebooks, LCD-TV und Monitore geeignet.

Die erfindungsgemäßen Flüssigkristallmedien ermöglichen es, bei Beibehaltung der nematischen Phase bis -20 °C und bevorzugt bis -30 °C, besonders bevorzugt bis -40 °C, und des Klärpunkts ≥ 70 °C, vorzugsweise ≥ 75 °C, gleichzeitig Rotationsviskositäten γ₁ von ≤ 110 mPa·s, besonders bevorzugt ≤ 90 mPa·s zu erreichen, wodurch hervorragende LC-Matrixanzeigen mit schnellen Schaltzeiten erzielt werden können.

Die dielektrische Anisotropie der erfindungsgemäßen Flüssigkristallmedien Δε ist vorzugsweise ≥ +5, besonders bevorzugt ≥ +10. Die Medien sind außerdem durch kleine Operationsspannungen gekennzeichnet. Die Schwellenspannung der erfindungsgemäßen Flüssigkristallmedien ist vorzugsweise ≤ 1,4 V, insbesondere ≤ 1,2 V, in dafür angepassten Medien auch ≤ 1,0 V.

Die Doppelbrechung Δn der erfindungsgemäßen Flüssigkristallmedien ist vorzugsweise ≥ 0,10, besonders bevorzugt ≥ 0,11. Δn ist bevorzugt ≤ 0,15, besonders bevorzugt ≤ 0,13.

Weitere besonders bevorzugte Ausführungsformen der Erfindung erstrecken sich auf folgende Parameter:

Der nematische Phasenbereich der erfindungsgemäßen Flüssigkristallmedien ist vorzugsweise mindestens 90°, insbesondere mindestens 100° breit. Vorzugsweise erstreckt sich dieser Bereich mindestens von -25 °C oder weniger bis +70 °C oder mehr, besonders bevorzugt von -30 bis 80 °C oder höher. In einer weiteren bevorzugten Ausführungsform ist der Klärpunkt zwischen 70 und 100 °C, besonders bevorzugt zwischen 75 und 90 °C.

Es versteht sich, dass durch geeignete Wahl der Komponenten der erfindungsgemäßen Medien auch höhere Klärpunkte (z.B. oberhalb 100 °C) bei höheren Schwellenspannungen oder niedrigere Klärpunkte bei niedrigeren Schwellenspannungen unter Erhalt der anderen vorteilhaften Eigenschaften realisiert werden können. Ebenso können bei entsprechend wenig erhöhten Viskositäten Medien mit größerem Δε und somit geringen Schwellen erhalten werden. Die erfindungsgemäßen LC-Matrixanzeigen arbeiten vorzugsweise im ersten Transmissionsminimum nach Gooch und Tarry [C.H. Gooch und H.A. Tarry, Electron. Lett. 10, 2-4, 1974; C.H. Gooch und H.A. Tarry, Appl. Phys., Vol. 8, 1575-1584, 1975], wobei hier neben besonders günstigen elektrooptischen Eigenschaften, wie z.B. hohe Steilheit der Kennlinie und geringe Winkelabhängigkeit des Kontrastes (DE-PS 30 22 818) bei gleicher Schwellenspannung wie in einer analogen Anzeige im zweiten Minimum, eine kleinere dielektrische Anisotropie ausreichend ist. Hierdurch lassen sich unter Verwendung der erfindungsgemäßen Medien im ersten Minimum deutlich höhere spezifische Widerstände verwirklichen als bei Medien mit Cyanverbindungen. Der Fachmann kann durch geeignete Wahl der einzelnen Komponenten und deren Gewichtsanteilen mit einfachen Routinemethoden die für eine vorgegebene Schichtdicke der LC-Matrixanzeige erforderliche Doppelbrechung einstellen.

Messungen des "Voltage Holding-ratio" (HR) [S. Matsumoto et al., Liquid Crystals 5, 1320 (1989); K. Niwa et al., Proc. SID Conference, San Francisco, June 1984, p. 304 (1984); G. Weber et al., Liquid Crystals 5, 1381 (1989)] haben ergeben, dass erfindungsgemäße Medien enthaltend Verbindungen der Formel I eine deutlich geringere Abnahme des HR unter UV-Belastung aufweisen als analoge Medien enthaltend Cyanophenylcyclohexane der Formel 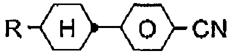 oder Ester der Formel 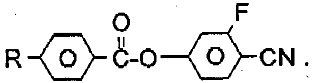 Die LC-Medien sind vorzugsweise zu 99 Gew.-%, besonders bevorzugt zu 100 Gew.-% frei von Benzonitrilderivaten.

Die LC-Medien können auch weitere, dem Fachmann bekannte und in der Literatur beschriebene Zusätze, wie z. B. UV-Stabilisatoren wie Tinuvin^{®} der Fa. Ciba, Antioxidantien, Radikalfänger, Nanopartikel, etc. enthalten. Beispielsweise können 0-15 % pleochroitische Farbstoffe oder chirale Dotierstoffe zugesetzt werden. Geeignete Dotierstoffe werden nachfolgend in der Tabelle C genannt.

Vorzugsweise enthalten die LC-Medien 0 bis 10 Gew.%, insbesondere 0,01 bis 5 Gew.% und besonders bevorzugt 0,1 bis 3 Gew.% an Stabilisatoren. Vorzugsweise enthalten die LC-Medien einen oder mehrere Stabilisatoren ausgewählt aus 2,6-Di-tert-butylphenolen, 2,2,6,6-Tetramethylpiperidinen oder 2-Benzotriazol-2-yl-phenolen. Diese Hilfsstoffe sind dem Fachmann bekannt und kommerziell erhältlich, z. B. als Lichtschutzmittel.

Die folgenden Beispiele erläutern die Erfindung . Vor- und nachstehend bedeuten Prozentangaben Gewichtsprozent. Alle Temperaturen sind in Grad Celsius angegeben. Ferner bedeuten Tg Glastemperatur, K = kristalliner Zustand, N = nematische Phase, Sm = smektische Phase und I = isotrope Phase. Die Angaben zwischen diesen Symbolen stellen die Übergangstemperaturen dar. Δn bedeutet optische Anisotropie (589 nm, 20 °C), Δε die dielektrische Anisotropie (1kHz, 20 °C) und γ₁ die Rotationsviskosität (in der Einheit mPa·s).

Die die Substituenten an den gezeichneten gesättigten 1,4-substituierten Ringsystemen der Synthesebeispiele sind, soweit nicht anders angegeben, trans-konfiguriert. Die übrigen Formeln stehen für beide Konfigurationen und bevorzugt für die trans-Konfiguration

In der vorliegenden Anmeldung und in den folgenden Beispielen sind die Strukturen der Flüssigkristallverbindungen durch Akronyme angegeben, wobei die Transformation in chemische Formeln gemäß folgender Tabellen A und B erfolgt. Alle Reste CₙH₂ₙ₊₁ und CₘH₂ₘ₊₁ sind geradkettige Alkylreste mit n bzw. m C-Atomen; n, m und k sind ganze Zahlen und bedeuten vorzugsweise 0, 1, 2, 3, 4, 5, 6, 7, 8, 9, 10, 11 oder 12. Die Codierung gemäß Tabelle B versteht sich von selbst. In Tabelle A ist nur das Akronym für den Grundkörper angegeben. Im Einzelfall folgt getrennt von Akronym für den Grundkörper mit einem Strich ein Code für die Substituenten R^{1*}, R^{2*}, L^{1*} und L^{2*}:

| Code für R^{1*}, R^{2*}, L^{1*}, L^{2*}, L^{3*} | R^{1*} | R^{2*} | L^{1*} | L^{2*} |
|---|---|---|---|---|
| nm | CₙH₂ₙ₊₁ | CₘH₂ₘ₊₁ | H | H |
| nOm | CₙH₂ₙ₊₁ | OCₘH₂ₘ₊₁ | H | H |
| nO.m | OCₙH₂ₙ₊₁ | CₘH₂ₘ₊₁ | H | H |
| n | CₙH₂ₙ₊₁ | CN | H | H |
| nN.F | CₙH₂ₙ₊₁ | CN | F | H |
| nN.F.F | CₙH₂ₙ₊₁ | CN | F | F |
| nF | CₙH₂ₙ₊₁ | F | H | H |
| nCl | CₙH₂ₙ₊₁ | Cl | H | H |
| nOF | OCₙH₂ₙ₊₁ | F | H | H |
| nF.F | CₙH₂ₙ₊₁ | F | F | H |
| nF.F.F | CₙH₂ₙ₊₁ | F | F | F |
| nOCF₃ | CₙH₂ₙ₊₁ | OCF₃ | H | H |
| nOCF₃.F | CₙH₂ₙ₊₁ | OCF₃ | F | H |
| n-Vm | CₙH₂ₙ₊₁ | -CH=CH-CₘH₂ₘ₊₁ | H | H |
| nV-Vm | CₙH₂ₙ₊₁-CH=CH- | -CH=CH-CₘH₂ₘ₊₁ | H | H |

Bevorzugte Mischungskomponenten finden sich in den Tabellen A und B.

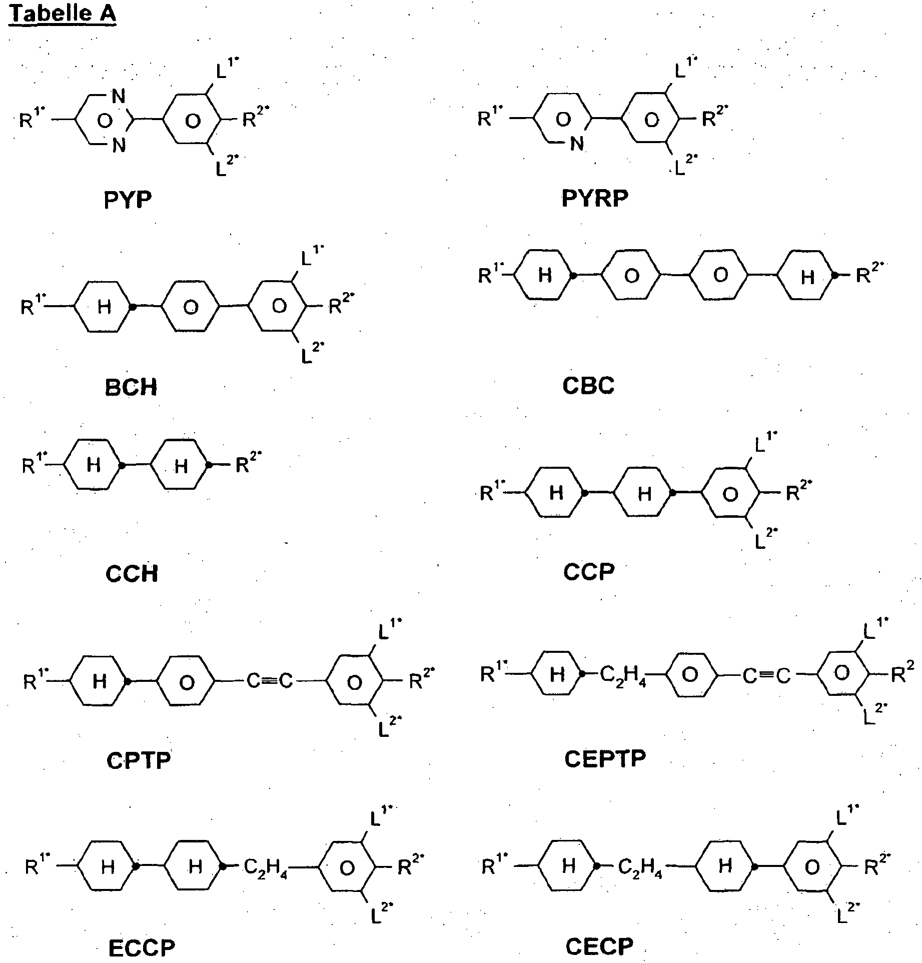

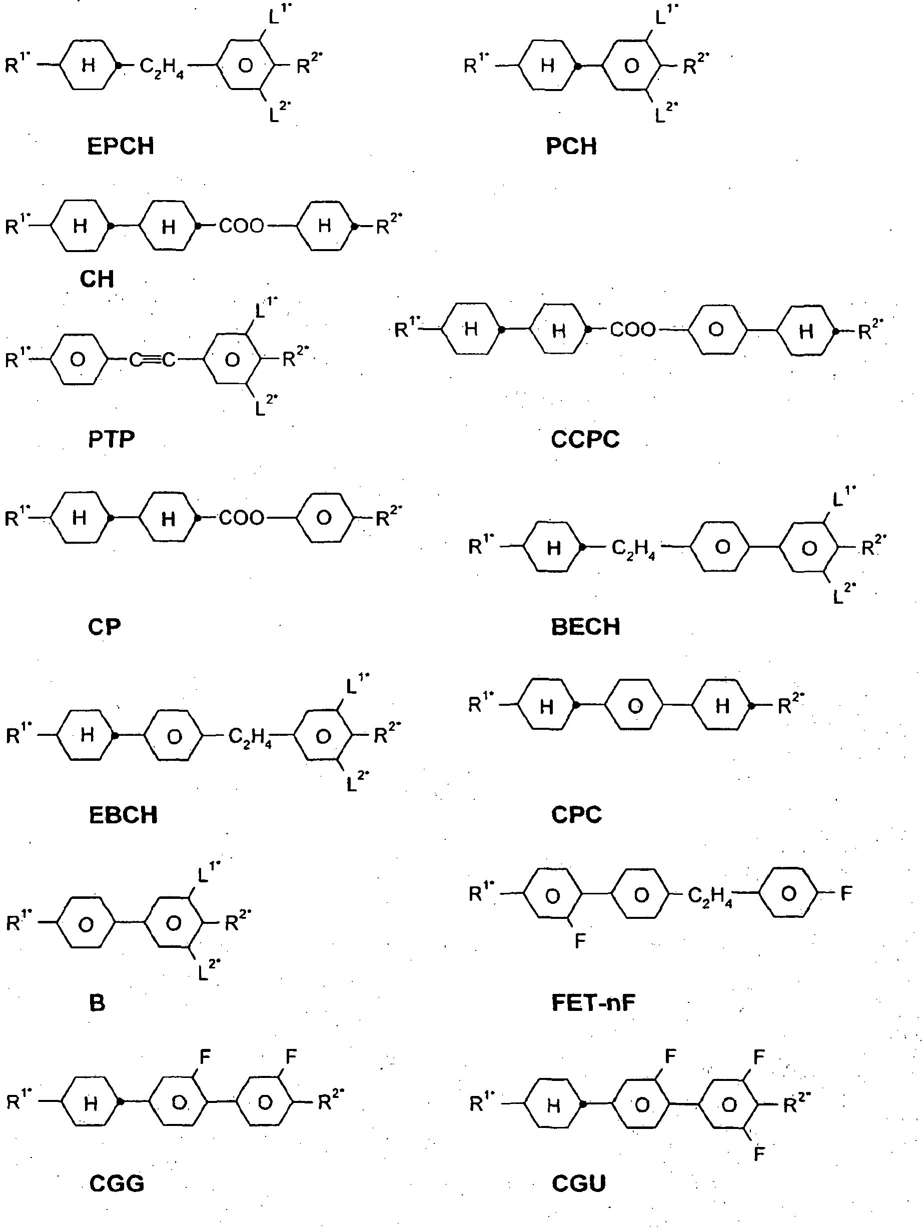

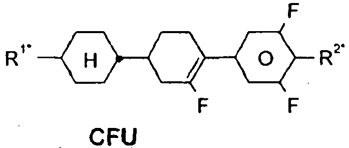

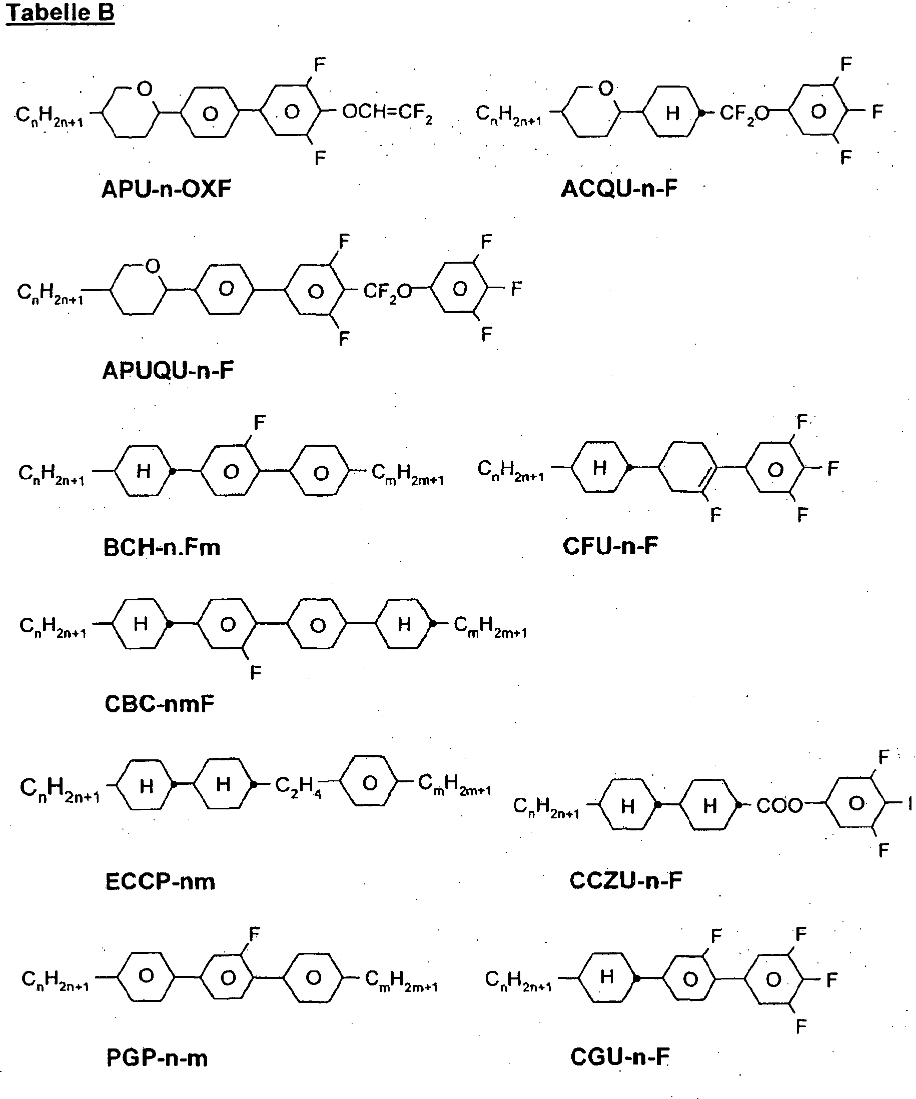

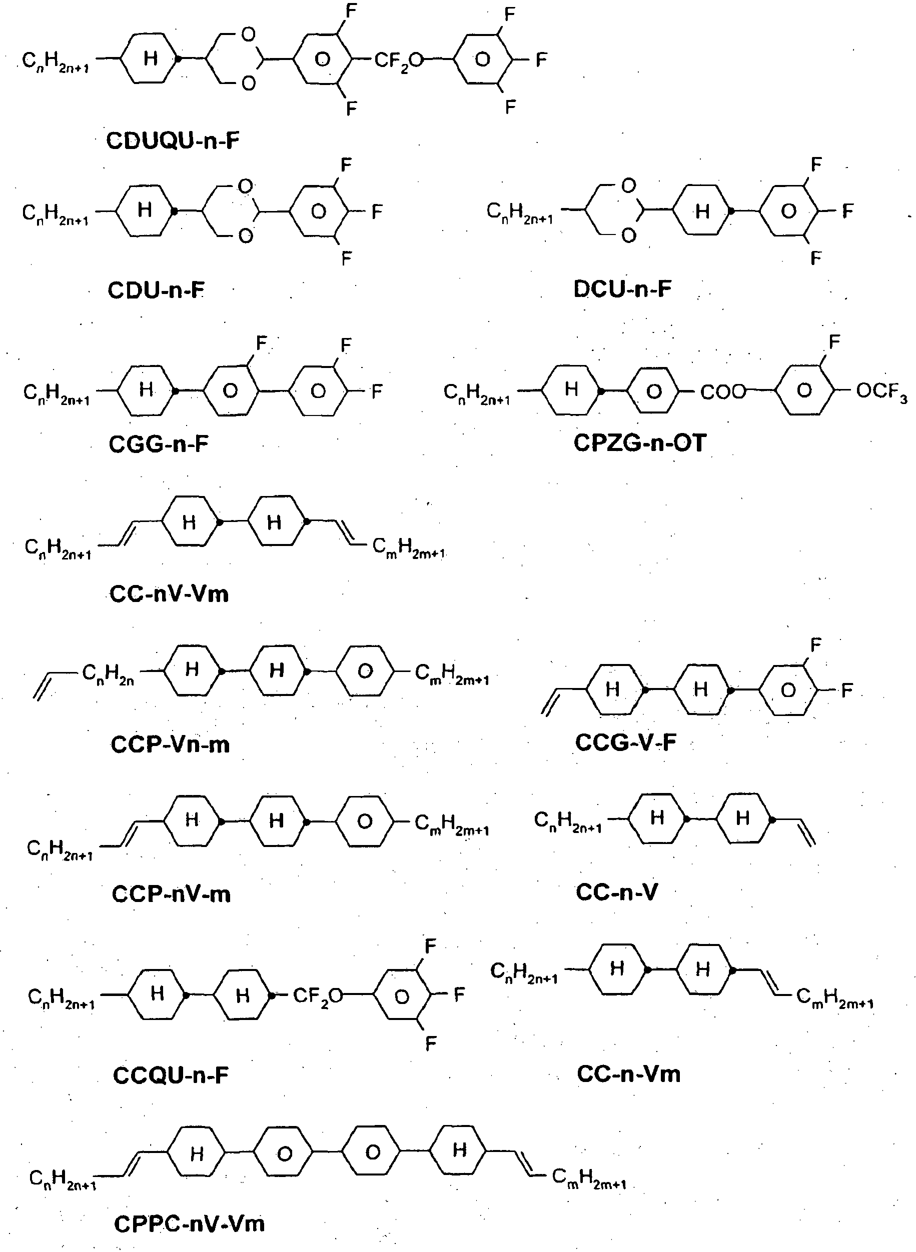

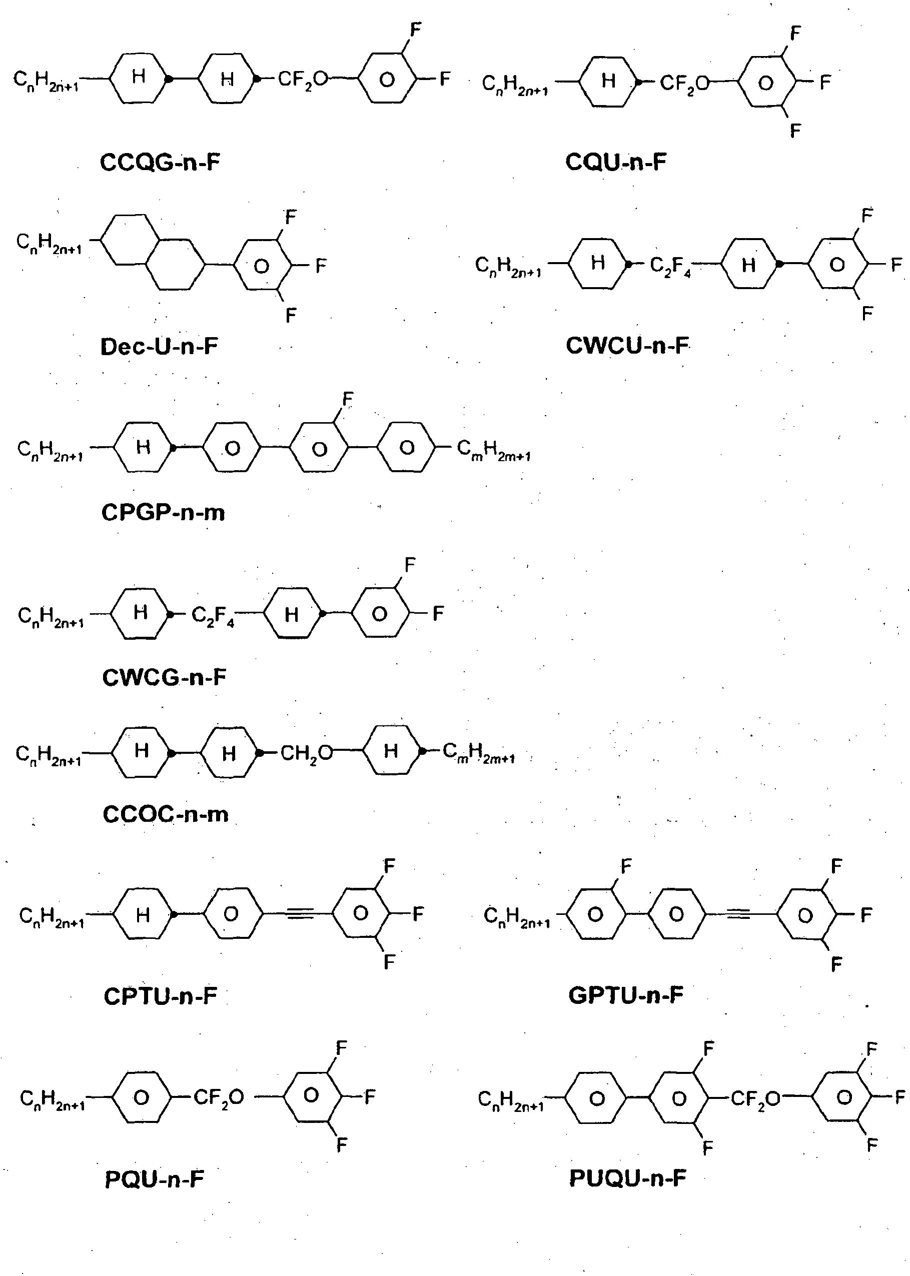

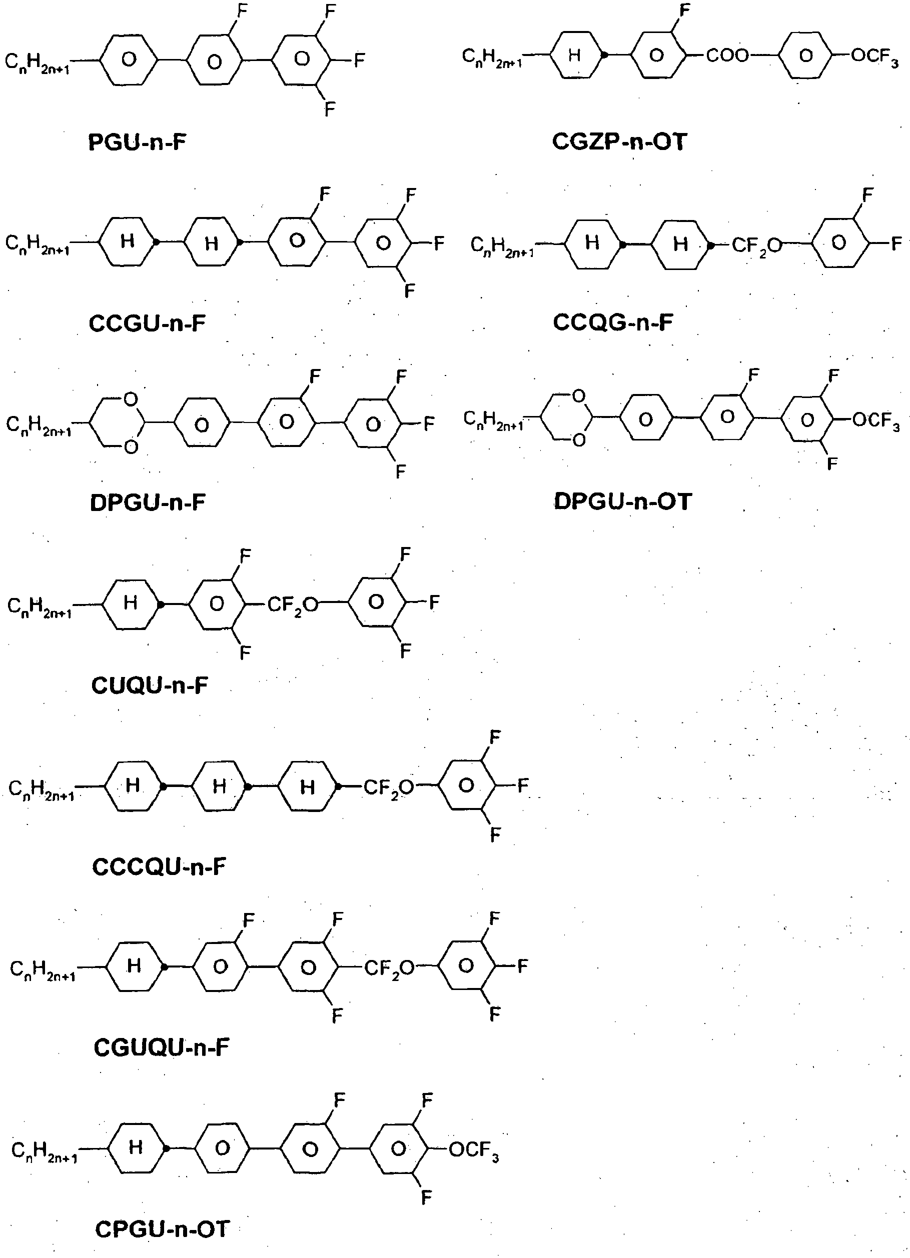

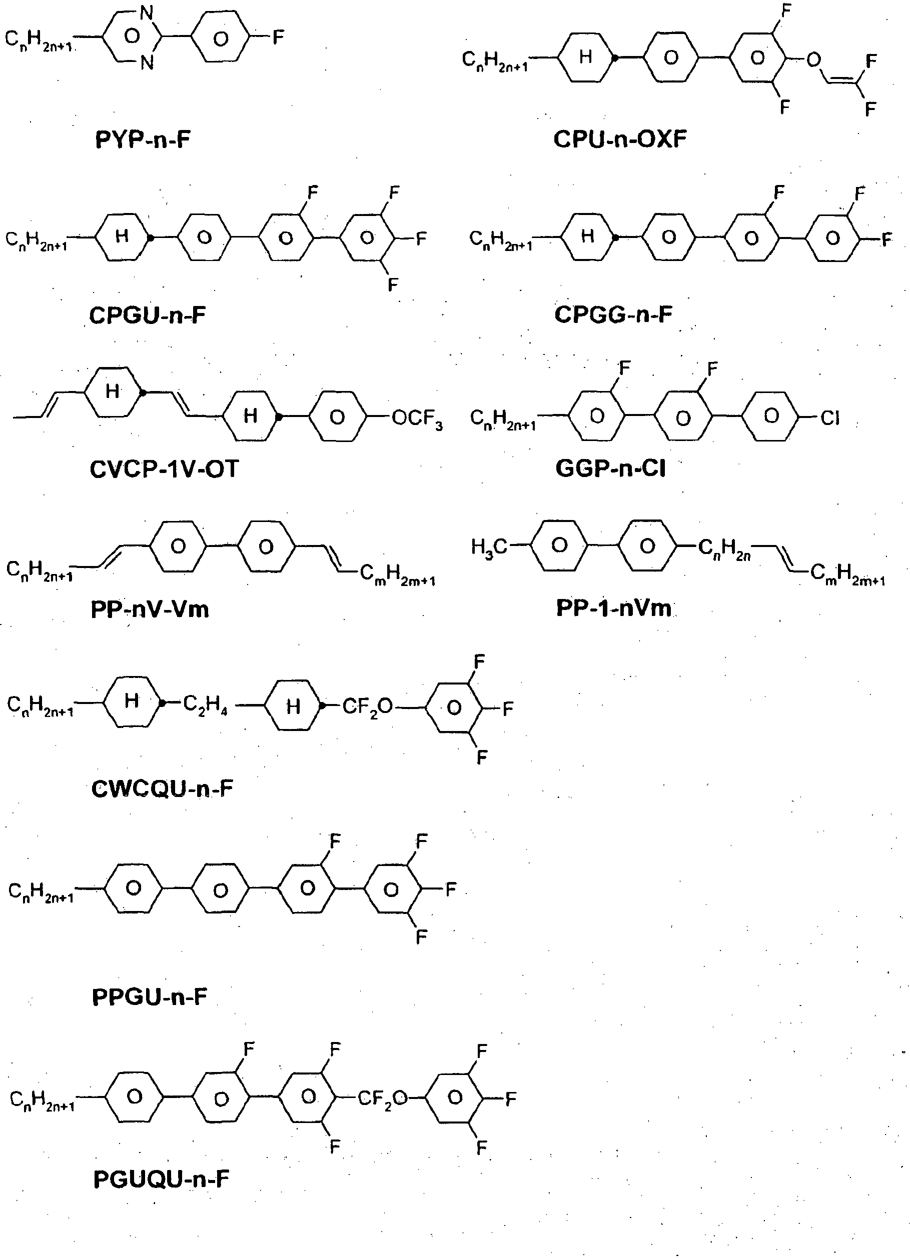

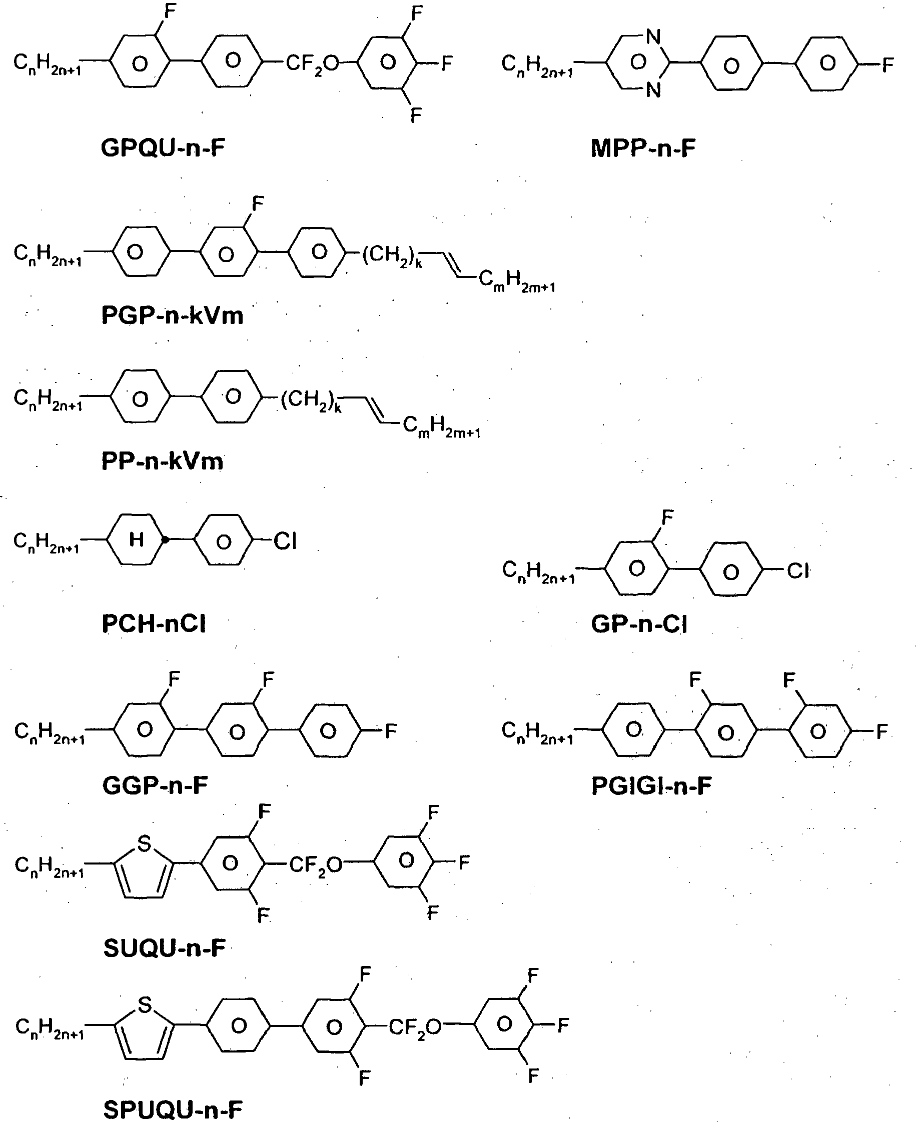

In einer bevorzugten Ausführungsform der vorliegenden Erfindung enthalten die erfindungsgemäßen LC-Medien eine oder mehrere Verbindungen ausgewählt aus der Guppe bestehend aus Verbindungen der Tabelle A und B.

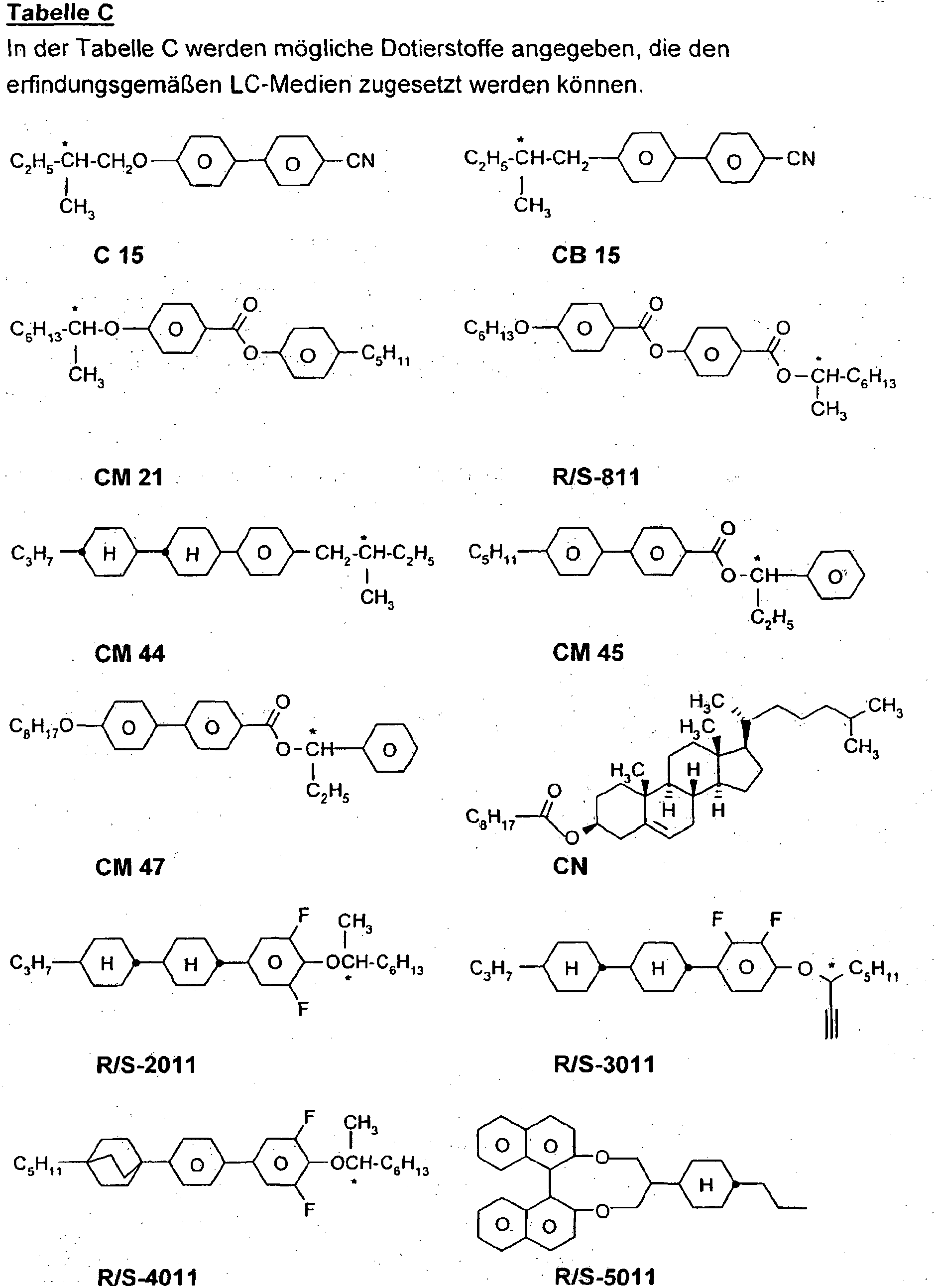

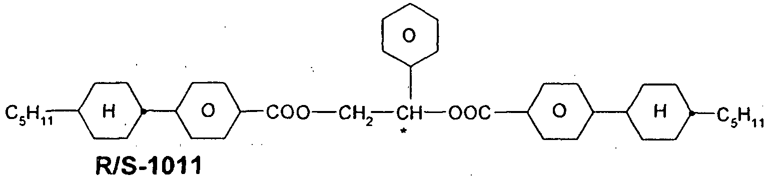

Vorzugsweise enthalten die LC-Medien 0 bis 10 Gew.%, insbesondere 0,01 bis 5 Gew.% und besonders bevorzugt 0,1 bis 3 Gew.% an Dotierstoffen. Vorzugsweise enthalten die LC-Medien einen oder mehrere Dotierstoffe ausgewählt aus der Guppe bestehend aus Verbindungen der Tabelle C.

Die Bestimmung physikalischer, physikochemischer beziehungsweise elektrooptischer Parameter erfolgt nach allgemein bekannten Verfahren, wie sie unter anderem beschrieben sind in der Broschüre "Merck Liquid Crystals - Licristal® - Physical Properties, of Liquid Crystals - Description, of the Measurements Methods", 1998, Merck KGaA, Darmstadt.
Die dielektrische Anisotropie Δε von einzelnen Substanzen wird bei 20 °C und 1 kHz bestimmt. Dazu werden 5-10 Gew.% der zu untersuchenden Substanz in der dielektrisch positiven Mischung ZLI-4792 (Merck KGaA) gelöst gemessen und der Messwert auf eine Konzentration von 100% extrapoliert. Die optische Anisotropie Δn wird bei 20°C und einer Wellenlänge von 589,3 nm bestimmt, die Rotationsviskosität γ₁ bei 20°C, beide ebenfalls durch lineare Extrapolation. Der Klärpunkt wird an der Reinsubstanz oder, wenn das nicht möglich ist, ebenfalls durch Extrapolation aus ZLI-4792 bestimmt.

Außerdem werden folgende Abkürzungen und Symbole verwendet:

| | |
|---|---|
| Vₒ | Schwellenspannung, kapazitiv [V] bei 20°C, |
| V₁₀ | optische Schwelle für 10 % relativen Kontrast [V] bei 20°C, |
| nₑ | außerordentlicher Brechungsindex bei 20°C und 589 nm, |
| nₒ | ordentlicher Brechungsindex bei 20°C und 589 nm, |
| Δn | optische Anisotropie bei 20°C und 589 nm, |
| ε_{⊥} | dielektrische Suszeptibilität senkrecht zum Direktor bei 20°C und 1 kHz, |
| ε_{∥} | dielektrische Suszeptibilität parallel zum Direktor bei 20°C und 1 kHz, |
| Δε | dielektrische Anisotropie bei 20°C und 1 kHz, |
| Kp., T(N,I) | Klärpunkt [°C], |
| γ₁ | Rotationsviskosität bei 20°C [mPa·s], |
| K₁ | elastische Konstante, "splay"-Deformation bei 20°C [pN], |
| K₂ | elastische Konstante, "twist"-Deformation bei 20°C [pN], |
| K₃ | elastische Konstante, "bend"-Deformation bei 20°C [pN], |
| LTS | "low temperature stability" (Phase), bestimmt in Testzellen oder an einer Vorratsmenge ('bulk'). |

Soweit nicht explizit anders vermerkt, sind in der vorliegenden Anmeldung alle Konzentrationen in Gewichtsprozent angegeben und beziehen sich auf die entsprechende Gesamtmischung ohne Lösungsmittel.

Der Begriff "Schweilenspannung" bezieht sich für die vorliegende Erfindung auf die kapazitive Schwelle (V₀), auch Freedericksz-Schwelle genannt, sofern nicht explizit anders angegeben. In den Beispielen kann auch, wie allgemein üblich, die optische Schwelle für 10 % relativen Kontrast (V₁₀) angegeben werden.

Die zur Messung der kapazitiven Schwellenspanung Vₒ sowie für V₁₀ verwendeten Testzellen sind aufgebaut aus Substraten bestehend aus Natriumglas (Sodalime Glas), die mit Polyimidorientierungsschichten (Durimid 32 mit Verdünner (70 % NMP + 30 % Xylol) im Verhältnis 1:4) der Firma Arch Chemicals beschichtet sind, welche antiparallel zueinander gerieben sind und einen Oberflächentilt von quasi 0 Grad aufweisen. Die Fläche der durchsichtigen, nahezu quadratischen Elektroden aus ITO, beträgt 1 cm². Zur Bestimmung der kapazitiven Schwellenspannung wird ein handelsübliches hochauflösendes LCR-Meter (z.B. LCR-Meter 4284A der Firma Hewlett Packard) eingesetzt.

Folgende Abkürzungen werden verwendet:

| | |
|---|---|
| LC | Flüssigkristall- bzw. flüssigkristallin |
| THF | Tetrahydrofuran |
| MTB-Ether | Methyl-*t*-butylether |

### Beispiel 1

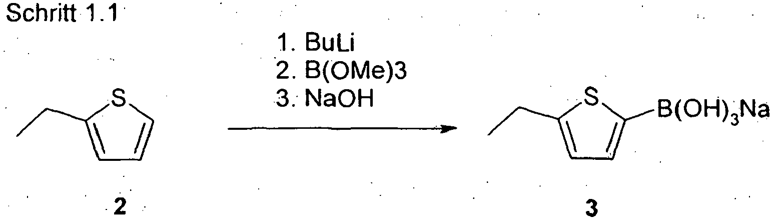

Unter Stickstoff wird eine Lösung von 116 g (950 mmol) 2-Ethylthiophen in 200 ml THF bei -70 °C mit 656 ml (15 %ige Lösung in *n*-Hexan) (1.04 mol) Butyllithium versetzt. Anschließend wird der Ansatz 30 min bei -70 °C und 20 min bei -20 °C gerührt. Bei -70 °C wird dem Gemisch 124 ml (1,09 mol) Trimethylborat zugesetzt und 30 min bei tiefer Temperatur gerührt. Das Kältebad wird entfernt, und der Ansatz wird bei -15 °C mit 500 ml Wasser verdünnt und mit Salzsäure angesäuert. Die wässerige Phase wird mit MTB-Ether extrahiert, die vereinigtenorganischen Phasen werden mit ges. Natriumchloridlösung gewaschen, über Natriumsulfat getrocknet und eingeengt. Der Rückstand wird in 1000 ml THF gelöst und unter Rühren und Kühlen mit 80 ml 50 %iger Natronlauge versetzt. Der Ansatz wird auf -10 °C abgekühlt und der ausgefallene Feststoff abgetrennt.

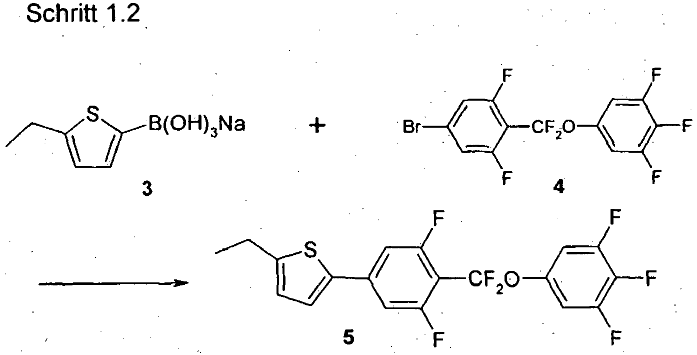

30,9 g (112 mmol) Natriummetaborat-Octahydrat werden in 45 ml Wasser und 125 ml THF vorgelegt und mit 1,1 g (1,5 mmol) Bis(triphenyphosphin)-palladium(II)chlorid und 0,1 ml (1,5 mmol) Hydraziniumhydroxid versetzt. Nach 5 min werden 14,7 g (75 mmol) des Boronats 3 und 29,2 g (75 mmol) des Bromids 4 hin zu gefügt, und der Ansatz wird 8 h zum Sieden erhitzt. Anschließend wird der Ansatz mit MTB-Ether verdünnt. Die organische Phase wird eingeengt, und der Rückstand über Kieselgel gegeben (*n*-Heptan). Die weitere Reinigung erfolgt durch Kristallisation aus *n*-Pentan (Schmp. 36 °C).
K 36 l
Δn = 0,128
Δε = 24
γ₁ = 47 mPa·s

Analog werden die folgenden Verbindungen hergestellt: 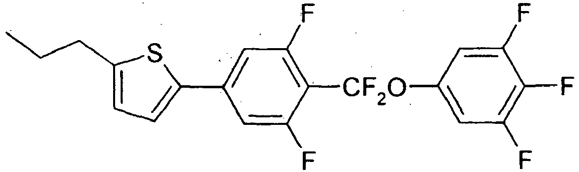 K 22 l
Δn = 0,126
Δε=24
γ₁ = 50 mPa·s

### Beispiel 2

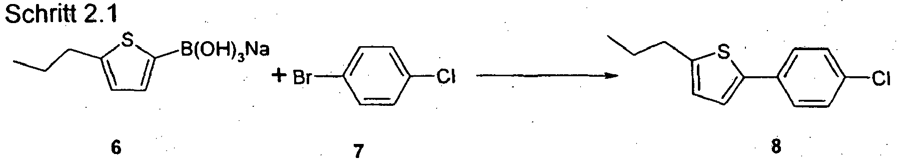

Analog zu Schritt 1.2 wird das Zwischenprodukt **8** hergestellt.

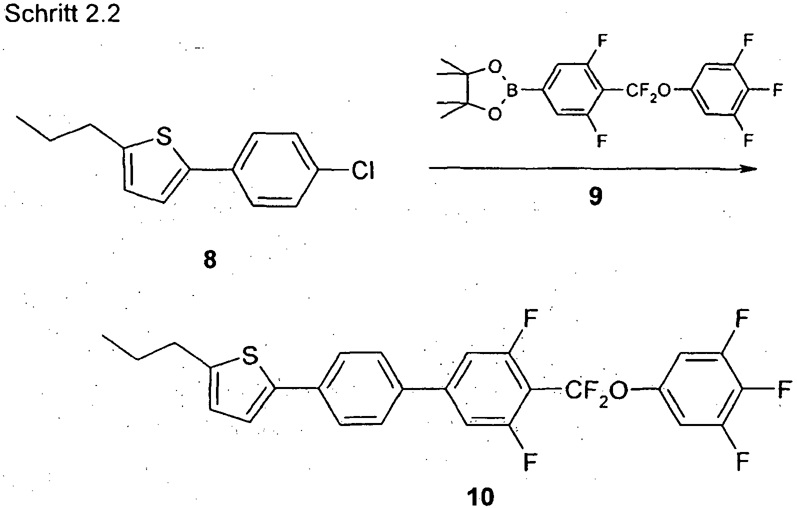

Gemäß der Reaktionsbedingungen zu Schritt 1.2 wird das Produkt **10** hergestellt.
K 143 N (138)l
Δn= 0,243
Δε=29

### Beispiel 3

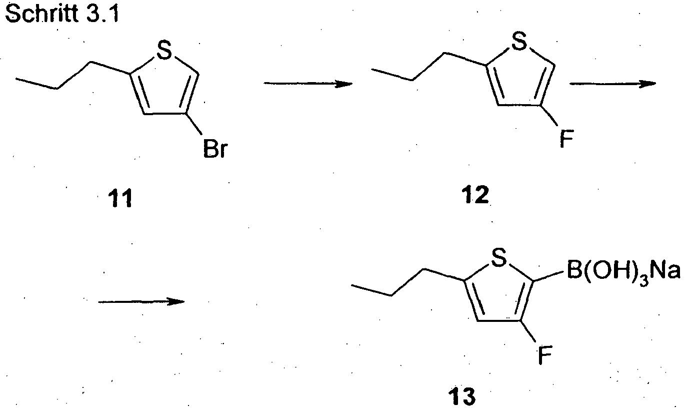

Nach Synth. Commun. 2008, 38 (1), 72-76 oder Eur. J. Org. Chem. 2005, 1, 91 - 97 lässt sich am Thiophenderivat **11** das Brom- gegen das Fluoratom austauschen. Anschließend wird das Boronat anlog der oben genannten Vorschrift (Schritt 1.1) hergestellt.

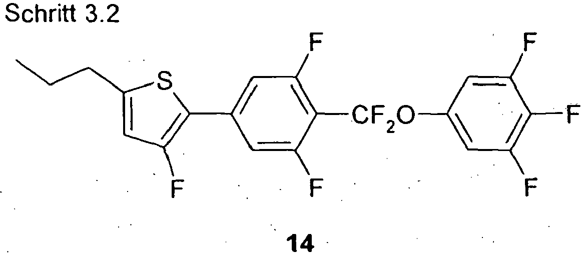

Mit dem Boronat der Formel 13 wird die Substanz 14 nach der unter Beispiel 1 (Schritt 1.2) beschriebenen Methode dargestellt.

### Beispiel 4

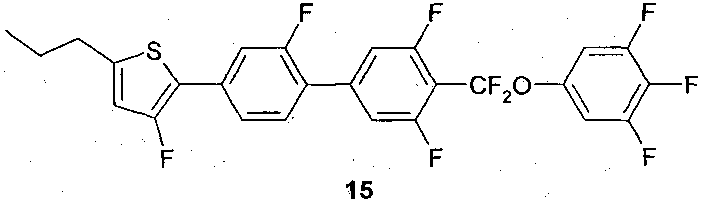

Analog zu Beispiel 2 und 3 wird die Verbindung 15 hergestellt.

### Beispiel 5

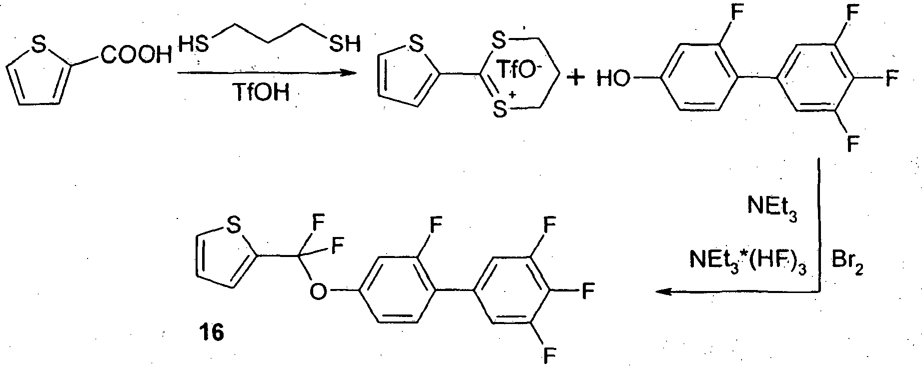

Die Verbindung 16 lässt sich aus den angegebenen bekannten

Vorprodukten gemäß den einschlägigen Verfahren (vgl. allgemeiner Teil) herstellen.
K 68 l
Δn = 0,098
Δε = 17
γ₁ = 57 mPa·s

Analog wird die folgende Verbindung hergestellt:

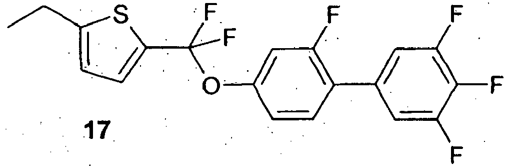

### Mischungsbeispiel M1

| | | | |
|---|---|---|---|
| CC-3-V | 32,5 % | S → N[°C]: | < -30,0 |
| CCQU-3-F | 7,0 % | Klärpunkt [°C]: | +81 |
| SUQU-2-F | 13,0 % | Δn [589 nm; 20°C]: | +0,127 |
| PGU-3-F | 2,0 % | Δε | +21,8 |
| CCP-V-1 | 7,5 % | γ₁[mPa·s; 20°C]: | 107 |
| APUQU-3-F | 8,0 % | | |
| PGUQU-3-F | 5,0 % | V₁₀[V]: | 0,97 |
| PGUQU-4-F | 8,0 % | V₉₀[V]: | 1,49 |
| PGUQU-5-F | 7,0 % | | |
| DPGU-4-F | 10,0 % | | |
| | 100,0 % | | |

Die Mischung ist gut geeignet für TN-TFT.

### Mischungsbeispiel M2

| | | | |
|---|---|---|---|
| CC-3-V | 35,0 % | S → N [°C]: | < -40 |
| SUQU-2-F | 14,0 % | Klärpunkt [°C]: | 80,5 |
| PGU-3-F | 5,0 % | Δn [589 nm; 20 °C]: | 0,126 |
| CCP-V-1 | 16,0 % | Δε | +16,5 |
| PGUQU-3-F | 5,0 % | γ₁ [mPa·s; 20°C]: | 89 |
| PGUQU-4-F | 7,0 % | | |
| PGUQU-5-F | 8,0 % | V₁₀[V]: | 1,10 |
| DPGU-4-F | 10,0 % | V₉₀[V]: | 1,55 |
| | 100,0 % | | |

Die Mischung ist gut geeignet für TN-TFT.

### Mischungsbeispiel M3

| | | | |
|---|---|---|---|
| CC-3-V | 29,5 % | S → N[°C]: | < -30 |
| SUQU-2-F | 15,0 % | Klärpunkt [°C]: | 78,5 |
| PGU-3-F | 8,5 % | Δn [589 nm; 20 °C]: | 0,130 |
| PGP-2-2V | 4,0 % | Δε | +18 |
| CCP-V-1 | 14,0 % | γ₁ [mPa·s; 20 °C]: | 95 |
| APUQU-2-F | 9,0 % | | |
| APUQU-3-F | 10,0 % | V₁₀[V]: | 1,07 |
| PGUQU-3-F | 5,0 % | V₉₀[V]: | 1,62 |
| CPGU-3-OT | 5,0 % | | |
| | 100,0 % | | |

Die Mischung ist gut geeignet für TN-TFT.

### Mischungsbeispiel M4

| | | | |
|---|---|---|---|
| CC-3-V | 30,0 % | S → N [°C]: | < -25 |
| SUQU-2-F | 15,0 % | Klärpunkt [°C]: | 80,5 |
| PGU-3-F | 7,0 % | Δn [589 nm; 20 °C]: | 0,129 |
| PGP-2-2V | 5,0 % | Δε | +16,3 |
| CCP-V-1 | 15,5 % | γ₁ [mPa·s; 20 °C]: | 92 |
| APUQU-2-F | 8,5 % | | |
| APUQU-3-F | 9,0 % | V₁₀ [V]: | 1,11 |
| PGUQU-3-F | 5,0 % | V₉₀ [V]: | 1,68 |
| CPGU-3-OT | 5,0 % | | |
| | 100,0 % | | |

Die Mischung ist gut geeignet für TN-TFT.

### Mischungsbeispiel M5

| | |
|---|---|
| CC-3-V | 35,0 % |
| SUQU-2-F | 12,5 % |
| CCP-V-1 | 15,0 % |
| PGUQU-3-F | 4,0 % |
| CPGU-3-OT | 5,0 % |
| APUQU-2-F | 3,0 % |
| APUQU-3-F | 10,0 % |
| PGP-2-2V | 7,5 % |
| PGU-3-F | 8,0 % |
| | 100,0 % |

Die Mischung ist gut geeignet für TN-TFT.

### Mischungsbeispiel M6

| | | | |
|---|---|---|---|
| CC-3-V | 40,0 % | | |
| CCGU-3-F | 6,0 % | Klärpunkt [°C]: | 75 |
| PGUQU-3-F | 5,0 % | Δn [589 nm; 20°C]: | 0,103 |
| APUQU-2-F | 3,0 % | Δε | +14,3 |
| APUQU-3-F | 11,0 % | γ₁[mPa·s; 20°C]: | 79 |
| CPGU-3-OT | 3,0 % | | |
| CCQU-3-F | 10,0 % | V₀ [V]: | 0,92 |
| CCQU-5-F | 7,0 % | | |
| PGU-2-F | 5,0 % | | |
| SUQU-2-F | 10,0 % | | |
| | 100,0 % | | |

Die Mischung ist gut geeignet für IPS.

### Mischungsbeispiel M7

| | | | |
|---|---|---|---|
| SUQU-2-F | 14,0 % | | |
| CCGU-3-F | 3,0 % | Klärpunkt [°C]: | 88 |
| CC-3-V | 32,0 % | Δn [589 nm; 20 °C]: | 0,124 |
| CCP-V-1 | 10,0 % | Δε | +11,3 |
| CCP-V2-1 | 11,0 % | γ₁[mPa·s; 20°C]: | 86 |
| PGP-2-3 | 5,0 % | | |
| PGP-2-4 | 5,0 % | V₀ [V]: | 1,16 |
| APUQU-2-F | 2,0 % | LTS (bulk, -30 °C): | >500 h |
| APUQU-3-F | 11,0 % | | |
| PGUQU-3-F | 7,0 % | | |
| | 100,0 % | | |

Die Mischung ist gut geeignet für IPS.

### Mischungsbeispiel M8

| | | | |
|---|---|---|---|
| PGUQU-3-F | 7,5 % | | |
| CPGU-3-OT | 3,5 % | Klärpunkt [°C]: | 74,5 |
| SUQU-2-F | 9,0 % | Δn [589 nm; 20 °C]: | 0,131 |
| PP-1-2V1 | 1,5 % | Δε | +7,4 |
| CC-3-V | 44,0 % | γ₁ [mPa·s; 20 °C]: | 56 |
| PGP-2-2V | 18,0 % | | |
| CPU-3-OXF | 16,5 % | V₁₀ [V]: | 1,55 |
| | 100,0 % | V₉₀ [V]: | 2,30 |

Die Mischung ist gut geeignet für TN-TFT.

### Mischungsbeispiel M9

| | | | |
|---|---|---|---|
| CC-3-V | 40,5 % | | |
| APUQU-2-F | 4,0 % | Klärpunkt [°C]: | 75 |
| APUQU-3-F | 8,5 % | Δn [589 nm; 20 °C]: | 0,126 |
| CPGU-3-OT | 2,0 % | Δε | +12,0 |
| PGUQU-3-F | 9,0 % | γ₁ [mPa·s; 20 °C]: | 71 |
| SUQU-3-F | 8,0 % | | |
| PGP-2-2V | 9,0 % | V₁₀ [V]: | 1,21 |
| CPU-3-OXF | 19,0 % | V₉₀ [V]: | 1,83 |
| | 100,0 % | | |

Die Mischung ist gut geeignet für TN-TFT.

Weitere Kombinationen der Ausführungsformen und Varianten der Erfindung gemäß der Beschreibung ergeben sich auch aus den folgenden Ansprüchen.

## Patentansprüche

1. Verbindungen der Formel I, 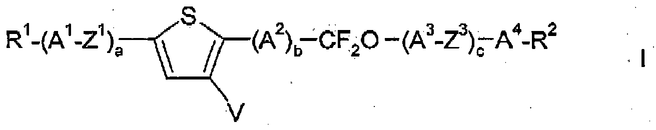 worin
R¹ H, einen halogenierten oder unsubstituierten Alkylrest mit 1 bis 15 C-Atomen, wobei in diesen Resten auch eine oder mehrere CH₂-Gruppen jeweils unabhängig voneinander durch -C≡C-, -CH=CH-, -(CO)O-, -O(CO)-, -(CO)- oder -O- so ersetzt sein können, dass O-Atome nicht direkt miteinander verknüpft sind, bedeutet,
R² F, Cl, OCF₃, OCHF₂, OCHFCF₃, OCF₂CHFCF₃, CF₃, CN, SF₅, NCS, NCO, SCN oder OCN bedeutet,
A¹, A² und A³ jeweils unabhängig voneinander, gleich oder verschieden:
a) trans-1,4-Cyclohexylen oder Cyclohexenylen, worin auch eine oder mehrere nicht benachbarte CH₂-Gruppen durch -O- und/oder -S- ersetzt sein können und worin H durch F substituiert sein kann,
b) 1,4-Phenylen, worin eine oder zwei CH-Gruppen durch N ersetzt sein können und worin auch ein oder mehrere H-Atome gegen Br, Cl, F, CN, Methyl, Methoxy oder eine ein- oder mehrfach fluorierte Methyl- oder Methoxygruppe ersetzt sein können,
oder
c) ein Rest aus der Gruppe 1,4-Bicyclo(2,2,2)-octylen, Piperidin-1,4-diyl, Cylcobut-1,3-diyl, Spiro[3.3]heptan-2,6-diyl, 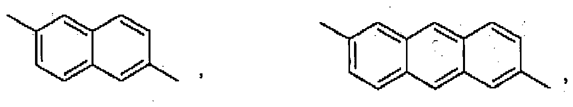 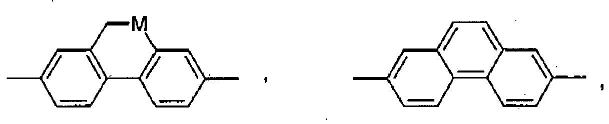 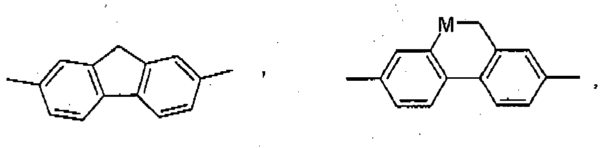 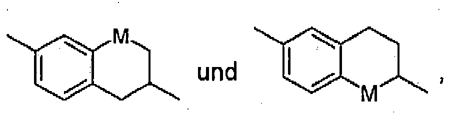 worin ein oder mehrere Wasserstoffatome durch F, CN, SCN, SF₅, CH₂F, CHF₂, CF₃, OCH₂F, OCHF₂ oder OCF₃ ersetzt sein können,
ein oder mehrere CH-Gruppen durch N ersetzt sein können,
eine oder mehrere Doppelbindungen durch Einfachbindungen ersetzt sein können,
M -O-, -S-, -CH₂-, -CHY- oder -CYY¹-,
und Y und Y¹ Cl, F, CN, OCF₃ oder CF₃ bedeuten,
A⁴ 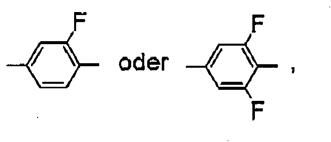
V H oder F,
Z¹ und Z³ jeweils unabhängig voneinander, gleich oder verschieden, eine Einfachbindung, -CH₂O-, -(CO)O-, -CF₂O-, -CH₂CH₂CF₂O-, -CF₂CF₂-, -CH₂CF₂-, -CH₂CH₂-, -(CH₂)₄-, -CH=CH-, -CH=CF-, -CF=CF- oder -C≡C-, wobei asymmetrische Brücken nach beiden Seiten orientiert sein können,
a 0, 1 oder 2,
b 1,2 oder 3, und
c 0, 1 oder 2
wobei a + b + c ≤ 4 ist,
bedeuten.

2. Verbindungen nach Anspruch 1 der Formel IA 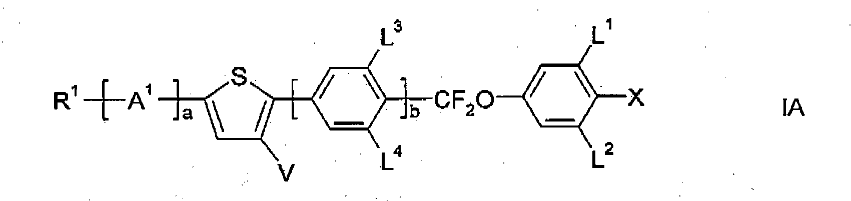 worin
R¹, A¹, a, b und V die in Anspruch 1 für Formel angegebenen Bedeutungen haben,
X F, OCF₃, CN, CF₃, SCN, SF₅, NCS, Cl, OCHF₂, OCHFCF₃, OCF₂CHFCF₃,
V H, F oder Cl, und
L¹ F,
L², L³ und L⁴ jeweils unabhängig voneinander H öder F,
bedeuten.

3. Verbindungen nach Anspruch 1 oder 2, **dadurch gekennzeichnet, dass**
R¹ Alkyl, Alkoxy, Alkenyl oder Alkenyloxy mit jeweils bis zu 8 Kohlenstoffatomen bedeutet.

4. Verbindungen nach einem oder mehreren der Ansprüche 1 bis 3 der Formeln I1 bis I6, 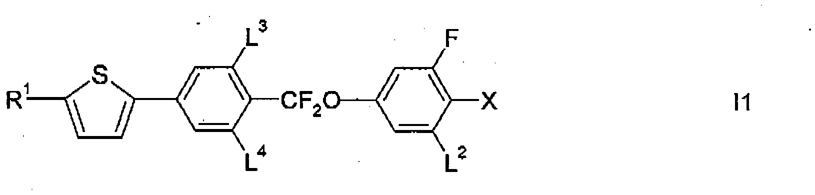 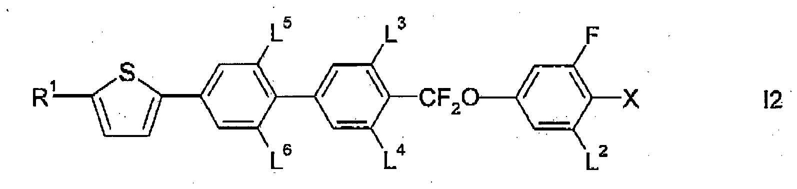 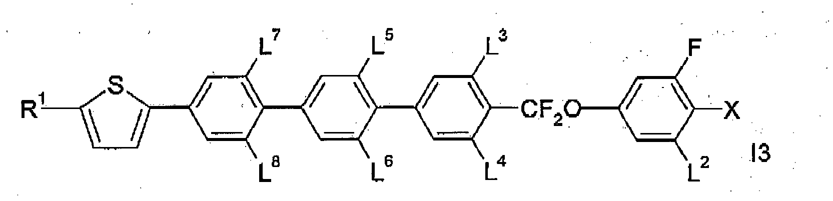 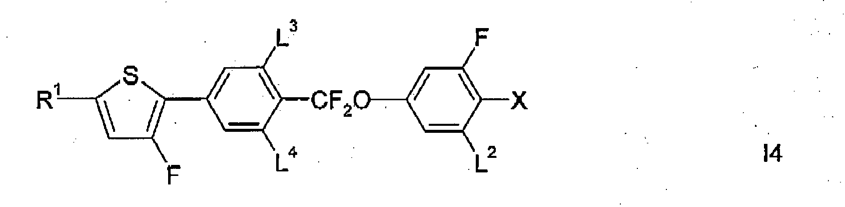 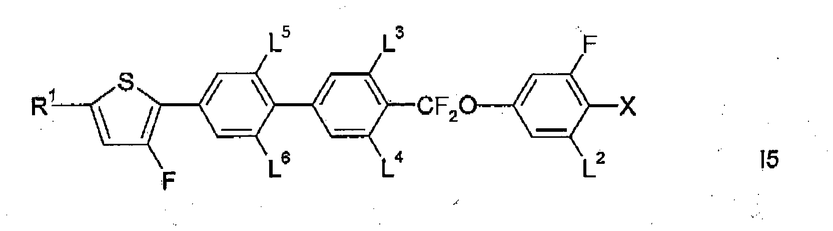 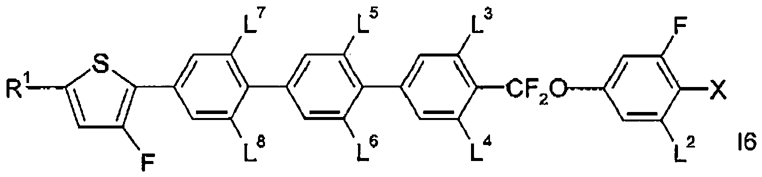 worin R¹ die in Anspruch 1 angegebenen Bedeutungen hat, und
X F, OCF₃, CN, CF₃, SCN, SF₅, NCS, Cl, OCHF₂, OCHFCF₃, OCF₂CHFCF₃ und
L², L³, L⁴, L⁵, L⁶, L⁷ und L⁸ unabhängig voneinander H oder F
bedeuten.

5. Verbindungen nach einem oder mehreren der Ansprüche 1 bis 4, **dadurch gekennzeichnet, dass** L¹ und L² Fluor bedeuten.

6. Verbindungen nach einem oder mehreren der Ansprüche 1 bis 5, **dadurch gekennzeichnet, dass** V Wasserstoff bedeutet.

7. Verbindungen nach einem oder mehreren der Ansprüche 1 bis 6, **dadurch gekennzeichnet, dass** V Fluor bedeutet.

8. Verfahren zur Herstellung von Verbindungen der Formel I nach einem oder mehreren der Ansprüche 1 bis 7, **dadurch gekennzeichnet, dass** es einen Verfahrensschritt umfasst, worin ein Thiophenderivat der Formeln IIa oder IIb 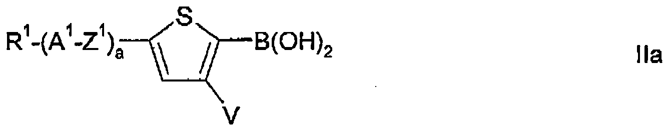 oder ein entsprechendes Boronat mit der Endgruppe -B(OH)₃⁻ oder -B(OH)₃M, worin M einen einwertigen ionischen Rest darstellt, 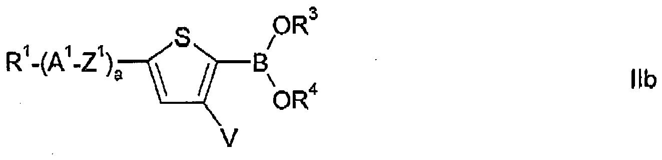 worin jeweils unabhängig R¹, A¹, Z¹, V und a wie in Anspruch 1 definiert sind, und
R³, R⁴ Alkyl mit 1-12 C-Atomen oder R³+R⁴ zusammen auch ein C₁-C₆-Alkylen,
oder 1,2-Phenylen bedeuten,
wobei R³, R⁴ und R³+R⁴ auch substituiert sein können,
mit einer Verbindung der Formel III
Hal-(A²)_{b}-CF₂O-(A³-Z³)_{c}-A⁴-R² III
worin Z³, A², A³, A⁴, b, c und R² wie in Anspruch 1 definiert sind, und Hal -O(SO₂)CF₃, Cl, Br oder bedeutet,
in Gegenwart eines Übergangsmetallkatalysators zur Reaktion gebracht wird.

9. Verwendung einer oder mehrerer Verbindungen der Formel I nach einem oder mehreren der Ansprüche 1 bis 7 als Komponenten in einem flüssigkristallinen Medium.

10. Flüssigkristallines Medium enthaltend mindestens zwei mesogene Verbindungen, **dadurch gekennzeichnet, dass** es mindestens eine Verbindung der Formel I nach einem oder mehreren der Ansprüche 1 bis 7 enthält.

11. Flüssigkristailines Medium nach Anspruch 10, dadurch gekenntzeichnet, dass es eine oder mehrere Verbindungen der Formel II und/oder III enthält: 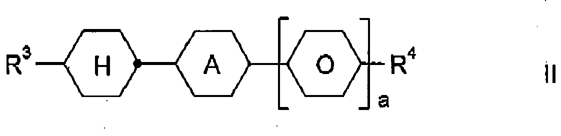 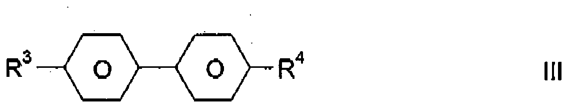 worin
Ring A 1,4-Phenylen oder trans-1,4-Cyclohexylen bedeutet,
a 0 oder 1 ist,
R³ jeweils unabhängig voneinander Alkyl mit 1 bis 9 C-Atomen oder Alkenyl mit 2 bis 9 C-Atomen bedeutet, und
R⁴ jeweils unabhängig voneinander einen unsubstituierten oder halogenierten Alkylrest mit 1 bis 12 C-Atomen, wobei auch eine oder zwei nicht benachbarte CH₂-Gruppen durch -O-, -CH=CH-, -CH=CF-, -(CO)-, -O(CO)- oder -(CO)O- so ersetzt sein können, dass O-Atome nicht direkt miteinander verknüpft sind.

12. Flüssigkristallines Medium nach Anspruch 10 oder 11, **dadurch gekennzeichnet, dass** es eine oder mehrere Verbindungen der Formeln X, XI und/oder XXVI enthält: 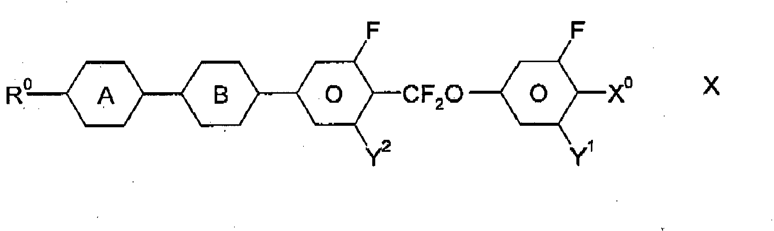 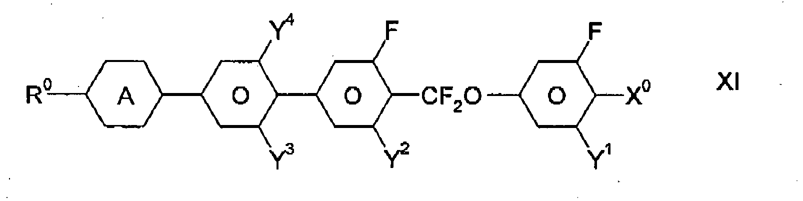 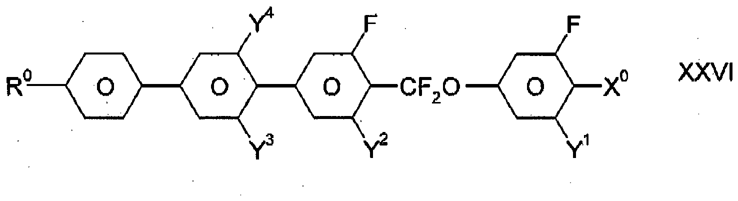 worin
R⁰ einen Alkyl- oder Alkoxyrest mit 1 bis 15 C-Atomen, wobei in diesen Resten auch eine oder mehrere CH₂-Gruppen jeweils unabhängig voneinander durch -C≡C-, -CF₂O-, -CH=CH-, 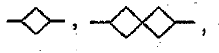 -O-, -(CO)O- oder -O(CO)- so ersetzt sein können, dass O-Atome nicht direkt miteinander verknüpft sind, und worin auch ein oder mehrere H-Atome durch Halogen ersetzt sein können, 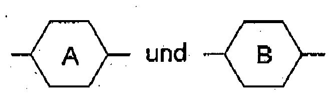 jeweils unabhängig voneinander 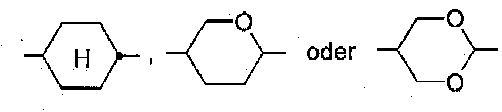 bedeuten,
X⁰ F, Cl, CN, SF₅, SCN, NCS, einen halogenierten Alkylrest, halogenierten Alkenylrest, halogenierten Alkoxyrest oder halogenierten Alkenyloxyrest mit jeweils bis zu 6 C-Atomen, und
Y¹, Y², Y³, Y⁴ jeweils unabhängig voneinander H oder F
bedeuten.

13. Flüssigkristallines Medium nach einem oder mehreren der Ansprüche 10 bis 12, **dadurch gekennzeichnet, dass** der Gesamtgehalt an CF₂O-verbrückten Verbindungen, einschließlich der Verbindungen der Formel I, 35 Gew.-% oder mehr beträgt.

14. Verwendung des flüssigkristallinen Mediums nach einem oder mehreren der Ansprüche 10 bis 13 für elektrooptische Zwecke.

15. Elektrooptische Flüssigkristallanzeige enthaltend ein flüssigkristallines Medium nach einem oder mehreren der Ansprüche 10 bis 13.

## Claims

1. Compounds of the formula I, 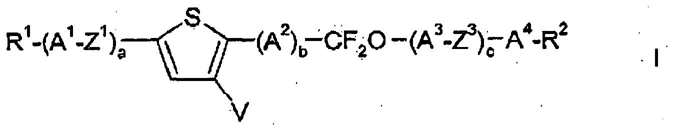 in which
R¹ denotes H, a halogenated or unsubstituted alkyl radical having 1 to 15 C atoms, where, in addition, one or more CH₂ groups in these radicals may each be replaced, independently of one another, by -C≡C-, -CH=CH-, -(CO)O-, -O(CO)-, -(CO)- or -O- in such a way that O atoms are not linked directly to one another,
R² denotes F, Cl, OCF₃, OCHF₂, OCHFCF₃, OCF₂CHFCF₃, CF₃, CN, SF₅, NCS, NCO, SCN or OCN,
A¹, A² and A³ each, independently of one another, identically or differently, denote:
a) trans-1,4-cyclohexylene or cyclohexenylene, in which, in addition, one or more non-adjacent CH₂ groups may be replaced by -O- and/or -S- and in which H may be substituted by F,
b) 1,4-phenylene, in which one or two CH groups may be replaced by N and in which, in addition, one or more H atoms may be replaced by Br, Cl, F, CN, methyl, methoxy or a mono- or polyfluorinated methyl or methoxy group,
or
c) a radical from the group 1,4-bicyclo[2.2.2]octylene, piperidine-1,4-diyl, cyclobutane-1,3-diyl, spiro[3.3]heptane-2,6-diyl, 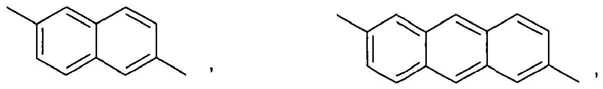 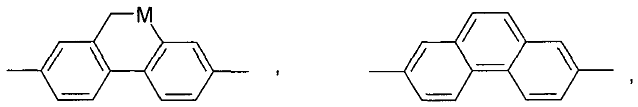 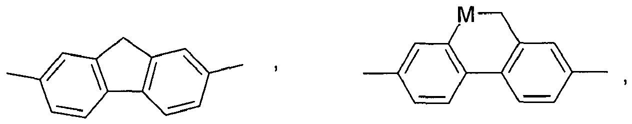 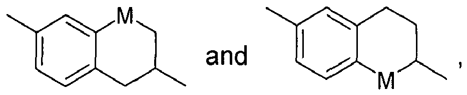 in which one or more hydrogen atoms may be replaced by F, CN, SCN, SF₅, CH₂F, CHF₂, CF₃, OCH₂F, OCHF₂ or OCF₃,
one or more CH groups may be replaced by N,
one or more double bonds may be replaced by single bonds,
M denotes -O-, -S-, -CH₂-, -CHY- or -CYY¹-,
and Y and Y¹ denote Cl, F, CN, OCF₃ or CF₃,
A⁴ denotes 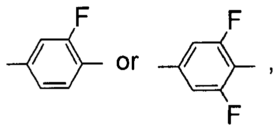
V denotes H or F,
Z¹ and Z³ each, independently of one another, identically or differently, denote a single bond, -CH₂O-, -(CO)O-, -CF₂O-, -CH₂CH₂CF₂O- -CF₂CF₂-, -CH₂CF₂-, -CH₂CH₂-, -(CH₂)₄-, -CH=CH-, -CH=CF-, -CF=CF- or -C≡C-, where asymmetrical bridges may be oriented to both sides,
a denotes 0, 1 or 2,
b denotes 1, 2 or 3, and
c denotes 0, 1 or 2,
where a + b + c ≤ 4.

2. Compounds according to Claim 1 of the formula IA 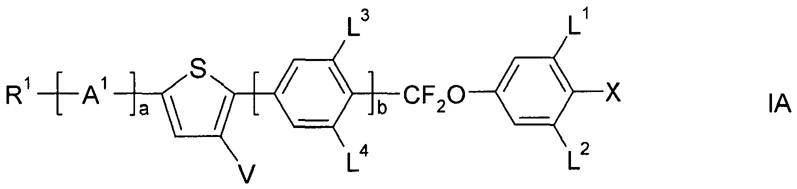 in which
R¹, A¹, a, b and V have the meanings indicated for formula I in Claim 1,
X denotes F, OCF₃, CN, CF₃, SCN, SF₅, NCS, Cl, OCHF₂, OCHFCF₃, OCF₂CHFCF₃,
V denotes H, F or Cl, and
L¹ denotes F,
L², L³ and L⁴ each, independently of one another, denote H or F.

3. Compounds according to Claim 1 or 2, **characterised in that**
R¹ denotes alkyl, alkoxy, alkenyl or alkenyloxy, each having up to 8 carbon atoms.

4. Compounds according to one or more of Claims 1 to 3 of the formulae I1 to I6, 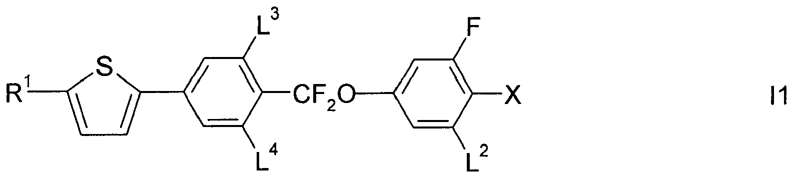 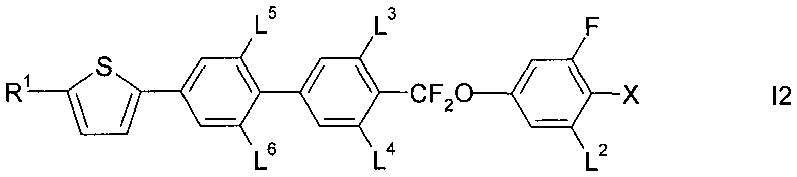 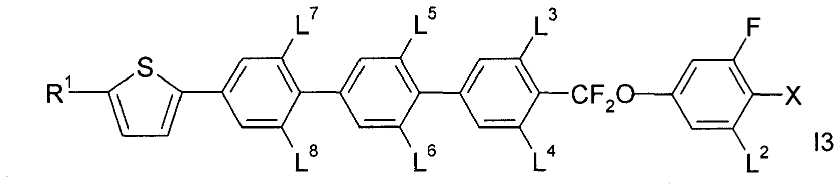 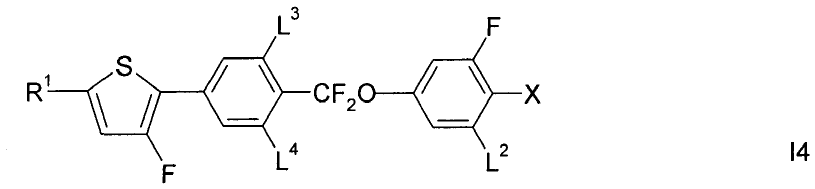 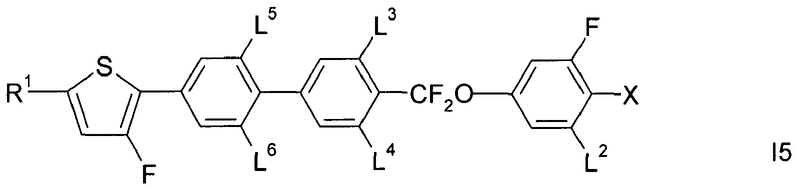 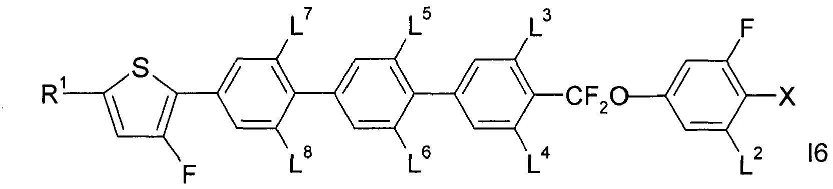 in which R¹ has the meanings indicated in Claim 1, and
X denotes F, OCF₃, CN, CF₃, SCN, SF₅, NCS, Cl, OCHF₂, OCHFCF₃, OCF₂CHFCF₃ and
L², L³, L⁴, L⁵, L⁶, L⁷ and L⁸, independently of one another, denote H or F.

5. Compounds according to one or more of Claims 1 to 4, **characterised in that** L¹ and L² denote fluorine.

6. Compounds according to one or more of Claims 1 to 5, **characterised in that** V denotes hydrogen.

7. Compounds according to one or more of Claims 1 to 6, **characterised in that** V denotes fluorine.

8. Process for the preparation of compounds of the formula I according to one or more of Claims 1 to 7, **characterised in that** it comprises a process step in which a thiophene derivative of the formula IIa or IIb 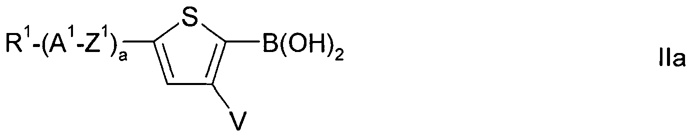 or a corresponding boronate containing the end group -B(OH)₃⁻ or -B(OH)₃M, in which M represents a monovalent ionic radical, 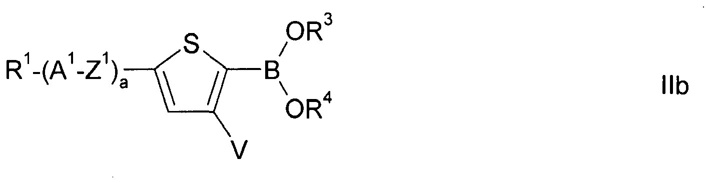 in which, in each case independently, R¹, A¹, Z¹, V and a are as defined in Claim 1, and
R³, R⁴ denote alkyl having 1-12 C atoms or R³+R⁴ together also denote C₁-C₆-alkylene, or 1,2-phenylene,
where R³, R⁴ and R³+R⁴ may also be substituted,
is reacted with a compound of the formula III
Hal-(_{A}²)_{b}-CF₂O-(A³-Z³)_{c}-A⁴-R² III
in which Z³, A², A³, A⁴, b, c and R² are as defined in Claim 1, and
Hal denotes -O(SO₂)CF₃, Cl, Br or I,
in the presence of a transition-metal catalyst.

9. Use of one or more compounds of the formula I according to one or more of Claims 1 to 7 as components in a liquid-crystalline medium.

10. Liquid-crystalline medium comprising at least two mesogenic compounds, **characterised in that** it comprises at least one compound of the formula I according to one or more of Claims 1 to 7.

11. Liquid-crystalline medium according to Claim 10, **characterised in that** it comprises one or more compounds of the formulae II and/or III: 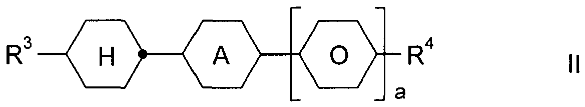 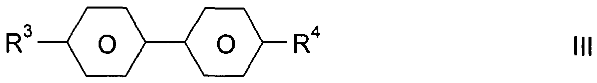 in which
ring A denotes 1,4-phenylene or trans-1,4-cyclohexylene,
a is 0 or 1,
R³ in each case, independently of one another, denotes alkyl having 1 to 9 C atoms or alkenyl having 2 to 9 C atoms, and
R⁴ in each case, independently of one another, denotes an unsubstituted or halogenated alkyl radical having 1 to 12 C atoms, where, in addition, one or two non-adjacent CH₂ groups may be replaced by -O-, -CH=CH-, -CH=CF-, -(CO)-, -O(CO)- or -(CO)O- in such a way that O atoms are not linked directly to one another.

12. Liquid-crystalline medium according to Claim 10 or 11, **characterised in that** it comprises one or more compounds of the formulae X, XI and/or XXVI: 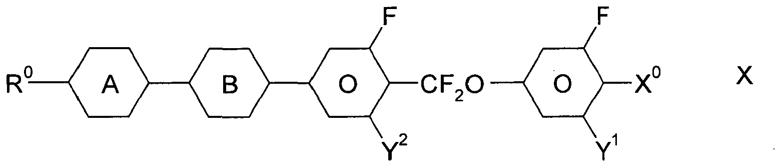 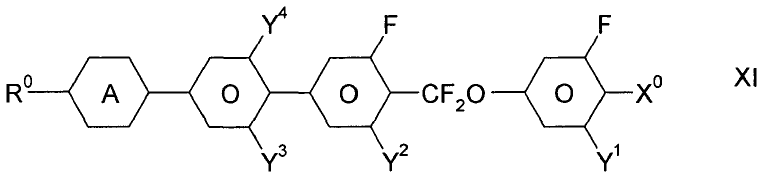 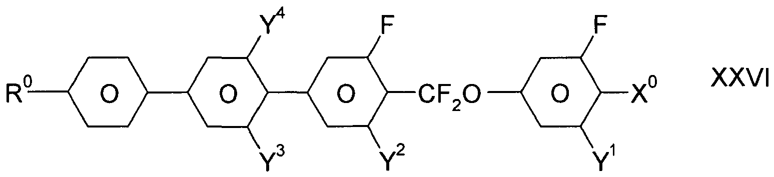 in which
R⁰ denotes an alkyl or alkoxy radical having 1 to 15 C atoms, where, in addition, one or more CH₂ groups in these radicals may each be replaced, independently of one another, by -C≡C-, -CF₂O-, -CH=CH-, 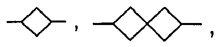 -O-, -(CO)O- or -O(CO)- in such a way that O atoms are not linked directly to one another, and in which, in addition, one or more H atoms may be replaced by halogen, 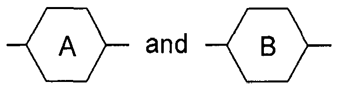 each, independently of one another, denote 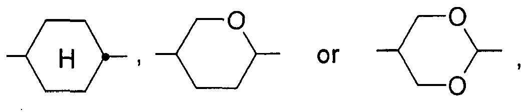
X⁰ denotes F, Cl, CN, SF₅, SCN, NCS, a halogenated alkyl radical, halogenated alkenyl radical, halogenated alkoxy radical or halogenated alkenyloxy radical, each having up to 6 C atoms, and
Y¹, Y², Y³, Y⁴ each, independently of one another, denote H or F.

13. Liquid-crystalline medium according to one or more of Claims 10 to 12, **characterised in that** the total content of CF₂O-bridged compounds, including the compounds of the formula I, is 35% by weight or more.

14. Use of the liquid-crystalline medium according to one or more of Claims 10 to 13 for electro-optical purposes.

15. Electro-optical liquid-crystal display containing a liquid-crystalline medium according to one or more of Claims 10 to 13.

## Revendications

1. Composés de la formule I : 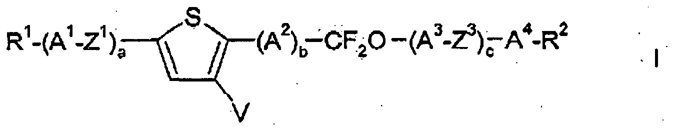 dans laquelle :
R¹ représente H, un radical alkyle halogéné ou non substitué comportant de 1 à 15 atome(s) de C, où, en outre, un ou plusieurs groupe(s) CH₂ dans ces radicaux peut/peuvent (chacun) être remplacé(s), indépendamment les uns des autres, par -C≡C-, -CH=CH-, -(CO)O-, -O(CO)-, -(CO)- ou -O- de telle sorte que des atomes de O ne soient pas liés directement les uns aux autres,
R² représente F, CI, OCF₃, OCHF₂, OCHFCF₃, OCF₂CHFCF₃, CF₃, CN, SF₅, NCS, NCO, SCN ou OCN,
A¹, A² et A³ représentent chacun, indépendamment les uns des autres, de manière identique ou différente :
a) trans-1,4-cyclohexylène ou cyclohexénylène, dans lequel, en outre, un ou plusieurs groupe(s) CH₂ non adjacents peut/peuvent être remplacé(s) par -O- et/ou -S- et dans lequel H peut être substitué par F,
b) 1,4-phénylène, où un ou deux groupe(s) CH peut/ peuvent être remplacé(s) par N et dans lequel, en outre, un ou plusieurs atome(s) de H peut/peuvent être remplacé(s) par Br, CI, F, CN, méthyle, méthoxy ou un groupe méthyle ou méthoxy monofluoré ou polyfluoré,
ou
c) un radical pris parmi le groupe 1,4-bicyclo[2.2.2]-octylène, pipéridine-1,4-diyle, cyclobutane-1,3-diyle, spiro[3.3]heptane-2,6-diyle, 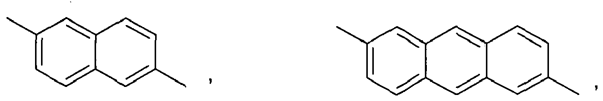 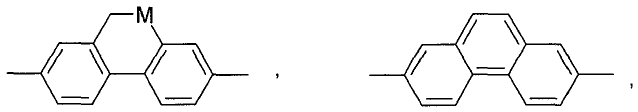 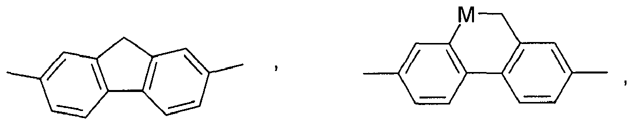 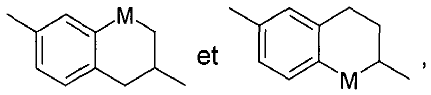 où un ou plusieurs atome(s) d'hydrogène peut/peuvent être remplacé(s) par F, CN, SCN, SF₅, CH₂F, CHF₂, CF₃, OCH₂F, OCHF₂ ou OCF₃,
un ou plusieurs groupe(s) CH peut/peuvent être remplacé(s) par N,
une ou plusieurs liaison(s) double(s) peut/peuvent être remplacée(s) par des liaisons simples,
M représente -O-, -S-, -CH₂-, -CHY- ou -CYY¹-,
et Y et Y¹ représentent Cl, F, CN, OCF₃ ou CF₃,
A⁴ représente 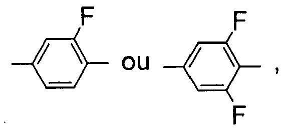
V représente H ou F,
Z¹ et Z³ représentent chacun, indépendamment l'un de l'autre, de manière identique ou différente, une liaison simple, -CH₂O-, -(CO)O-, -CF₂O-, -CH₂CH₂CF₂O- -CF₂CF₂-, -CH₂CF₂-, -CH₂CH₂-, -(CH₂)₄-, -CH=CH-, -CH=CF-, -CF=CF- ou -C≡C-, où des ponts asymétriques peuvent être orientés sur les deux côtés,
a représente 0, 1 ou 2,
b représente 1, 2 ou 3, et
c représente 0, 1 ou 2,
où a + b + c ≤ 4.

2. Composés selon la revendication 1 de la formule IA : 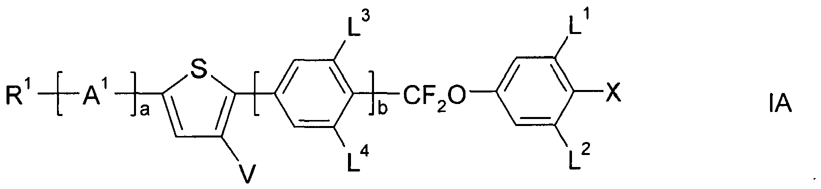 dans laquelle :
R¹, A¹, a, b et V présentent les significations indiquées pour la formule I selon la revendication 1,
X représente F, OCF₃, CN, CF₃, SCN, SF₅, NCS, CI, OCHF₂, OCHFCF₃, OCF₂CHFCF₃,
V représente H, F ou Cl, et
L¹ représente F,
L², L³ et L⁴ représentent chacun, indépendamment les uns des autres, H ou F.

3. Composés selon la revendication 1 ou 2, **caractérisés en ce que** :
R¹ représente alkyle, alcoxy, alkényle ou alkényloxy, dont chacun comporte jusqu'à 8 atomes de carbone.

4. Composés selon une ou plusieurs des revendications 1 à 3 des formules I1 à I6 : 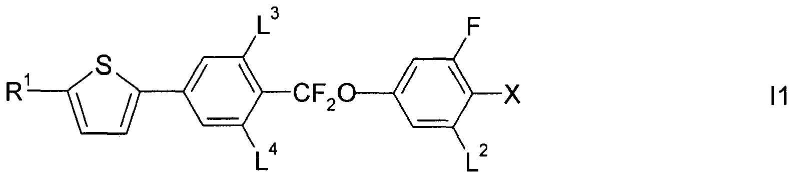 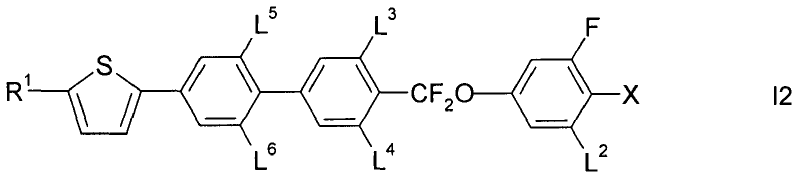 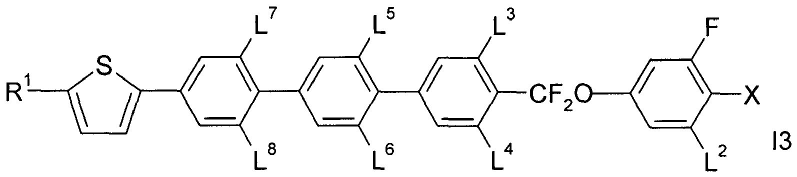 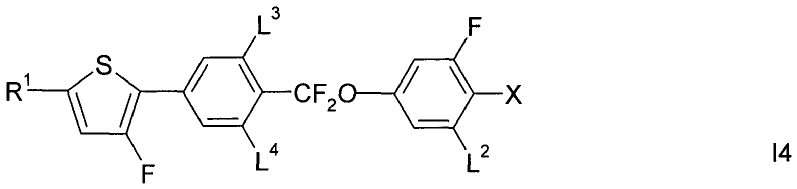 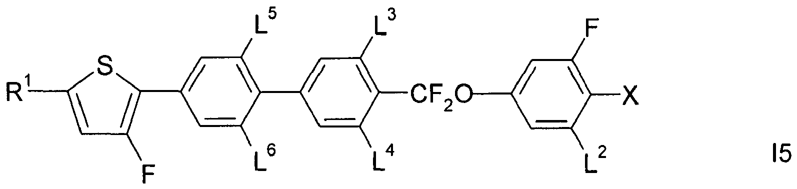 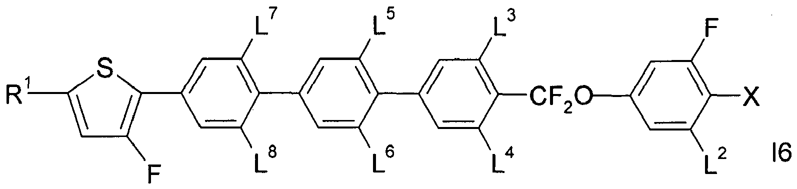 dans lesquelles R¹ présente les significations indiquées selon la revendication 1, et
X représente F, OCF₃, CN, CF₃, SCN, SF₅, NCS, CI, OCHF₂, OCHFCF₃, OCF₂CHFCF₃ et
L², L³, L⁴, L⁵, L⁶, L⁷ et L⁸ représentent,
indépendamment les uns des autres, H ou F.

5. Composés selon une ou plusieurs des revendications 1 à 4, **caractérisés en ce que** L¹ et L² représentent fluor.

6. Composés selon une ou plusieurs des revendications 1 à 5, **caractérisés en ce que** V représente hydrogène.

7. Composés selon une ou plusieurs des revendications 1 à 6, **caractérisés en ce que** V représente fluor.

8. Procédé pour la préparation de composés de la formule I selon une ou plusieurs des revendications 1 à 7, **caractérisé en ce qu'**il comprend une étape de procédé selon laquelle un dérivé thiophène de la formule IIa ou IIb : 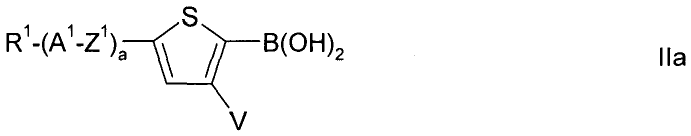 ou un boronate correspondant contenant le groupe d'extrémité -B(OH)₃⁻ ou -B(OH)₃M, dans lequel M représentes un radical ionique monovalant, 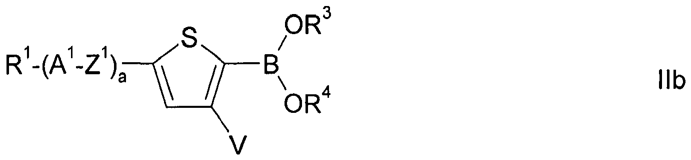 où, dans chaque cas de manière indépendante, R¹, A¹, Z¹, V et a sont comme défini selon la revendication 1, et
R³, R⁴ représentent alkyle comportant 1-12 atome(s) de C ou R³+R⁴ représentent ensemble également C₁-C₆-alkylène,
ou 1,2-phénylène,
où R³, R⁴ et R³+R⁴ peuvent également être substitués,
est amené à réagir avec un composé de la formule III :
Hal-(A²)_{b}-CF₂O-(A³-Z³)_{c}-A⁴-R² III
dans laquelle Z³, A², A³, A⁴, b, c et R² sont comme défini selon la revendication 1, et
Hal représente -O(SO₂)CF₃, Cl, Br ou I,
en présence d'un catalyseur constitué par un métal de transition.

9. Utilisation d'un ou de plusieurs composé(s) de la formule I selon une ou plusieurs des revendications 1 à 7 en tant que composants dans un milieu cristallin liquide.

10. Milieu cristallin liquide comprenant au moins deux composés mésogènes, **caractérisé en ce qu'**il comprend au moins un composé de la formule I selon une ou plusieurs des revendications 1 à 7.

11. Milieu cristallin liquide selon la revendication 10, **caractérisé en ce qu'**il comprend un ou plusieurs composé(s) des formules II et/ou III : 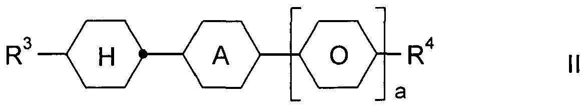 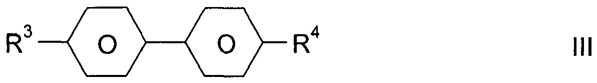 dans lesquelles :
cycle A représente 1,4-phénylène ou trans-1,4-cyclohexylène,
a est 0 ou 1,
R³ représente dans chaque cas, indépendamment des autres, alkyle comportant de 1 à 9 atome(s) de C ou alkényle comportant de 2 à 9 atomes de C, et
R⁴ représente dans chaque cas, indépendamment des autres, un radical alkyle non substitué ou halogéné comportant de 1 à 12 atome(s) de C, où, en outre, un ou deux groupe(s) CH₂ non adjacents peut/peuvent être remplacé(s) par -O-, -CH=CH-, -CH=CF-, -(CO)-, -O(CO)- ou -(CO)O- de telle sorte que des atomes de O ne soient pas liés directement les uns aux autres.

12. Milieu cristallin liquide selon la revendication 10 ou 11, **caractérisé en ce qu'**il comprend un ou plusieurs composé(s) des formules X, XI et/ou XXVI : 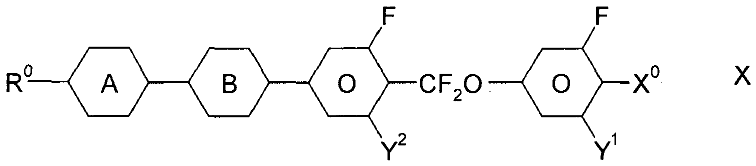 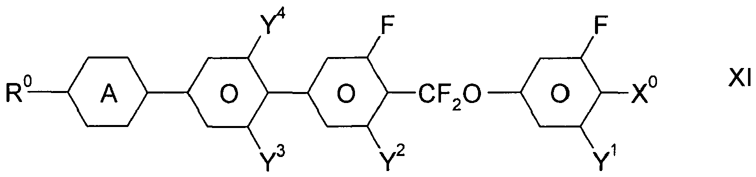 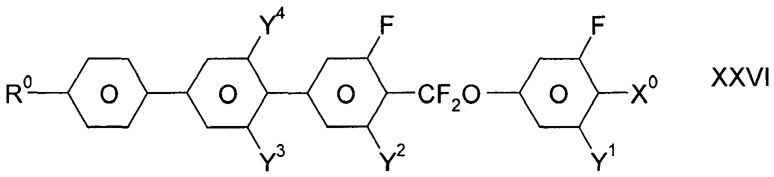 dans lesquelles :
R⁰ représente un radical alkyle ou alcoxy comportant de 1 à 15 atome(s) de C, où, en outre, un ou plusieurs groupe(s) CH₂ dans ces radicaux peut/peuvent (chacun) être remplacé(s), indépendamment les uns des autres, par -C≡C-, -CF₂O-, -CH=CH-, 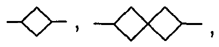 -O-, -(CO)O- ou -O(CO)- de telle sorte que des atomes de O ne soient pas liés directement les uns aux autres, et dans lesquelles, en outre, un ou plusieurs atome(s) de H peut/peuvent être remplacé(s) par halogène, 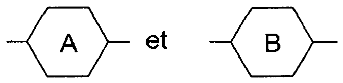 représentent, chacun indépendamment de l'autre, 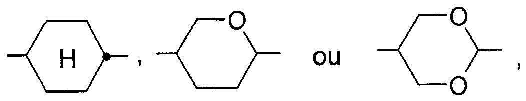
X⁰ représente F, CI, CN, SF₅, SCN, NCS, un radical alkyle halogéné, un radical alkényle halogéné, un radical alcoxy halogéné ou un radical alkényloxy halogéné, chacun comportant jusqu'à 6 atomes de C, et
Y¹, Y², Y³, Y⁴ représentent chacun, indépendamment des autres, H ou F.

13. Milieu cristallin liquide selon une ou plusieurs des revendications 10 à 12, **caractérisé en ce que** la teneur totale de composés pontés CF₂O, incluant les composés de la formule I, est de 35% en poids ou plus.

14. Utilisation du milieu cristallin liquide selon une ou plusieurs des revendications 10 à 13 à des fins électro-optiques.

15. Affichage électro-optique à cristaux liquides contenant un milieu cristallin liquide selon une ou plusieurs des revendications 10 à 13.
